(19) **Europäisches Patentamt / European Patent Office / Office européen des brevets**

(11) **EP 3 829 618 B1**

(12) **EUROPEAN PATENT SPECIFICATION**

(45) Date of publication and mention
of the grant of the patent:
**16.07.2025 Bulletin 2025/29**

(21) Application number: **19779554.5**

(22) Date of filing: **30.07.2019**

(51) International Patent Classification (IPC):
*A61K 38/10* $^{(2006.01)}$   *A61K 39/00* $^{(2006.01)}$
*A61K 39/155* $^{(2006.01)}$   *A61P 31/14* $^{(2006.01)}$
*A61P 43/00* $^{(2006.01)}$   *C07K 14/135* $^{(2006.01)}$
*C12N 15/62* $^{(2006.01)}$

(52) Cooperative Patent Classification (CPC):
**C07K 14/135; A61K 38/10; A61K 39/00;
A61K 39/155; A61P 31/14; A61P 43/00;
C12N 15/62**

(86) International application number:
**PCT/IB2019/056501**

(87) International publication number:
**WO 2020/026147 (06.02.2020 Gazette 2020/06)**

(54) **ANTIGEN PURIFICATION METHOD**

ANTIGEN-REINIGUNGSMETHODEN

METHODE DE PURIFICATION D'ANTIGENE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **31.07.2018 US 201862712280 P**

(43) Date of publication of application:
**09.06.2021 Bulletin 2021/23**

(73) Proprietor: **GlaxoSmithKline Biologicals SA
1330 Rixensart (BE)**

(72) Inventors:
• **BAKSHI, Kunal
Rockville, Maryland 20850 (US)**
• **BUGNO, Marcin Krzysztof
Rockville, Maryland 20850 (US)**
• **CHANDRAMOULI, Sumana
Rockville, Maryland 20850 (US)**
• **TYSON, Matthew Albert
Wendell, North Carolina 27591 (US)**
• **WANG, Zihao
Rockville, Maryland 20850 (US)**
• **WILSON, Mark Jonathan
Rockville, Maryland 20850 (US)**

(74) Representative: **Thornley, Rachel Mary
GSK
Legal & Compliance - Global Patents
79 New Oxford Street
London WC1A 1DG (GB)**

(56) References cited:
**WO-A1-2014/160463     WO-A1-2017/172890**

Remarks:
The complete document including Reference
Table(s) and the Sequence Listing(s) can be
downloaded from the EPO website

**Description**

FIELD OF THE INVENTION

The present invention provides methods and compositions for purifying antigens from various samples, as well as methods and compositions for the use of said antigens.

BACKGROUND TO THE INVENTION

Respiratory syncytial virus (RSV) is an important human pathogen belonging to the Paramyxoviridae family. Closely related viruses include mumps, measles and parainfluenza viruses. RSV is a leading cause of infant hospitalization, and early childhood infection is strongly correlated with wheezing and increased occurrence of childhood asthma. This results in a significant burden on children, parents and healthcare systems, with an estimated cost of billions of dollars each year for interventions. After malaria, it is the single most common transmissible cause of mortality in children aged 1 month to 1 year old.

No vaccine currently exists for RSV, and the only mode of prophylaxis is through passive administration of a monoclonal antibody, palivizumab, targeting the fusion protein F. The prohibitive cost of this strategy has restricted its use to high-risk infants at the start of the RSV season each year, and further underscores the need for a broadly available effective vaccine. On the other hand, protection conferred by the monoclonal antibody suggests that humoral immunity against the virus is effective in prophylaxis of infection.

RSV F, the primary target of neutralizing antibodies against the virus, is a Class I fusion glycoprotein abundant on the viral envelope. Like other viral fusion proteins, F exists in a trimeric "prefusion" state on the viral surface. Once entry is triggered, it inserts hydrophobic fusion loops into the cell membrane and undergoes extensive conformational change to fold into an energetically favorable trimeric "postfusion" state, fusing the viral and cell membranes together in the process.

The precursor RSV F protein sequence contains a signal peptide (25 residues), a 529 residue ectodomain including 3 heptad repeats (HRA, HRB, and HRC), a transmembrane region and a short cytoplasmic tail. The wildtype protein is expressed as a single polypeptide (F0) that is eventually cleaved internally at two furin sites. Furin proteolysis releases a 27 amino acid peptide (p27) and separates the protein into two chains, F1 and F2, which remain linked by two disulfide bonds (C37-C439; C69-C212). These cleavages free the N-terminus of the hydrophobic fusion peptide (F1 N-terminus), making it available for eventual membrane insertion, which allows F to function as a fusogen. The final mature RSV F protein also contains glycosylations at three N-linked glycosylation sites (N27, N70 and N500).

Exogenously expressed F ectodomain spontaneously folds into a highly-stable trimer in the postfusion conformation. The postfusion F crystal structure has been solved and shows the preservation of two important, surface exposed, neutralizing epitopes, Site A and Site C.

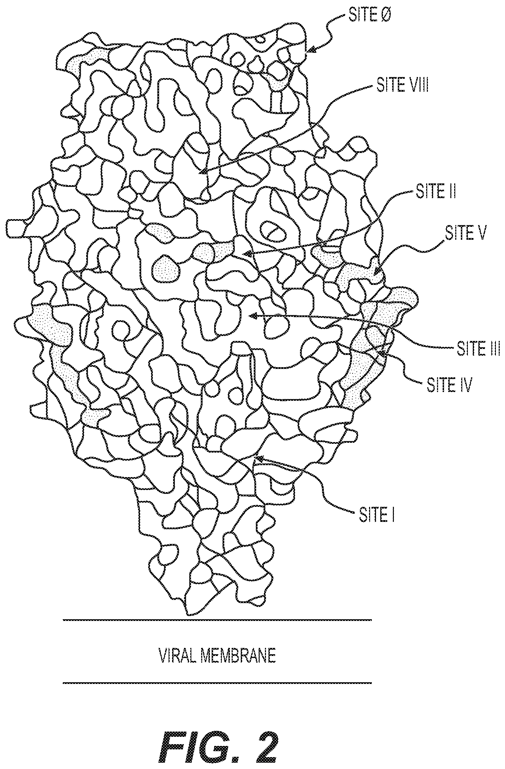

The prefusion F structure was solved by co-expressing the F ectodomain with the Fab fragment of a prefusion F-specific antibody. It and two related antibodies recognize an epitope on F that is unique to its prefusion conformation, designated Site Ø. This epitope is near the top of the molecule and is formed by residues 60-75 (F2) and the HRA helix residues 196-209 (F1). In the extensive structural rearrangement that occurs when F flips to its postfusion conformation, this epitope is destroyed, with ~180° change in the relative position of the loop with respect to the HRA helix in the pre- and postfusion molecules. By contrast, the other two neutralizing sites in F, Site A and Site C, are preserved in both the prefusion and postfusion conformations. The quaternary epitope site V overlaps with a $\beta_3$-$\beta_4$ motif and is present on the trimeric form of prefusion RSV-F. See McLellan et al (2015) "Characterization of a Prefusion-Specific Antibody That Recognizes a Quaternary, Cleavage-Dependent Epitope on the RSV Fusion Glycoprotein," PLOS Pathog, 11:e1005035. Site V is shown in Fig.2.

To stabilize the F ectodomain in its prefusion conformation, three main changes to the ectodomain sequence are required. A C-terminal trimerization domain is needed. An example is the foldon which is a 27-residue domain from the C-terminus of the T4 bacteriophage fibritin molecule that spontaneously trimerizes upon expression. In the absence of this domain but with the additional changes, the F protein will take up the prefusion conformation, but fails to form trimers, and is not highly immunogenic. The foldon has been linked to the F C-terminus by a short 4-residue non-cleavable linker, Ser-Ala-Ile-Gly.

The other two changes are stabilizing mutations designed based on the antibody-bound structure to help maintain F in its prefusion state without antibody addition. Following several testing/design iterations, a combination of two sets of mutations gave the best results. This molecule, termed DS-Cav1 in McLellan et. al. Science (2013) vol. 342 p. 592), contains a disulfide ("DS") introduced by mutating Ser residues at 155 and 290 to Cys (S155C-S290C) to prevent it from flipping to the postfusion state. It also contains cavity-filling mutations, S190F and V207L, to stabilize Site Ø at the apex. This RSV soluble F protein polypeptide comprising S155C, S290C, S190F, and V207L substitutions raises neutralizing antibodies much more potently than postfusion F and is able to deplete over 85% of the RSV-specific neutralizing antibodies in human sera.

Notwithstanding the present availability of published methods for the production of bench-top amounts of the RSV soluble F protein polypeptide, there remains a need for pure and stable RSV soluble F protein polypeptide that does not comprise an affinity tag, i.e., tagless; an affinity tag could itself induce a spurious immune response or, even if cleaved, leave an artifactual post-cleavage amino acid sequence that may induce a spurious immune response. Moreover, when produced recombinantly in sufficient quantities for use in a vaccine, this RSV soluble F protein polypeptide co-purifies with host-cell proteins that may reduce immunogenicity, proteolyze the recombinant product, or induce an immune response. Accordingly, there remains a need for pure and stable RSV soluble F protein polypeptide at vaccine manufacturing scale that is substantially free of host-cell proteins.

## SUMMARY OF THE INVENTION

Applicant herein provides an antigen composition according to claim 1 and discloses processes for the manufacture of such a composition of highly pure, tagless recombinant RSV soluble F protein polypeptide comprising S155C, S290C, S190F, and V207L substitutions, processes for the manufacture of highly stable compositions of the same, as well as compositions and methods for using the highly pure recombinant RSV soluble F protein polypeptide. Also disclosed is an antigen composition comprising a population of trimers of recombinant RSV soluble F protein polypeptides, wherein each trimer of said population is comprised of three recombinant RSV soluble F protein polypeptides selected independently from the group consisting of: (a) a recombinant RSV soluble F protein polypeptide comprising a F1 chain having S155C, S290C, S190F, and V207L substitutions ("F protomer"); and (b) a recombinant RSV soluble F protein polypeptide comprising a F1 chain having S155C, S290C, S190F, and V207L substitutions, further comprising at least 10 amino acids of the p27 peptide ("F' protomer") described by the formula

$$nF + (3-n)F' \qquad \text{(Formula I)}$$

wherein n is an integer from 0-3, $F$ is an F protomer and $F'$ is an F' protomer, wherein at least 1%, such as at least 2%, at least 3%, at least 4%, at least 5%, at least 6%, at least 7%, at least 8% at least 9%, at least 10%, at least 11%, at least 12%, at least 13%, at least 14%, at least 15%, at least 20%, at least 25%, of the recombinant RSV soluble F protein polypeptides in the population of trimers are F' protomers.

## DESCRIPTION OF DRAWINGS/FIGURES

FIG.1A The wildtype RSV F protein sequence is depicted in schematic form: signal peptide (25 residues), 529 residue ectodomain including 3 heptad repeats (HRA, HRB, and HRC), transmembrane region (TM), short cytoplasmic tail, glycosylation sites (circled G), and furin cleavage sites (scissors). The signal peptide (SP) and p27 peptide are removed during protein maturation, resulting in the F1 and F2 chains (diamond symbol = post-cleavage site left after p27 removal).

FIG.1B The recombinant RSV soluble F protein polypeptide is shown with the S155C, S290C, S190F, and V207L substitutions (vertical lettering) and foldon; the other figure labels are as described in FIG.1A.

FIG.2 Various antibody binding sites are shown on a representation of the prefusion RSV F protein, including Site Ø.

FIG.3A Viable cell density and viability of the recombinant RSV soluble F protein polypeptide-producing CHO cell clone versus increment of production time in a production-scale bioreactor.

FIG.3B. Recombinant RSV soluble F protein polypeptide titer accumulation in a production-scale bioreactor.

FIG.4 Glycan profile of the recombinant RSV soluble F protein polypeptide produced by the process disclosed in the examples. Peaks #2 and #3 represent sialyated glycans.

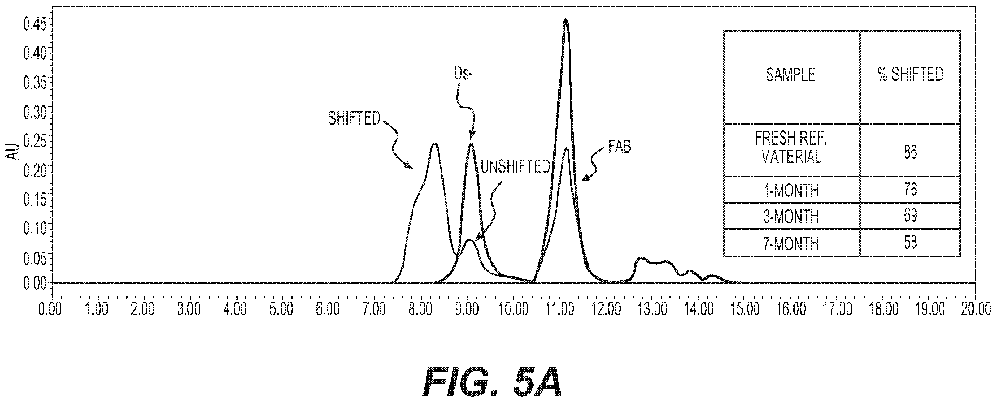

FIG.5A Fab Shift SEC-LC results of recombinant RSV soluble F protein polypeptide after storage at 5°C in liquid buffer for 3 months. See Example 3(F). From left to right, the peaks are shifted recombinant RSV soluble F protein polypeptide ("Shifted"); recombinant RSV soluble F protein polypeptide alone (major peak, "Ds") and unshifted recombinant RSV soluble F protein polypeptide (minor peak, "Unshifted"); Site Ø binding Fab (major peak, not labelled), and Site Ø binding Ab Fab (minor peak, "Fab"). The inset panel reports the percent of recombinant RSV soluble F protein polypeptide that is shifted (retains Site Ø binding) at time 0, 1-month, 3-months, and 7-months (in FIG.5A, only the chromatogram for 3-month material is shown).

FIG.5B Fab Shift SEC-LC Results of recombinant RSV soluble F protein polypeptide after lyophilized recombinant RSV soluble F protein polypeptide was stored for 2 weeks at 40°C and then reconstituted with the appropriate buffer, peaks as labelled and described in FIG.5A.

FIG.5C Fab Shift SEC-LC Results of recombinant RSV soluble F protein polypeptide after lyophilized recombinant RSV soluble F protein polypeptide was stored at 5°C for 6 months and then reconstituted with the appropriate buffer, peaks as labelled and described in FIG.5A.

FIG.6A SDS-PAGE Gel of recombinant RSV soluble F protein polypeptide fresh reference material (1st lane) and recombinant RSV soluble F protein polypeptide 3-month stability material (2nd lane) (a portion of the 3-month stability material had lost Site Ø binding). However, the gel band patterns were very similar between the fresh reference and 3-month stability materials, suggesting that the Site Ø binding antibody-binding loss in the 3-month material was not due to a protein clipping event.

FIG.6B Liquid Chromatography-Mass Spectrometry (LC-MS) Butterfly Peptide Map of recombinant RSV soluble F protein polypeptide fresh reference material (top) and 3-month stability material (bottom). The fresh reference and 3-month materials had a comparable peptide fingerprint, which suggested that chemical modifications, such as oxidation, deamination, and disulfide bond scrambling, were also not cause of the loss of Site Ø binding antibody-binding.

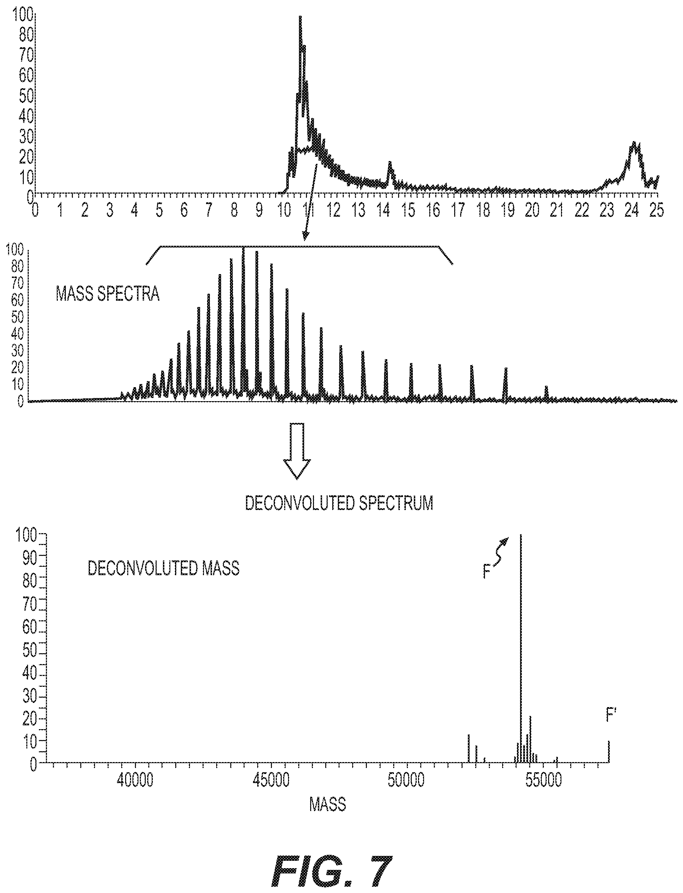

FIG.7 Determination of the intact mass of de-N-glycosylated recombinant RSV soluble F protein polypeptide by LC-MS.

FIG.8 Reversed-phase liquid chromatography (RP-HPLC) chromatogram of recombinant RSV soluble F protein polypeptide, showing elution of the F' protomer (minor peak) followed by the elution of the F protomer (major peak). Samples were run in duplicate.

FIG.9. The integration of drug substance (antigen composition) by RP HPLC (p27 = F'; RSV preF = F).

FIG.10. The integration of drug subtance (antigen composition) standard load 10μL by RP-UPLC (p27 = F'; RSV preF = F).

FIG.11. Typical weak cation exchange profile of trimers of recombinant RSV soluble F protein polypeptide. Peak 1 is FFF and Peak 2 is F'-containing trimer.

FIG.12. Fractions were collected for further analysis (grey-shading).

FIG.13. Reversed phase liquid chromatography profile of trimers of recombinant RSV soluble F protein polypeptide (labelled RSVPreF3 {originally identified using the term "DS-Cav1", herein re-termed RSVPreF3 to distinguish it from the material produced as disclosed in McLellan et. al. Science (2013) vol. 342 p. 592, WO2014160463, and ClinicalTrials.gov Identifier: NCT03049488}, bottom graph line), Peak 1 (second graph line) and Peak 2 (third graph line from bottom) from weak cation exchange chromatography.

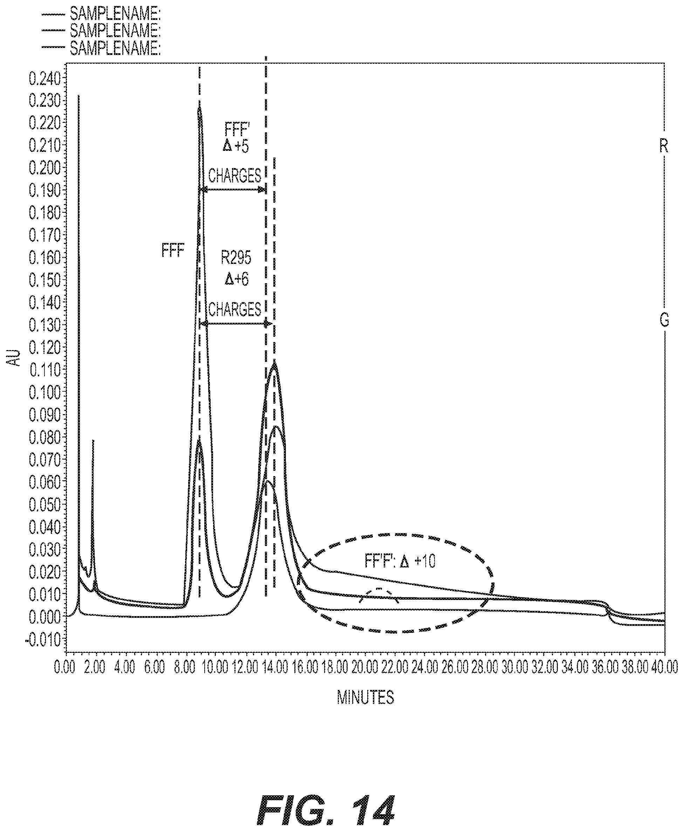

FIG.14. Weak cation exchange chromatography profile of recombinant RSV soluble F protein polypeptide, recombinant RSV soluble F protein polypeptide +R295, and R295. The left-most peak (delineated by a dotted vertical line) represents the retention time for a FFF trimer. The second peak from the left (delineated by the left-hand vertical bold dashed line) represents the retention time for a FFF' trimer ($\Delta$+5 charge). The right-most peaks represent the retention time for a R295 F'F'F' (delineated by the right-hand vertical dashed line) trimer ($\Delta$+6 charge). Note the absence of a peak at the predicted retention time for a FF'F' ($\Delta$+10 charge)(grey dashed curve within bold dashed circle).

FIG.15 High molecular weight species (HMWS) in RSVPreF3 and DS-Cav1 were investigated using size exclusion high pressure liquid chromatography (SE-HPLC). The chromatogram in FIG.15 from left to right depicts peaks for RSVPreF3 (left, taller) and DS-Cav1 (right, shorter). RSVPreF3 is observed to be symmetrical, while DS-Cav1 appears not symmetrical (large shoulders with no resolved peaks) due to heterogeneity.

FIG.16 Sedimentation determined by analytical ultracentrifugation (AUC) for the following samples: RSVPreF3 lyophilization sample 1 (peak labelled 4.05s); RSVPreF3 lyophilization sample 2 (peak labelled 3.99s); RSVPreF3 liquid sample (peak labelled 4.09s); DS-Cav1 liquid sample (peak labelled 4.17s).

FIG.17A Host Cell Protein (HCP)-content investigated by non-reduced and reduced SDS-PAGE. From left-to-right, the lanes are: Size marker; non-reduced RSVPreF3; non-reduced DS-Cav1; reduced RSVPreF3; reduced DS-Cav1; and size marker. Note the presence of a minor band representing FFF' (F1+p27) just above the major band in RSVPreF3, non-reduced and reduced.

FIG.17B Western blot (from gel) with GRP78 detected by primary Ab: GRP78/BiP Antibody (Thermo Fisher Scientific, Catalog #: PA1-014A, Host/Isotype: Rabbit/IgG, Polyclonal); Secondary Ab: Alexa Fluor 647 chicken anti-rabbit IgG (H+L), (Invitrogen, A21443).

FIG.18 RP-HPLC was used to investigate content and purity of RSVPreF3 and DS-Cav1 compositions. Note the absence of a p27 peak in the DS-Cav1 chromatograph trace.

FIG.19 Weak Cation Exchange (WCX) was used to investigate the FFF and FFF' populations of RSVPreF3 and DS-Cav1. The RSVPreF3 composition shows 2 distinct, symmetrical, well resolved peaks (FFF and FFF'), whereas DS-Cav1 is a single relatively broad peak with retention time between FFF and FFF'.

FIG.20 Immuno-characterization of the RSVPreF3 (lyophilized) in cows by measuring the boosting of RSV A neutralizing antibody (Nab) titers in the groups receiving the DS-Cav1 or RSVPreF3 formulations compared to Saline group. D -7 = 7 days prior to immunization; dPI = days post-immunization.

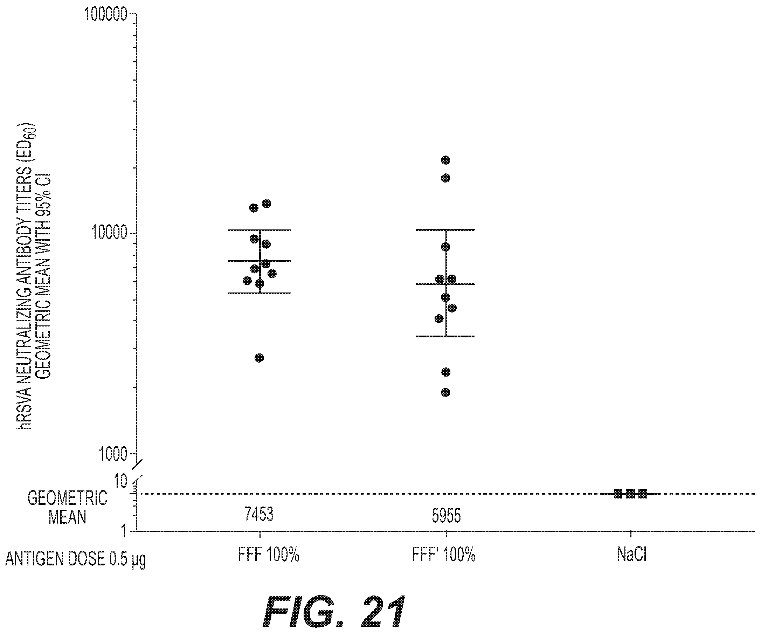

FIG.21 The impact of p27 on immunogenicity in naïve CB6F1 mice was assessed by observing hRSV A neutralizing antibody titers induced by FFF- or FFF'-formulations in naïve CB6F1 mice at 14 days post immunization. FFF' induced RSV A neutralizing Ab responses that are non-inferior to the NAb responses induced by FFF.

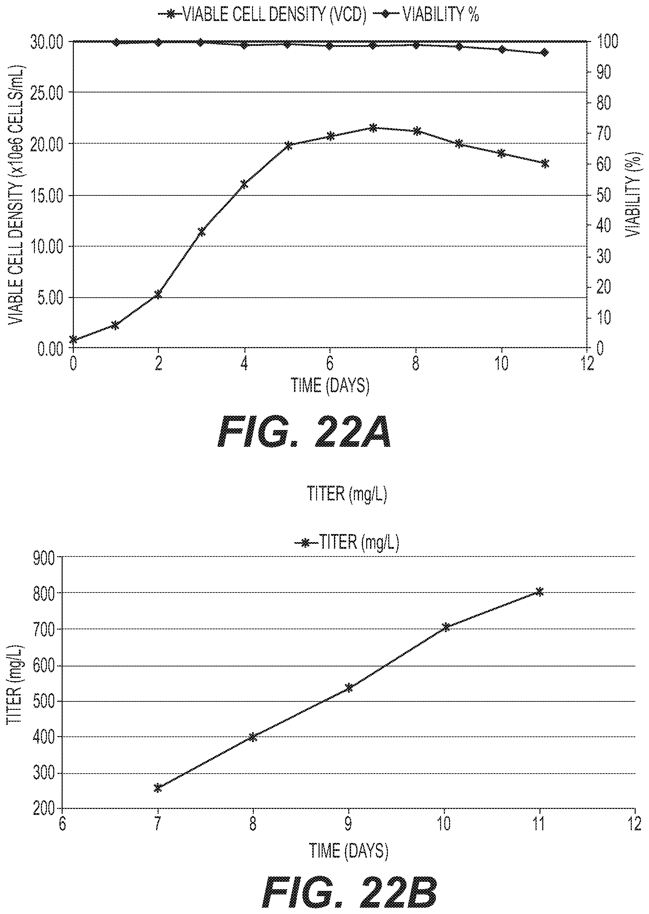

FIG.22A Viable cell density and viability of the recombinant RSV soluble F protein polypeptide-producing CHO cell clone versus increment of production time in a production-scale bioreactor.

FIG.22B. Recombinant RSV soluble F protein polypeptide titer accumulation in a production-scale bioreactor.

DETAILED DESCRIPTION OF THE INVENTION

Applicant has observed that the recombinant RSV soluble F protein polypeptide is unstable under certain storage conditions and undergoes a spontaneous conformational change leading to a measurable decrease (by Fab antibody shift assay) in antibody binding at Site Ø and Site V, which conformational change affects an increasing proportion of recombinant RSV soluble F protein polypeptide during storage in liquid buffer. Compositions and methods are disclosed herein for arresting or minimizing the fraction of recombinant RSV soluble F protein polypeptide that undergoes this conformational change. Applicant provides herein antigen compositions comprising RSV soluble F protein polypeptide having S155C, S290C, S190F, and V207L substitutions that are observed to be of greater purity, exhibit lower aggregation, lower amounts of partial glycosylation, and lower pI than previously published recombinant RSV soluble F protein polypeptide having S155C, S290C, S190F, and V207L substitutions. It is expected that the recombinant RSV soluble F protein polypeptides disclosed herein and the processes for their manufacture result in recombinant RSV soluble F protein polypeptide of greater purity, exhibiting lower host-cell protein content and lower aggregation, lower amounts of partial glycosylation, and lower pI than recombinant RSV soluble F protein polypeptides produced as disclosed in WO2014160463.

DEFINITIONS

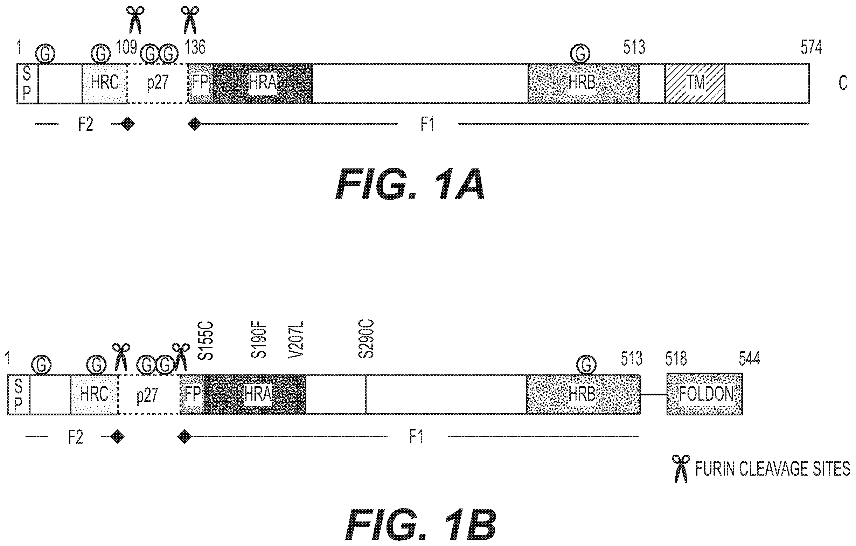

By the term "recombinant RSV soluble F protein polypeptide" or "soluble F protein polypeptide" is intended a RSV F protein comprising S155C, S290C, S190F, and V207L substitutions and including a trimerization foldon domain. An example of such a substituted RSV F protein is disclosed in WO2014160463 and the substituted amino acid positions are described with reference to the wildtype RSV F protein sequence set forth as sequence identifier 124 therein. A recombinant RSV soluble F protein polypeptide is expressed as a single polypeptide (F0) that is eventually cleaved internally by furin. An exemplary F0 recombinant RSV soluble F protein polypeptide is depicted in FIG.1B and an exemplary F0 polypeptide sequence is set forth in SEQ ID NO:1. Furin proteolysis separates the protein into two chains, one comprising an F1 amino acid sequence and one comprising an F2 amino acid sequence, and thus a recombinant RSV soluble F protein polypeptide will comprise at least an F1 chain and an F2 chain. Because of the presence of two furin cleavage sites, proteolysis can result in the two chains, F1 and F2, as well as the release of the p27 peptide, however, Applicants have observed that a fraction of mature recombinant RSV soluble F protein polypeptide may be cleaved at only one furin cleavage site and the p27 peptide is not released. This is described elsewhere herein.

By "F1 chain" is intended the C-terminal portion of the cleaved F0 molecule. An exemplary F1 chain is indicated in FIG.1B and exemplary F1 chain polypeptide sequences are set forth in SEQ ID NO:2 and SEQ ID NO:8.

By "F2 chain" is intended the N-terminal portion of the cleaved F0 molecule from which the signal protein has been removed. An exemplary F2 chain is indicated in FIG.1B and an exemplary F2 chain polypeptide sequence is set forth in SEQ ID NO:3.

By "F protomer" or "preF" is intended a recombinant RSV soluble F protein polypeptide having S155C, S290C, S190F, and V207L substitutions, but lacking a p27 peptide amino acid sequence.

By "F' protomer" or "preF'''" is intended a recombinant RSV soluble F protein polypeptide having S155C, S290C, S190F, and V207L substitutions, but comprising at least ten contiguous amino acids of the p27 peptide up to the entirety of the p27 peptide.

By "p27 peptide" is intended an amino acid sequence corresponding to the full-length of amino acids of the polypeptide (F0) that would be released through complete proteolysis at both of the two furin sites. In some embodiments, furin proteolysis releases a 27 amino acid p27 peptide which may vary RSV strain to RSV strain. In some embodiments, a p27 peptide has the amino acid sequence of SEQ ID NO:7.

By "trimer" or "trimer of recombinant RSV soluble F protein polypeptides" is intended a protein complex of three recombinant RSV soluble F protein polypeptides wherein each recombinant RSV soluble F protein polypeptide comprises an F1 chain and a F2 chain and, further, wherein each recombinant RSV soluble F protein polypeptide of the complex is independently selected from the group consisting of: F' and F protomer; such that structure of the trimer is described by the formula

$$nF + (3-n)F' \qquad \text{(Formula I)}$$

wherein n is an integer from 0-3, F is an F protomer and *F'* is an F' protomer.

By "uncleaved furin cleavage site" is intended the minimal amino acid sequence ... $RXRRX_1$... (SEQ ID NO. 9) where X and $X_1$ are any amino acids (see, e.g., Moehring et al. (1993) "Strains of CHO-K1 cells resistant to Pseudomonas exotoxin A and cross-resistant to diphtheria toxin and viruses," Infect. Immun. 41:998-1009.)

By "antigen" is intended a compound, composition, or substance that can stimulate the production of antibodies and/or a T cell response in an animal, including compositions that are injected, absorbed or otherwise introduced into an animal. Accordingly, by the term "antigenic polypeptide" is intended a polypeptide that can stimulate the production of antibodies and/or a T cell response in an animal. The term "antigen" includes all related antigenic epitopes. The term "epitope" or "antigenic determinant" refers to a site on an antigen to which B and/or T cells respond. The "dominant antigenic epitopes" or "dominant epitope" are those epitopes to which a functionally significant host immune response, e.g., an antibody response or a T-cell response, is made. Thus, with respect to a protective immune response against a pathogen, the dominant antigenic epitopes are those antigenic epitopes that when recognized by the host immune system result in protection from disease caused by the pathogen. The term "T-cell epitope" refers to an epitope that when bound to an appropriate MHC molecule is specifically bound by a T cell (via a T cell receptor). A "B-cell epitope" is an epitope that is specifically bound by an antibody (or B cell receptor molecule).

By "antigen composition" is intended a composition comprising an antigenic polypeptide (including higher order forms of the polypeptide, such as a trimer or the like) or two or more different antigenic polypeptides (including their higher order forms). As described herein, an antigen composition may undergo purification to remove unwanted molecules, such as host-cell proteins when the antigenic composition results from expression of an antigenic polypeptide in host-cells, other contaminating molecules, and the like.

By "purification" is intended the process of removing components from a composition, the presence of which is not desired. Purification is a relative term, and does not require that all traces of the undesirable component be removed from the composition. In the context of vaccine production, purification includes such processes as centrifugation, dialization, ion-exchange chromatography, and size-exclusion chromatography, affinity-purification or precipitation. Thus, the term "purified" does not require absolute purity; rather, it is intended as a relative term. Thus, for example, a purified antigen composition is one in which the specified antigen is more enriched than the antigen is in its generative environment, for instance within a cell or in a biochemical reaction chamber. A preparation of substantially pure antigen can be purified such that the desired antigen represents at least 95%, such as at least 96%, at least 97%, at least 98%, or at least 99% or more w/w of the total of antigen plus unwanted protein content of the preparation as determined by ELISA, such as Cygnus CHO hcp ELISA.

By "host-cell" is intended a cell comprising a nucleic acid encoding a recombinant polypeptide, such as the recombinant RSV soluble F protein polypeptide herein. Exemplary host-cells include prokaryotic (i.e., bacterial) host-cells, such as E. *coli,* as well as numerous eukaryotic host-cells, including fungal (e.g., yeast) cells, insect cells, and mammalian cells (such as CHO, VERO and HEK293 cells).

By "host-cell protein" or "HCP" is intended a polypeptide produced by the host-cell in the absence of a vector encoding a recombinant polypeptide. For instance, the recombinant RSV soluble F protein polypeptide would not be considered a host-cell protein whereas the GRP78 protein would be considered a host-cell protein.

By "GRP78" is intended the 78 kD glucose-regulated or heat shock 70 protein 5 (HSPA5) host-cell protein, Accession Number NP_001233668 for CHO.

By "unaggregated" in the context of a recombinant RSV soluble F protein polypeptide trimer is intended a trimer having a molecular weight predicted for a trimer of three protomers, i.e., greater than 150 kDa but less than 200 kDa as determined by size exclusion high pressure liquid chromatography (SE-HPLC) (described elsewhere herein). In contrast, an aggregated recombinant RSV soluble F protein polypeptide trimer may present as a dimer of the trimer or even higher order.

By "sialyated" or "% sialyated" in the context of a recombinant RSV soluble F protein polypeptide is intended the proportion of sialyated glycan out of the total N-glycan on the recombinant RSV soluble F protein polypeptide as determined by glycan profiling carried out as follows: first cleaving N-glycans from recombinant RSV soluble F protein polypeptide, then labelling the cleaved N-glycans with 2-AB through reductive amination, then resolving the cleaved N-glycans by hydrophilic interaction liquid chromatography (HILIC), and detecting and quantifying the identity of the N-glycan in each peak by FLR and MS detectors.

By "immunogenic composition" is intended a composition of matter suitable for administration to a human or animal subject (e.g., in an experimental setting) that is capable of eliciting a specific immune response, e.g., against a pathogen, such as RSV. As such, an immunogenic composition includes one or more antigens (for example, polypeptide antigens) or antigenic epitopes. An immunogenic composition can also include one or more additional components capable of eliciting or enhancing an immune response, such as an excipient, carrier, and/or adjuvant. In certain instances, immunogenic compositions are administered to elicit an immune response that protects the subject, wholly or partially, against symptoms or conditions induced by a pathogen. In some cases, symptoms or disease caused by a pathogen is prevented (or reduced or ameliorated) by inhibiting replication of the pathogen (e.g., RSV) following exposure of the subject to the pathogen. In the context of this disclosure, the term immunogenic composition will be understood to encompass compositions that are intended for administration to a subject or population of subjects for the purpose of eliciting a protective pre-exposure immune response against RSV or palliative post-exposure immune response against RSV (that is, vaccine compositions or vaccines).

By "pharmaceutically acceptable" is intended that the referent is suitable for administration to a subject (e.g., a human or animal subject). Remington's Pharmaceutical Sciences, by E. W. Martin, Mack Publishing Co., Easton, PA, 15th Edition (1975), describes compositions and formulations (including diluents) suitable for pharmaceutical delivery of therapeutic and/or prophylactic compositions, including immunogenic compositions.

By "adjuvant" is intended an agent that enhances the production of an immune response in a non-specific manner. Common adjuvants include suspensions of minerals (alum, aluminium hydroxide, aluminium phosphate) onto which antigen is adsorbed; emulsions, including water-in-oil, and oil-in-water (and variants thereof, including double emulsions and reversible emulsions), liposaccharides, lipopolysaccharides, immunostimulatory nucleic acids (such as CpG oligonucleotides), liposomes, Toll-like Receptor agonists (particularly, TLR2, TLR4, TLR7/8 and TLR9 agonists), and various combinations of such components.

By "kosmotropic agent" is intended a polar water-structure making molecule or ion that generally stabilizes proteins. In some embodiments a kosmotropic agent is a polar or charged molecule. In some embodiments a kosmotropic agent is a charged ion from a salt.

By "chaotropic agent" is intended a water-structure breaking molecule that generally destabilizes proteins. In some embodiments a chaotropic agent is a polar or charged molecule. In some embodiments a chaotropic agent is a charged ion from a salt.

ANTIGEN COMPOSITIONS

In some embodiments of the invention, antigen compositions are provided comprising a recombinant respiratory syncytial virus (RSV) soluble F protein polypeptide comprising S155C, S290C, S190F, and V207L substitutions, wherein said composition comprises greater than 96%, such as greater than 97%, greater than 98%, greater than 99%, greater than 99.1%, greater than 99.2%, greater than 99.3%, greater than 99.4%, greater than 99.5%, greater than 99.6%, greater than 99.7%, greater than 99.8%, or greater than 99.9% recombinant RSV soluble F protein polypeptide w/w versus host-cell protein. In this embodiment, the weight of RSV soluble F protein is compared to the combined total weight of host-cell protein added to the weight of RSV soluble F protein. Thus, in this embodiment an antigenic composition may comprise a substantial amount of other non-host-cell proteins, such as a further recombinant antigen, a polypeptide immune-stimulant or adjuvant, or the like, and yet still comprise for instance at least 99.9% RSV soluble F protein w/w versus host-cell protein.

The identity of host-cell proteins may be determined by mass spectrometry, and the quantity present by isotope dilution mass spectrometry as follows. The antigen composition is reduced by 1,4-Dithiothreitol (DTT) based on determined concentrations, alkylated by isoacetamide, and digested by trypsin overnight at 37°C. The digest is acidified and analyzed by LC-MS/MS using a device, such as a Thermo Fusion Orbitrap operated under data-dependent acquisition mode. Peak lists from the raw data are searched against a protein database containing antigen sequence and sequences of all known and predicted host-cell proteins using the a search engine such as the Marscot search engine. To quantitate the amount of any contaminating or unwanted protein, one may carry out an isotope dilution mass spectrometry method.

To determine the quantity of HCPs overall, an ELISA assay may be utilized. Suitable ELISA kits are available in the art and following the manufacturer's instruction. For instance, for HCPs from CHO cells, a suitable kit is available from Cygnus Technologies, LLC, 4332 Southport Supply Rd. SE, Southport, NC 28461. See CHO HCP ELISA Product Insert, 800-F015, Rev. 3, 10Jan2018, available at www-dot-cygnustechnologies.com.

Also disclosed herein are antigen compositions comprising a recombinant respiratory syncytial virus (RSV) soluble F protein polypeptide comprising S155C, S290C, S190F, and V207L substitutions, wherein said composition comprises greater than 96%, such as greater than 97%, greater than 98%, greater than 99%, greater than 99.1%, greater than 99.2%, greater than 99.3%, greater than 99.4%, greater than 99.5%, greater than 99.6%, greater than 99.7%, greater than 99.8%, or greater than 99.9% recombinant RSV soluble F protein polypeptide w/w versus total protein.

Also disclosed herein are antigenic compositions comprising less than 0.19%, such as less than 0.18%, such as less than 0.17%, 0.16%, 0.15%, 0.14%, 0.13%, 0.12%, 0.11%, 0.10%, 0.09%, 0.08%, 0.07%, 0.06%, 0.05%, 0.04%, 0.03%, 0.02, 0.019%, 0.018%, 0.017%, 0.016%, 0.015%, 0.014%, or 0.013% host-cell protein w/w versus recombinant RSV soluble F protein polypeptide. Also disclosed herein are antigenic compositions compriseing less than 0.18%, such as less than 0.17%, 0.16%, 0.15%, 0.14%, 0.13%, 0.12%, 0.11%, 0.10%, 0.09%, 0.08%, 0.07%, 0.06%, 0.05%, 0.04%, 0.03%, 0.02, 0.019%, 0.018%, 0.017%, 0.016%, 0.015%, 0.014%, or 0.013% host-cell protein w/w versus total protein.

Also disclosed herein are compositions in which the only host-cell protein detectable by the method herein is a GRP78 host-cell protein. In some embodiments of the present invention, the antigenic composition comprises less than 0.18%, such as less than 0.17%, 0.16%, 0.15%, 0.14%, 0.13%, 0.12%, 0.11%, 0.10%, 0.09%, 0.08%, 0.07%, 0.06%, 0.05%, 0.04%, 0.03%, 0.02, 0.019%, 0.018%, 0.017%, 0.016%, 0.015%, 0.014%, or 0.013% GRP78 host-cell protein w/w versus recombinant RSV soluble F protein polypeptide. Also disclosed herein are antigenic compositions comprising less than 0.18%, such as less than 0.17%, 0.16%, 0.15%, 0.14%, 0.13%, 0.12%, 0.11%, 0.10%, 0.09%, 0.08%, 0.07%, 0.06%, 0.05%, 0.04%, 0.03%, 0.02, 0.019%, 0.018%, 0.017%, 0.016%, 0.015%, 0.014%, or 0.013% GRP78 host-cell protein w/w versus total protein.

In some embodiments of the present invention, the only host-cell protein in an antigen composition detectable by the method herein is a GRP78 host-cell protein and the antigenic composition comprises less than 0.18%, such as less than 0.17%, 0.16%, 0.15%, 0.14%, 0.13%, 0.12%, 0.11%, 0.10%, 0.09%, 0.08%, 0.07%, 0.06%, 0.05%, 0.04%, 0.03%, 0.02, 0.019%, 0.018%, 0.017%, 0.016%, 0.015%, 0.014%, or 0.013% GRP78 host-cell protein w/w versus recombinant RSV soluble F protein polypeptide. In some embodiments disclosed herein, the only host-cell protein in an antigen composition detectable by the method herein is a GRP78 host-cell protein and the antigenic composition comprises less than 0.18%, such as less than 0.17%, 0.16%, 0.15%, 0.14%, 0.13%, 0.12%, 0.11%, 0.10%, 0.09%, 0.08%, 0.07%, 0.06%, 0.05%, 0.04%, 0.03%, 0.02, 0.019%, 0.018%, 0.017%, 0.016%, 0.015%, 0.014%, or 0.013% GRP78 host-cell protein w/w versus total protein.

Also disclosed herein are antigenic compositions comprising greater than 80%, such as greater than 81%, greater than 82%, greater than 83%, greater than 84%, greater than 85%, greater than 86%, greater than 87%, greater than 88%, greater than 89%, greater than 90%, greater than 91%, greater than 92%, greater than 93%, greater than 94%, greater than 95%, greater than 96%, or greater than 97% unaggregated trimer w/w versus aggregated trimer.

To determine the percentage of unaggregated recombinant RSV soluble F protein polypeptide trimer, one may utilize SE-HPLC. Such experiments may be performed with commercially available HPLC devices such as a Waters Alliance with PDA detector utilizing size exclusion chromatography columns such as TOSOH, TSKgel UltraSW Aggregate column, 3 μm, 300 Å, 7.8 x 300 mm, cat. #228556 and TOSOH, TSKgel UltraSW Aggregate guard column, 3 μm, 6 x 40 mm, cat. #A00027. An acceptable mobile phase buffer is 100 mM Sodium Phosphate, 300 mM Sodium Chloride, pH 7.05. Samples are diluted to 220 μg/mL with the mobile phase, injected into the SE-HPLC, and run for 30 minutes at 0.5 mL/min. The sample chromatograms are then integrated within a time window of 9-23.5 minutes. Timed groups are used to simplify the determination of percent low molecular weight (LMW) and high molecular weight (HMW) species present in each sample. Exemplary timed group retention time windows are as follows:

- HMW timed group from 9.0 to 17.3 min.
- Main peak apex is present at around 17.6 to 17.8 min
- LMW timed group from 19.1 to 23.5 min.

The percentage of LMW, main peak, and HMW species present in each recombinant RSV soluble F protein polypeptide sample chromatogram is calculated using the timed groups; all integrated area should add to 100%.

In some embodiments, a glycosylated recombinant RSV soluble F protein polypeptide has a molecular mass greater than 59.5 kDa, such as greater than 60.0 kDa, greater than 60.5 kDa, or greater than 61.0 kDa, inclusive of glycosylation. LC-MS may be utilized to determine the molecular mass.

In some embodiments, a recombinant RSV soluble F protein polypeptide has a pl of less than 7.8, such as less than 7.7, 7.6, 7.5, 7.4, 7.3, 7.2, 7.1, 7.0, 6.9. 6.8, 6.7, 6.6, 6.5, 6.4, 6.3, 6.2, 6.1, 6.0, 5.9, 5.8, 5.7, or less than 5.4 as determined by isoelectric focusing gel electrophoresis. In some embodiments, a recombinant RSV soluble F protein polypeptide has a pl in the range of 7.8-5.3, 7.4-5.3, 7.0-5.3, 6.8-5.3, 6.6-5.3, 6.4-5.3, 6.2-5.3, or 6.0-5.3. In some embodiments, a recombinant RSV soluble F protein polypeptide has a pl in the range of 6.0-5.3.

In some embodiments, a recombinant RSV soluble F protein polypeptide comprises glycan groups, wherein at least 40%, such as at least 45%, at least 50%, at least 55%, at least 60%, at least 65%, at least 70%, or at least 75% of said glycan groups are sialyated as determined by glycan profiling using HILIC-FLR/MS.

An antigen composition disclosed herein comprises a population of trimers of recombinant RSV soluble F protein polypeptides, wherein each trimer of said population is comprised of three recombinant RSV soluble F protein polypeptides selected independently from the group consisting of: (a) a recombinant RSV soluble F protein polypeptide comprising a F1 chain having S155C, S290C, S190F, and V207L substitutions ("F protomer"); and (b) a recombinant RSV soluble F protein polypeptide comprising a F1 chain having S155C, S290C, S190F, and V207L substitutions, further comprising at least 10 amino acids of the p27 peptide ("F' protomer"). In such embodiments the trimers are described by the formula

$$nF + (3-n)F' \qquad \text{(Formula I)}$$

wherein n is an integer from 0-3, F is an F protomer and F' is an F' protomer. In some embodiments, n is an integer from 1-3. In some embodiments of this disclosure, n is an integer from 2-3. In some embodiments, n is 3. In some embodiments, at least 1%, such as at least 2%, at least 3%, at least 4%, at least 5%, at least 6%, at least 7%, at least 8% at least 9%, at least 10%, at least 11%, at least 12%, at least 13%, at least 14%, at least 15%, at least 20%, or at least 25% of the recombinant RSV soluble F protein polypeptides in the population of trimers are F' protomers. The separation of RSV soluble F protein polypeptides into F' protomers and F protomers and determination of their quantitation may be carried out by reversed-phase liquid chromatography (RP-HPLC) under denaturing conditions. As described elsewhere herein, by using RP-HPLC, trimers are denatured into F and F' protomers and the protomers are then resolved based on their differences in hydrophobicity.

In some embodiments of this disclosure, at least 1%, such as at least 2%, at least 3%, at least 4%, at least 5%, at least 6%, at least 7%, at least 8% at least 9%, at least 10%, at least 11%, at least 12%, at least 13%, at least 14%, at least 15%, at least 20%, at least 25%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45% of said trimers of recombinant RSV soluble F protein polypeptides comprise at least one F' protomer. For instance, in one embodiment it was observed that when roughly 20% of protomers were F', roughly 40% of trimers in a population comprised at least one F' protomer. The separation of trimers into trimers of recombinant RSV soluble F protein polypeptides comprising at least one F' protomer and recombinant RSV soluble F protein polypeptides comprising zero F'1 protomers (and their quantitation) may be carried out by weak cation exchange liquid chromatography (WCX-LC) under native conditions. As described elsewhere herein, by using WCX-LC, the trimer conformation is maintained and then resolved based on their intrinsic charge difference.

In some embodiments of this disclosure, the F' protomer comprises a F1 chain comprising an uncleaved furin cleavage site. In some embodiments, the F' protomer has a molecular mass at least 1.0 kDa, such as at least 1.5 kDa, at least 2.0 kDa, at least 2.5 kDa, at least 3.0 kDa greater than the molecular mass of the F protomer. In some embodiments, the ratio of trimers of recombinant RSV soluble F protein polypeptides comprising at least one F' protomer to trimers of recombinant RSV soluble F protein polypeptides comprising zero F' protomers is at least 1:1.2; such as at least 1:1.3; at least 1:1.4; at least 1:1.5; at least 1:1.6; at least 1:1.7; at least 1:1.8; at least 1:1.9; at least 1:2.0; at least 1:2.1; at least 1:2.2; at least 1:2.3; at least 1:2.4; at least 1:2.5; at least 1:2.6; at least 1:2.7; at least 1:2.8; at least 1:2.9; at least 1:3.0; at least 1:3.5; at least 1:4; at least 1:9; at least 1:19; or at least 1:29.

The population trimers of recombinant RSV soluble F protein polypeptides are separable by chromatography into a first and second peak detectable by sedimentation, said first peak having a sedimentation coefficient of about 3.96S and said second peak having a sedimentation coefficient of about 4.13S. Sedimentation coefficient is determined as described in Example 3(H).

According to the present invention, a recombinant RSV soluble F protein polypeptide comprises a F1 chain and a F2 chain wherein the F1 chain comprises the amino acid sequence of SEQ ID NO:2. In some embodiments, the F1 chain consists of the amino acid sequence of SEQ ID NO:2, for example in the case of a F protomer.

In some embodiments, a F' protomer comprises a F1 chain comprising the amino acid sequence of SEQ ID NO:6. In some embodiments, an F' protomer comprises a F1 chain having a N-terminal p27 peptide. In some embodiments, an F' protomer comprises a F1 chain comprising the amino acid sequence of SEQ ID NO:7.

According to the present invention, a recombinant RSV soluble F protein polypeptide comprises a F1 chain and a F2 chain wherein the F2 chain comprises such as consists of, the amino acid sequence of SEQ ID NO:3. Both an F and F' protomer are described by this embodiment.

Thus, according to the present invention, a recombinant RSV soluble F protein polypeptide comprises a F1 chain and a F2 chain wherein the F1 chain comprises an amino acid sequence of SEQ ID NO:2 and the F2 chain comprises an amino acid sequence of SEQ ID NO:3. Suitably the F1 chain comprises the amino acid sequence of SEQ ID NO:2 and the F2 chain comprises, such as consists of, the amino acid sequence of SEQ ID NO:3. Both an F and F' protomer are described by this embodiment. In such embodiments the F1 chain may suitably consist of the amino acid sequence of SEQ ID NO:2, for example in the case of a F protomer.

In some embodiments, a recombinant RSV soluble F protein polypeptide comprises a F1 chain comprising an amino acid sequence having at least 95%, at least 96%, at least 97%, at least 98%, at least 99% identity to SEQ ID NO:8 and a F2 chain comprising an amino acid sequence having at least 95%, at least 96%, at least 97%, at least 98%, at least 99% identity to SEQ ID NO:3, for example in the case of a F' protomer. In some embodiments of the invention the F1 chain comprises, such as consists of, the amino acid sequence of SEQ ID NO:8 and the F2 chain comprises, such as consists of, the amino acid sequence of SEQ ID NO:3.

In some embodiments, a recombinant RSV soluble F protein polypeptide comprises the entirety of the F0 molecule, such as before processing during expression. Suitably the F0 chain comprises, such as consists of, the amino acid sequence of SEQ ID NO:1.

Engineered variants of a recombinant RSV soluble F protein polypeptide that share sequence similarity with the aforementioned sequences can also be employed in the context of the embodiments described above. It will be understood by those of skill in the art, that the similarity between a recombinant RSV soluble F protein polypeptide (and polynucleotide) sequences as described below, as for polypeptide (and nucleotide) sequences in general, can be expressed in terms of the similarity between the sequences, otherwise referred to as sequence identity. Sequence identity is frequently measured in terms of percentage identity (or similarity); the higher the percentage, the more similar are the primary structures of the two sequences. In general, the more similar the primary structures of two amino acid (or polynucleotide) sequences, the more similar are the higher order structures resulting from folding and assembly. Variants of a recombinant RSV soluble F protein polypeptide (and polynucleotide) sequences typically have one or a small number of amino acid deletions, additions or substitutions but will nonetheless share a very high percentage of their amino acid, and generally their polynucleotide sequence. More importantly, the variants retain the structural and, thus, conformational attributes of the reference sequences disclosed herein.

Methods of determining sequence identity are well known in the art, and are applicable to recombinant RSV soluble F protein polypeptides, as well as the nucleic acids that encode them (e.g., as described below). Various programs and alignment algorithms are described in: Smith and Waterman, Adv. Appl. Math. 2:482, 1981; Needleman and Wunsch, J. Mol. Biol. 48:443, 1970; Higgins and Sharp, Gene 73:237, 1988; Higgins and Sharp, CABIOS 5:151, 1989; Corpet et al., Nucleic Acids Research 16:10881, 1988; and Pearson and Lipman, Proc. Natl. Acad. Sci. USA 85:2444, 1988. Altschul et al., Nature Genet. 6:119, 1994, presents a detailed consideration of sequence alignment methods and homology calculations. The NCBI Basic Local Alignment Search Tool (BLAST) (Altschul et al., J. Mol. Biol. 215:403, 1990) is available from several sources, including the National Center for Biotechnology Information (NCBI, Bethesda, MD) and on the internet, for use in connection with the sequence analysis programs blastp, blastn, blastx, tblastn and tblastx. A description of how to determine sequence identity using this program is available on the NCBI website on the internet.

Also disclosed herein is a recombinant RSV soluble F protein polypeptide having one or more amino acid modification relative to the amino acid sequence of the naturally occurring strain from which it is derived (e.g., in addition to the aforementioned stabilizing modifications). Such differences can be an addition, deletion or substitution of one or more amino acids. A variant typically differs by no more than 20%, such as no more than 15%, 10%, 5%, 2%, or 1% of the amino acid residues. For example, those having deletions of up to 5 (such as 1 or 2) residues at 0-5 locations (such as 0, 1 or 2), insertions of up to 5 residues (such as 1 or 2) at 0-5 five locations (such as 0, 1 or 2) and substitution of up to 20 residues (such as up to 10 residues, in particular up to 5 residues) as compared to the exemplary F1 chain, F2 chain, F0 molecule, and p27 peptide sequences of SEQ ID NOs:1, 2, 3, 6, 7, and 8 that do not alter the conformation or immunogenic epitopes of the resulting recombinant RSV soluble F protein polypeptides. Thus, a variant in the context of recombinant RSV soluble F protein polypeptide, typically shares at least 80%, or 85%, more commonly, at least 90% or more, such as 95%, or even 98% or 99% sequence identity with a reference protein, e.g., the reference sequences illustrated in the exemplary F1 chain, F2 chain, F0 molecule, and p27 peptide sequences of SEQ ID NOs:1, 2, 3, 6, 7, and 8 or any of the exemplary recombinant RSV soluble F protein polypeptide disclosed herein.

In some embodiments of the invention, the antigen composition comprises a trimer of said soluble F protein polypeptides. Thus, in some embodiments, the antigen composition comprises a trimer comprising three identical RSV soluble F protein polypeptides. In some embodiments, the antigen composition comprises a trimer comprising at least one recombinant RSV soluble F protein polypeptide that differs at the sequence level from another recombinant RSV soluble F protein polypeptide member of the trimer. In some embodiments, the antigen composition comprises a trimer of two identical recombinant RSV soluble F protein polypeptides and one recombinant RSV soluble F protein polypeptide that differs at the sequence level from the other two recombinant RSV soluble F protein polypeptides. In some embodiments, the antigen composition comprises three different recombinant RSV soluble F protein polypeptides (in which each recombinant RSV soluble F protein polypeptide differs at the amino acid sequence level).

## NUCLEIC ACIDS THAT ENCODE A RSV SOLUBLE F PROTEIN POLYPEPTIDE; HOST-CELLS COMPRISING SAME; METHODS OF PRODUCING RSV SOLUBLE F PROTEIN POLYPEPTIDES

Another aspect of this disclosure concerns recombinant nucleic acids that encode recombinant RSV soluble F protein polypeptides as described above. In certain embodiments, the recombinant nucleic acids are codon optimized for expression in a selected prokaryotic or eukaryotic host-cell. To facilitate replication and expression, the nucleic acids can be incorporated into a vector, such as a prokaryotic or a eukaryotic expression vector. Host-cells including recombinant RSV soluble F protein polypeptides-encoding nucleic acids are also a feature of this disclosure. Favorable host-cells include prokaryotic (i.e., bacterial) host-cells, such as E. coli, as well as numerous eukaryotic host-cells, including fungal (e.g., yeast) cells, insect cells, and mammalian cells (such as CHO, VERO and HEK293cells).

Exemplary procedures sufficient to guide one of ordinary skill in the art through the production of recombinant RSV soluble F protein polypeptide-encoding nucleic acids can be found in Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2003); and Ausubel et al., Short Protocols in Molecular Biology: A Compendium of Methods from Current Protocols in Molecular Biology, 4th ed., Wiley & Sons, 1999.

Recombinant RSV soluble F protein polypeptides disclosed herein are produced using well established procedures for the expression and purification of recombinant proteins. Procedures sufficient to guide one of skill in the art can be found in the following references: Sambrook et al., Molecular Cloning: A Laboratory Manual, Cold Spring Harbor Laboratory Press, Cold Spring Harbor, NY, 200; and Ausubel et al. Short Protocols in Molecular Biology, 4th ed., John Wiley & Sons, Inc., 999. Additional and specific details are provided hereinbelow. The nucleic acids previously disclosed can suitably be introduced into host-cells and used to produce the recombinant RSV soluble F protein polypeptides described herein.

Host-cells that include recombinant RSV soluble F protein polypeptides-encoding nucleic acids are, thus, also a feature of this disclosure. Favorable host-cells include prokaryotic (*i.e.,* bacterial) host-cells, such as *E. coli,* as well as numerous eukaryotic host-cells, including fungal (*e.g.,* yeast, such as *Saccharomyces cerevisiae* and *Picchia pastoris*) cells, insect cells, plant cells, and mammalian cells (such as CHO and HEK293 cells). Recombinant RSV soluble F protein polypeptides nucleic acids are introduced (*e.g.,* transduced, transformed or transfected) into host-cells, for example, via a vector, such as an expression vector. As described above, the vector is most typically a plasmid, but such vectors can also be, for example, a viral particle, a phage, etc. Examples of appropriate expression hosts include: bacterial cells, such as *E. coli, Streptomyces,* and *Salmonella typhimurium;* fungal cells, such as *Saccharomyces cerevisiae, Pichia pastoris,* and *Neurospora crassa;* insect cells such as *Drosophila* and *Spodoptera frugiperda;* mammalian cells such as 3T3, COS, CHO, BHK, HEK 293 or Bowes melanoma; plant cells, including algae cells, etc.

The host-cells can be cultured in conventional nutrient media modified as appropriate for activating promoters, selecting transformants, or amplifying the inserted polynucleotide sequences. The culture conditions, such as temperature, pH and the like, are typically those previously used with the host-cell selected for expression, and will be apparent to those skilled in the art and in the references cited herein, including, *e.g.,* Freshney (1994) Culture of Animal Cells, a Manual of Basic Technique, third edition, Wiley- Liss, New York and the references cited therein. Expression products corresponding to the nucleic acids of the invention can also be produced in non-animal cells such as plants, yeast, fungi, bacteria and the like. In addition to Sambrook, Berger and Ausubel, details regarding cell culture can be found in Payne et al. (1992) Plant Cell and Tissue Culture in Liquid Systems John Wiley & Sons, Inc. New York, NY; Gamborg and Phillips (eds) (1995) Plant Cell, Tissue and Organ Culture; Fundamental Methods Springer Lab Manual, Springer-Verlag (Berlin Heidelberg New York) and Atlas and Parks (eds) The Handbook of Microbiological Media (1993) CRC Press, Boca Raton, FL.

In bacterial systems, a number of expression vectors can be selected depending upon the use intended for the expressed product. For example, when large quantities of a polypeptide or fragments thereof are needed for the production of antibodies, vectors which direct high level expression of fusion proteins that are readily purified are favorably employed. Such vectors include, but are not limited to, multifunctional *E. coli* cloning and expression vectors such as BLUESCRIPT (Stratagene), in which the coding sequence of interest, *e.g.,* a polynucleotide of the invention as described above, can be ligated into the vector in-frame with sequences for the amino-terminal translation initiating Methionine and the subsequent 7 residues of beta-galactosidase producing a catalytically active beta galactosidase fusion protein; pIN vectors (Van Heeke & Schuster (1989) J Biol Chem 264:5503-5509); pET vectors (Novagen, Madison WI).

Similarly, in yeast, such as *Saccharomyces cerevisiae,* a number of vectors containing constitutive or inducible promoters such as alpha factor, alcohol oxidase and PGH can be used for production of the desired expression products. For reviews, see Berger, Ausubel, and, *e.g.,* Grant et al. (1987; Methods in Enzymology 153:516-544). In mammalian host-cells, a number of expression systems, including both plasmids and viral-based systems, can be utilized.

A host-cell is optionally chosen for its ability to modulate the expression of the inserted sequences or to process the expressed protein in the desired fashion. Such modifications of the protein include, but are not limited to, glycosylation, (as well as, *e.g.,* acetylation, carboxylation, phosphorylation, lipidation and acylation). Post-translational processing for example, which cleaves a precursor form into a mature form of the protein (for example, by a furin protease) is optionally performed in the context of the host-cell. Different host-cells such as 3T3, COS, CHO, HeLa, BHK, MDCK, 293, WI38, etc. have specific cellular machinery and characteristic mechanisms for such post-translational activities and can be chosen to ensure the correct modification and processing of the introduced, foreign protein.

For long-term, high-yield production of recombinant RSV soluble F protein polypeptides disclosed herein, stable expression systems are typically used. For example, cell lines which stably express recombinant RSV soluble F protein polypeptide are introduced into the host-cell using expression vectors which contain viral origins of replication or endogenous expression elements and a selectable marker gene. Following the introduction of the vector, cells are allowed to grow for 1-2 days in an enriched media before they are switched to selective media. The purpose of the selectable marker is to confer resistance to selection, and its presence allows growth and recovery of cells which successfully express the introduced sequences. For example, resistant groups or colonies of stably transformed cells can be proliferated using tissue culture techniques appropriate to the cell type. Host-cells transformed with a nucleic acid encoding a recombinant RSV soluble F protein polypeptide are optionally cultured under conditions suitable for the expression and recovery of the encoded protein from cell culture.

Following transduction of a suitable host-cell line and growth of the host-cells to an appropriate cell density, the selected promoter is induced by appropriate means (e.g., temperature shift or chemical induction) and cells are cultured for an additional period. Optionally, the medium includes components and/or additives that decrease degradation of expressed proteins by proteinases. For example, the medium used for culturing cells to produce recombinant RSV soluble F protein polypeptide can include a protease inhibitor, such as a chelating agent or small molecule inhibitor (*e.g.,* AZ11557272, AS111793, *etc.*), to reduce or eliminate undesired cleavage by cellular, or extracellular (*e.g.,* matrix) proteinases.

The secreted polypeptide product is then recovered from the culture medium. Alternatively, cells can be harvested by depth filtration using primary and secondary filters, centrifugation, disrupted by physical or chemical means, etc., and the resulting crude extract retained for further purification. Eukaryotic or microbial cells employed in expression of proteins can be disrupted by any convenient method, including freeze-thaw cycling, sonication, mechanical disruption, or use of cell lysing agents, or other methods, which are well known to those skilled in the art.

PROCESSES FOR PURIFICATION OF RSV SOLUBLE F PROTEIN POLYPEPTIDES

Also disclosed herein are processes for the manufacture of the antigen compositions described elsewhere herein comprising culturing host-cells comprising a vector encoding the RSV soluble F protein polypeptide to express the RSV soluble F protein polypeptide, then subjecting the expressed RSV soluble F protein polypeptide to a purification process comprising a step of multimodal liquid chromatographic purification utilizing a ligand linked substrate wherein the ligand can interact with proteins via electrostatic interactions, hydrogen bonding, or hydrophobic interactions, or by a combination thereof.

Media for multimodal chromatography are known by those of skill in the art. For instance, multimodal ion exchange ligands may be utilized to interact with the target molecule through multiple types of interactions including ionic interaction, hydrogen bonding and hydrophobic interaction. In some embodiments, the process comprises first loading the RSV soluble F protein polypeptide on said ligand linked substrate in the presence of a loading buffer and second eluting the RSV soluble F protein polypeptide from said ligand linked substrate in the presence of an elution buffer.

In some embodiments, the ligand linked substrate is a multimodal anion exchange resin. In some embodiments, the ligand comprises N-benzyl-N-methyl ethanolamine. Multimodal anion exchange resin comprising N-benzyl-N-methyl ethanolamine is available commercially from GE Healthcare. See Capto adhere Multimodal media instructions, 28-9064-05 AA, available at the internet address www-dot-gehealthcare.com.

In some embodiments, the elution buffer comprises a kosmotropic agent. In some embodiments, the elution buffer comprises a chaotropic agent. Kosmotropic agents include $HPO_4$, citrate, $SO_4$, F, OAc, $CO_2$, $SO_2$, SCN, $HPO_2$, Mg, Li, Zn, Al, tert-butanol, trehalose, glucose, proline, and glutamate. Chaotropic agents include K, Cs, $NH_4$, $(CH_3)_4N$, n-butanol, ethanol, guanidinium chloride, perchlorate, acetate, chloride, bromide, iodide, phenol, 2-propanol, sodium dodecyl sulfate, thiourea, urea, arginine, lysine, and histidine. See, e.g., Kgwu et al., (2004) "The Effect of Buffers on Protein Conformational Stability" Pharmaceutical Technology, 28:86-108.

In some embodiments, the elution buffer comprises both a kosmotropic agent and a chaotropic agent. In such embodiments, counter-acting effects of a kosmotropic agent and a chaotropic agent are applied to the bound substrate. It has been observed that this methodology elutes the desired polypeptide with minimal accompanying undesired host-cell protein. While multimodal anion exchange is used in applications such as the purification of monoclonal antibodies, this is performed in the flow-through mode and/or where the antibody is eluted using a pH gradient buffer, in contrast to the present embodiment wherein a buffer comprising counter-acting kosmotropic agents and chaotropic agents is utilized.

In some embodiments, the elution buffer comprises citrate ions and arginine. In some embodiments, the elution buffer comprises between 150 - 350 mM sodium citrate, 200 - 300 mM sodium citrate, 225 - 275 mM sodium citrate, 230 - 270 mM sodium citrate, 235 -265 mM sodium citrate, 240 - 260 mM sodium citrate, or 245 - 255 mM sodium citrate; and between 200 - 400 mM arginine, 250 - 350 mM arginine, 275 - 325 mM arginine, 280 - 320 mM arginine, 285 - 315 mM arginine, 290 - 310 mM arginine, or 295 - 305 mM arginine. In one embodiment, the elution buffer comprises 250 mM sodium citrate and 300 mM arginine. In some embodiments, the elution buffer further comprises 10-30 mM sodium phosphate, 15-25 mM sodium phosphate, 16-24 mM sodium phosphate, 17-23 mM sodium phosphate, 18-22 mM sodium phosphate, 19-21 mM sodium phosphate. In some embodiments, the elution buffer comprises 20 mM sodium phosphate.

Conductivity of an electrolyte solution is a measure of its ability to conduct electricity. The SI unit of conductivity is siemens per meter (S/m). A millisiemens per centimeter (mS/cm) is a decimal fraction of the SI unit of electrical conductivity siemens per meter. 1 mS/cm = 0.1 S/m. Conductivity measurements are used in routinely in many industrial applications to measure the ionic content in a solution. In some embodiments, the conductivity of the loading buffer may be favorably adjusted to enhance loading of the ligand with the target polypeptide of interest such that the loading buffer has a conductivity of between 20 - 34 mS/cm, 21 - 33 mS/cm, 22 - 32 mS/cm, 23 - 31 mS/cm, 24 - 30 mS/cm, or 25 - 29 mS/cm. In some embodiments, the loading buffer has a conductivity of 25 - 29 mS/cm. In some embodiments, the conductivity of the loading buffer may be favorably adjusted to enhance loading of the ligand with the target polypeptide of interest such that the loading buffer has a conductivity of between between 11 - 34 mS/cm, 13 - 31 mS/cm, 15 - 27 mS/cm, 17 - 25 mS/cm, 19 - 23 mS/cm, or 20 - 22 mS/cm. In some embodiments, the loading buffer has a conductivity of 19-23 mS/cm.

In some embodiments, the loading buffer comprises between 150 - 300 mM, such as between 175 - 275 mM, between 200 - 250 mM, between 210 - 240 mM, between 220 - 230 mM sodium chloride. In some embodiments, the loading buffer comprises 225 mM sodium chloride. In some embodiments, the loading buffer comprises between 100 - 300 mM, such as between 120 - 250 mM, between 140 - 225 mM, between 160 - 200 mM, between 170 - 190 mM sodium chloride. In some embodiments, the loading buffer comprises 180 mM sodium chloride.

In some embodiments, the loading buffer and the elution buffer independently have a pH of between 6.5 - 7.9, 6.6 - 7.8, 6.7 - 7.7, 6.8 - 7.6, 6.9 - 7.5, or 7.0 - 7.4. In some embodiments, the loading buffer and the elution buffer independently have a pH of 7.0-7.4.

In some embodiments, the loading buffer has a pH of between 7.0 - 8.0, 7.1 - 7.9, 7.2 - 7.8, or 7.3 - 7.7. In some embodiments, the loading buffer has a pH of 7.3 - 7.7.

In some embodiments, the elution buffer has a pH of between 6.9 - 7.9, 7.0 - 7.8, 7.1 - 7.7, or 7.2 - 7.6. In some embodiments, the elution buffer has a pH of 7.2 - 7.6.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising a multimodal liquid chromatographic purification utilizing a ligand linked substrate comprising N-benzyl-N-methyl ethanolamine, wherein liquid chromatographic purification involves the steps of (a) loading the liquid medium containing the RSV soluble F protein polypeptide on the substrate in a loading buffer comprising between 10-30 mM, such as between 15-25 mM, between 16-24 mM, between 17-23 mM, between 18-22 mM, between 19-21 mM sodium phosphate; between 150 - 300 mM, such as between 175 - 275 mM, between 200 - 250 mM, between 210 - 240 mM, between 220 - 230 mM sodium chloride; pH between 6.5 - 7.9, such as between 6.6 - 7.8, between 6.7 - 7.7, between 6.8 - 7.6, between 6.9 - 7.5, or between 7.0 - 7.4; and (b) eluting the RSV soluble F protein from the substrate utilizing an elution buffer comprising (i) between 150 - 350 mM, such as between 200 - 300 mM, between 225 - 275 mM, between 230 - 270 mM, between 235 -265 mM, between 240 - 260 mM, or between 245 - 255 mM sodium citrate; (ii) between 200 - 400 mM, such as between 250 - 350 mM, between 275 - 325 mM, between 280 - 320 mM, between 285 - 315 mM, between 290 - 310 mM, or between 295 - 305 mM arginine; and (iii) between 10-30 mM, such as between 15-25 mM, between 16-24 mM, between 17-23 mM, between 18-22 mM, between 19-21 mM sodium phosphate; pH between 6.5 - 7.9, such as between 6.6 - 7.8, between 6.7 - 7.7, between 6.8 - 7.6, between 6.9 - 7.5, or between 7.0 - 7.4.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising a multimodal liquid chromatographic purification utilizing a ligand linked substrate comprising N-benzyl-N-methyl ethanolamine, wherein liquid chromatographic purification involves the steps of (a) loading the liquid medium containing the RSV soluble F protein polypeptide on the substrate in a loading buffer comprising between 10-30 mM, such as between 15-25 mM, between 16-24 mM, between 17-23 mM, between 18-22 mM, between 19-21 mM sodium phosphate; between 100 - 300 mM, such as between 120 - 250 mM, between 140 - 225 mM, between 160 - 200 mM, between 170 - 190 mM sodium chloride sodium chloride; pH between 7.0 - 8.0, such as between 7.1 - 7.9, between 7.2 - 7.8, or between 7.3 - 7.7; and (b) eluting the RSV soluble F protein from the substrate utilizing an elution buffer comprising (i) between 150 - 350 mM, such as between 200 - 300 mM, between 225 - 275 mM, between 230 - 270 mM, between 235 -265 mM, between 240 - 260 mM, or between 245 - 255 mM sodium citrate; (ii) between 200 - 400 mM, such as between 250 - 350 mM, between 275 - 325 mM, between 280 - 320 mM, between 285 - 315 mM, between 290 - 310 mM, or between 295 - 305 mM arginine; and (iii) between 10-30 mM, such as between 15-25 mM, between 16-24 mM, between 17-23 mM, between 18-22 mM, between 19-21 mM sodium phosphate; pH between 6.9 - 7.9, such as between 7.0 - 7.8, between 7.1 - 7.7, or between 7.2 - 7.6.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising a multimodal liquid chromatographic purification utilizing a capto adhere ligand linked substrate comprising N-benzyl-N-methyl ethanolamine, wherein liquid chromatographic purification involves the steps of (a) loading the liquid medium containing the recombinant RSV soluble F protein on the substrate in a loading buffer comprising 20 mM sodium phosphate; 235 mM sodium chloride; pH 7.0-7.4; and (b) eluting the recombinant RSV soluble F protein from the substrate utilizing an elution buffer comprising (i) 250 mM sodium citrate; (ii) 300 mM arginine; and (iii) 20 mM sodium phosphate; pH 7.0-7.4.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising a multimodal liquid chromatographic purification utilizing a capto adhere ligand linked substrate comprising N-benzyl-N-methyl ethanolamine, wherein liquid chromatographic purification involves the steps of (a) loading the liquid medium containing the recombinant RSV soluble F protein on the substrate in a loading buffer comprising 20 mM sodium phosphate; 225 mM sodium chloride; pH 7.0-7.4; and (b) eluting the recombinant RSV soluble F protein from the substrate utilizing an elution buffer comprising (i) 250 mM sodium citrate; (ii) 300 mM arginine; and (iii) 20 mM sodium phosphate; pH 7.0-7.4.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising a multimodal liquid chromatographic purification utilizing a capto adhere ligand linked substrate comprising N-benzyl-N-methyl ethanolamine, wherein liquid chromatographic purification involves the steps of (a) loading the liquid medium containing the recombinant RSV soluble F protein on the substrate in a loading buffer comprising 20 mM sodium phosphate; 180 mM sodium chloride; pH 7.3 - 7.7; and (b) eluting the recombinant RSV soluble F protein from the substrate utilizing an elution buffer comprising (i) 250 mM sodium citrate; (ii) 300 mM arginine; and (iii) 20 mM sodium phosphate; pH 7.2 - 7.6.

In some embodiments, prior to multimodal liquid chromatographic purification the recombinant RSV soluble F protein polypeptide is subject to anion exchange chromatography. In some embodiments, the anion exchange chromatography comprises the steps of (a) loading the recombinant RSV soluble F protein polypeptide on a resin wherein the resin comprises a ligand bearing a quaternary amine group, wherein the resin is equilibrated with a buffer comprising 10-30 mM phosphate and 100-130 mM sodium chloride, pH 6.0-8.0; and (b) eluting the RSV soluble F protein polypeptide from the resin using a buffer comprising 10-30 mM sodium phosphate, 200-250 mM sodium chloride, pH 6.0-8.0. Resin for anion exchange chromatography bearing quaternary amine group is commercially available, see for instance Capto Q ImpRes Ion exchange chromatography data file 28-9837-63AC, available on the internet address www-dot-gelifesciences.com/-bioprocess. In some embodiments, the anion exchange chromatography comprises the steps of (a) loading the recombinant RSV soluble F protein polypeptide on a resin wherein the resin comprises a ligand bearing substrate comprising Capto Q ImpRes substrate comprising a quaternary amine group, wherein the resin is equilibrated with a buffer comprising 20 mM phosphate, 115 mM sodium chloride, pH 7.0-7.4; and (b) eluting the recombinant RSV soluble F protein polypeptide from the resin using a buffer comprising 20 mM sodium phosphate, 225 mM sodium chloride, pH 7.0-7.4.

In some embodiments, prior to multimodal liquid chromatographic purification the recombinant RSV soluble F protein polypeptide is subject to anion exchange chromatography. In some embodiments, the anion exchange chromatography comprises the steps of (a) loading the recombinant RSV soluble F protein polypeptide on a resin wherein the resin comprises a ligand bearing a quaternary amine group, wherein the resin is equilibrated with a buffer comprising 10-30 mM phosphate and 40-120 mM sodium chloride, pH 6.0-8.0; and (b) eluting the RSV soluble F protein polypeptide from the resin using a buffer comprising 10-30 mM sodium phosphate, 130-230 mM sodium chloride, pH 6.0-8.0. Resin for anion exchange chromatography bearing quaternary amine group is commercially available, see for instance Capto Q ImpRes Ion exchange chromatography data file 28-9837-63AC, available on the internet address www-dot-gelifesciences.com/-bioprocess. In some embodiments, the anion exchange chromatography comprises the steps of (a) loading the recombinant RSV soluble F protein polypeptide on a resin wherein the resin comprises a ligand bearing substrate comprising Capto Q ImpRes substrate comprising a quaternary amine group, wherein the resin is equilibrated with a buffer comprising 20 mM phosphate, 80 mM sodium chloride, pH 7.3-7.7; and (b) eluting the recombinant RSV soluble F protein polypeptide from the resin using a buffer comprising 20 mM sodium phosphate, 180 mM sodium chloride, pH 7.3-7.7.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising the steps of (a) loading the liquid medium containing the recombinant RSV soluble F protein polypeptide on a resin wherein the resin comprises a ligand bearing a quaternary amine group, wherein the resin is equilibrated with a buffer comprising 10-30 mM phosphate and 100-130 mM sodium chloride, pH 6.0-8.0; and (b) eluting the recombinant RSV soluble F protein polypeptide from the resin using a buffer comprising 10-30 mM sodium phosphate, 200-250 mM sodium chloride, pH 6.0-8.0; (c) loading the recombinant RSV soluble F protein on a ligand linked substrate comprising N-benzyl-N-methyl ethanolamine in a loading buffer comprising between 10-30 mM, such as between 15-25 mM, between 16-24 mM, between 17-23 mM, between 18-22 mM, between 19-21 mM sodium phosphate; between 150 - 300 mM, such as between 175 - 275 mM, between 200 - 250 mM, between 210 - 240 mM, between 220 - 230 mM sodium chloride; pH between 6.5 - 7.9, such as between 6.6 - 7.8, between 6.7 - 7.7, between 6.8 - 7.6, between 6.9 - 7.5, between 7.0 - 7.4, or between 7.1 - 7.3; and (d) eluting the recombinant RSV soluble F protein from the substrate utilizing an elution buffer comprising (i) between 150 - 350 mM, such as between 200 - 300 mM, between 225 - 275 mM, between 230 - 270 mM, between 235 -265 mM, between 240 - 260 mM, or between 245 - 255 mM sodium citrate; (ii) between 200 - 400 mM, such as between 250 - 350 mM, between 275 - 325 mM, between 280 - 320 mM, between 285 - 315 mM, between 290 - 310 mM, or between 295 - 305 mM arginine; and (iii) between 10-30 mM, such as between 15-25 mM, between 16-24 mM, between 17-23 mM, between 18-22 mM, between 19-21 mM sodium phosphate; pH between 6.5 - 7.9, such as between 6.6 - 7.8, between 6.7 - 7.7, between 6.8 - 7.6, between 6.9 - 7.5, or between 7.0 - 7.4.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising the steps of (a) loading the liquid medium containing the recombinant RSV soluble F protein polypeptide on a resin wherein the resin comprises a ligand bearing a quaternary amine group, wherein the resin is equilibrated with a buffer comprising 10-30 mM phosphate and 40-120 mM sodium chloride, pH 6.0-8.0; and (b) eluting the recombinant RSV soluble F protein polypeptide from the resin using a buffer comprising 10-30 mM sodium phosphate, 130-230 mM sodium chloride, pH 6.0-8.0; (c) loading the recombinant RSV soluble F protein on a ligand linked substrate comprising N-benzyl-N-methyl ethanolamine in a loading buffer comprising between 10-30 mM, such as between 15-25 mM, between 16-24 mM, between 17-23 mM, between 18-22 mM, between 19-21 mM sodium phosphate; between 100 - 300 mM, such as between 120 - 250 mM, between 140 - 225 mM, between 160 - 200 mM, between 170 - 190 mM sodium chloride sodium chloride; pH between 7.0 - 8.0, such as between 7.1 - 7.9, between 7.2 - 7.8, or between 7.3 - 7.7; and (d) eluting the recombinant RSV soluble F protein from the substrate utilizing an elution buffer comprising (i) between 150 - 350 mM, such as between 200 - 300 mM, between 225 - 275 mM, between 230 - 270 mM, between 235 -265 mM, between 240 - 260 mM, or between 245 - 255 mM sodium citrate; (ii) between 200 - 400 mM, such as between 250 - 350 mM, between 275 - 325 mM, between 280 - 320 mM, between 285 - 315 mM, between 290 - 310 mM, or between 295 - 305 mM arginine; and (iii) between 10-30 mM, such as between 15-25 mM, between 16-24 mM, between 17-23 mM, between 18-22 mM, between 19-21 mM sodium phosphate; pH between 6.9 - 7.9, such as between 7.0 - 7.8, between 7.1 - 7.7, or between 7.2 - 7.6.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising the steps of (a) loading the recombinant RSV soluble F protein polypeptide on a resin wherein the resin comprises a ligand bearing substrate comprising Capto Q ImpRes substrate comprising a quaternary amine group, wherein the resin is equilibrated with a buffer comprising 20 mM phosphate, 115 mM sodium chloride, pH 7.0-7.4; (b) eluting the recombinant RSV soluble F protein polypeptide from the resin using a buffer comprising 20 mM sodium phosphate, 225 mM sodium chloride, pH 7.0-7.4.; (c) loading the recombinant RSV soluble F protein on a capto adhere ligand linked substrate comprising N-benzyl-N-methyl ethanolamine in a loading buffer comprising 20 mM sodium phosphate; 225 mM sodium chloride; pH 7.0-7.4; and (d) eluting the recombinant RSV soluble F protein from the substrate utilizing an elution buffer comprising (i) 250 mM sodium citrate; (ii) 300 mM arginine; and (iii) 20 mM sodium phosphate; pH 7.0-7.4.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising the steps of (a) loading the recombinant RSV soluble F protein polypeptide on a resin wherein the resin comprises a ligand bearing substrate comprising Capto Q ImpRes substrate comprising a quaternary amine group, wherein the resin is equilibrated with a buffer comprising 20 mM phosphate, 80 mM sodium chloride, pH 7.3-7.7; (b) eluting the recombinant RSV soluble F protein polypeptide from the resin using a buffer comprising 20 mM sodium phosphate, 180 mM sodium chloride, pH 7.3-7.7; (c) loading the recombinant RSV soluble F protein on a capto adhere ligand linked substrate comprising N-benzyl-N-methyl ethanolamine in a loading buffer comprising 20 mM sodium phosphate; 180 mM sodium chloride; pH 7.3-7.7 and (d) eluting the recombinant RSV soluble F protein from the substrate utilizing an elution buffer comprising (i) 250 mM sodium citrate; (ii) 300 mM arginine; and (iii) 20 mM sodium phosphate; pH 7.2-7.6.

In some embodiments the purification process comprises the steps of (a) loading the recombinant RSV soluble F protein polypeptide on a resin wherein the resin comprises a ligand bearing substrate comprising Capto Q ImpRes substrate comprising a quaternary amine group, wherein the resin is equilibrated with a buffer comprising 20 mM phosphate, 115 mM sodium chloride, pH 7.0-7.4; (b) eluting the recombinant RSV soluble F protein polypeptide from the resin using a buffer comprising 20 mM sodium phosphate, 225 mM sodium chloride, pH 7.0-7.4.; (c) loading the recombinant RSV soluble F protein on a capto adhere ligand linked substrate comprising N-benzyl-N-methyl ethanolamine in a loading buffer comprising 20 mM sodium phosphate; 225 mM sodium chloride; pH 7.0-7.4; and (d) eluting the recombinant RSV soluble F protein from the substrate utilizing an elution buffer comprising (i) 250 mM sodium citrate; (ii) 300 mM arginine; and (iii) 20 mM sodium phosphate; pH 7.0-7.4.

In some embodiments the purification process comprises the steps of (a) loading the recombinant RSV soluble F protein polypeptide on a resin wherein the resin comprises a ligand bearing substrate comprising Capto Q ImpRes substrate comprising a quaternary amine group, wherein the resin is equilibrated with a buffer comprising 20 mM phosphate, 80 mM sodium chloride, pH 7.3-7.7; (b) eluting the recombinant RSV soluble F protein polypeptide from the resin using a buffer comprising 20 mM sodium phosphate, 180 mM sodium chloride, pH 7.3-7.7; (c) loading the recombinant RSV soluble F protein on a capto adhere ligand linked substrate comprising N-benzyl-N-methyl ethanolamine in a loading buffer comprising 20 mM sodium phosphate; 180 mM sodium chloride; pH 7.3-7.7; and (d) eluting the recombinant RSV soluble F protein from the substrate utilizing an elution buffer comprising (i) 250 mM sodium citrate; (ii) 300 mM arginine; and (iii) 20 mM sodium phosphate; pH 7.2-7.6.

In some embodiments, prior to the step of anion exchange chromatography the recombinant RSV soluble F protein polypeptide is subject to a step of affinity-like chromatography comprising the steps of (a) loading the recombinant RSV soluble F protein polypeptide on a media comprising dextran sulfate wherein the resin is equilibrated with 40-60 mM sodium phosphate at pH 6.0-8.0; and (b) eluting the recombinant RSV soluble F protein polypeptide from the media using a buffer comprising 10-30 mM sodium phosphate, 200-270 mM sodium chloride, pH 6.0-8.0.

In some embodiments, prior to the step of anion exchange chromatography the recombinant RSV soluble F protein polypeptide is subject to a step of affinity-like chromatography comprising the steps of (a) loading the recombinant RSV soluble F protein polypeptide on a media comprising dextran sulfate wherein the resin is equilibrated with 30-60 mM sodium phosphate at pH 6.0-8.0; and (b) eluting the recombinant RSV soluble F protein polypeptide from the media using a buffer comprising 10-30 mM sodium phosphate, 200-270 mM sodium chloride, pH 6.0-8.0.

Media for affinity-like chromatography comprising dextran sulfate is commercially available, see for instance Capto DeVirS Affinity chromatography data file 28-9616-49 AA, available at the internet address www-dot-gelifesciences.com/bioprocess. In some embodiments, the affinity-like chromatography comprises the steps of (a) loading the recombinant RSV soluble F protein polypeptide on a Capto DeVirS media comprising dextran sulfate wherein the resin is equilibrated with 47 mM sodium phosphate, pH 7.0-7.4; and (b) eluting the recombinant RSV soluble F protein polypeptide from the media using a buffer comprising 20 mM sodium phosphate, 235 mM sodium chloride, at pH 7.0-7.4.

In some embodiments, the affinity-like chromatography comprises the steps of (a) loading the recombinant RSV soluble F protein polypeptide on a Capto DeVirS media comprising dextran sulfate wherein the resin is equilibrated with 40 mM sodium phosphate, pH 7.1-7.5; and (b) eluting the recombinant RSV soluble F protein polypeptide from the media using a buffer comprising 20 mM sodium phosphate, 240 mM sodium chloride, at pH 7.2-7.6 .

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising the steps of (a) loading the recombinant RSV soluble F protein polypeptide on a media comprising dextran sulfate wherein the resin is equilibrated with 40-60 mM sodium phosphate at pH 6.0-8.0; (b) eluting the recombinant RSV soluble F protein polypeptide from the media using a buffer comprising 10-30 mM sodium phosphate, 200-270 mM sodium chloride, pH 6.0-8.0; (c) loading the recombinant RSV soluble F protein polypeptide on a resin wherein the resin comprises a ligand bearing a quaternary amine group, wherein the resin is equilibrated with a buffer comprising 10-30 mM phosphate and 100-130 mM sodium chloride, pH 6.0-8.0; (d) eluting the recombinant RSV soluble F protein polypeptide from the resin using a buffer comprising 10-30 mM sodium phosphate, 200-250 mM sodium chloride, pH 6.0-8.0; (e) loading the recombinant RSV soluble F protein on a ligand linked substrate comprising N-benzyl-N-methyl ethanolamine in a loading buffer comprising between 10-30 mM, such as between 15-25 mM, between 16-24 mM, between 17-23 mM, between 18-22 mM, between 19-21 mM sodium phosphate; between 150 - 300 mM, such as between 175 - 275 mM, between 200 - 250 mM, between 210 - 240 mM, between 220 - 230 mM sodium chloride; pH between 6.5 - 7.9, such as between 6.6 - 7.8, between 6.7 - 7.7, between 6.8 - 7.6, between 6.9 - 7.5, or between 7.0 - 7.4; and (f) eluting the recombinant RSV soluble F protein from the substrate utilizing an elution buffer comprising (i) between 150 - 350 mM, such as between 200 - 300 mM, between 225 - 275 mM, between 230 - 270 mM, between 235 -265 mM, between 240 - 260 mM, or between 245 - 255 mM sodium citrate; (ii) between 200 - 400 mM, such as between 250 - 350 mM, between 275 - 325 mM, between 280 - 320 mM, between 285 - 315 mM, between 290 - 310 mM, or between 295 - 305 mM arginine; and (iii) between 10-30 mM, such as between 15-25 mM, between 16-24 mM, between 17-23 mM, between 18-22 mM, between 19-21 mM sodium phosphate; pH between 6.5 - 7.9, such as between 6.6 - 7.8, between 6.7 - 7.7, between 6.8 - 7.6, between 6.9 - 7.5, or between 7.0 - 7.4.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising the steps of (a) loading the recombinant RSV soluble F protein polypeptide on a media comprising dextran sulfate wherein the resin is equilibrated with 30-60 mM sodium phosphate at pH 6.0-8.0; (b) eluting the recombinant RSV soluble F protein polypeptide from the media using a buffer comprising 10-30 mM sodium phosphate, 200-270 mM sodium chloride, pH 6.0-8.0; (c) loading the recombinant RSV soluble F protein polypeptide on a resin wherein the resin comprises a ligand bearing a quaternary amine group, wherein the resin is equilibrated with a buffer comprising 10-30 mM phosphate and 40-120 mM sodium chloride, pH 6.0-8.0; (d) eluting the recombinant RSV soluble F protein polypeptide from the resin using a buffer comprising 10-30 mM sodium phosphate, 130-230 mM sodium chloride, pH 6.0-8.0; (e) loading the recombinant RSV soluble F protein on a ligand linked substrate comprising N-benzyl-N-methyl ethanolamine in a loading buffer comprising between 10-30 mM, such as between 15-25 mM, between 16-24 mM, between 17-23 mM, between 18-22 mM, between 19-21 mM sodium phosphate; between 100 - 300 mM, such as between 120 - 250 mM, between 140 - 225 mM, between 160 - 200 mM, between 170 - 190 mM sodium chloride sodium chloride; pH between 7.0 - 8.0, such as between 7.1 - 7.9, between 7.2 - 7.8, or between 7.3 - 7.7; and (f) eluting the recombinant RSV soluble F protein from the substrate utilizing an elution buffer comprising (i) between 150 - 350 mM, such as between 200 - 300 mM, between 225 - 275 mM, between 230 - 270 mM, between 235 -265 mM, between 240 - 260 mM, or between 245 - 255 mM sodium citrate; (ii) between 200 - 400 mM, such as between 250 - 350 mM, between 275 - 325 mM, between 280 - 320 mM, between 285 - 315 mM, between 290 - 310 mM, or between 295 - 305 mM arginine; and (iii) between 10-30 mM, such as between 15-25 mM, between 16-24 mM, between 17-23 mM, between 18-22 mM, between 19-21 mM sodium phosphate; pH between 6.9 - 7.9, such as between 7.0 - 7.8, between 7.1 - 7.7, or between 7.2 - 7.6.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising the steps of (a) loading the recombinant RSV soluble F protein polypeptide on a Capto DeVirS media comprising dextran sulfate wherein the resin is equilibrated with 47 mM sodium phosphate, pH 7.0-7.4; and (b) eluting the recombinant RSV soluble F protein polypeptide from the media using a buffer comprising 20 mM sodium phosphate, 235 mM sodium chloride, at pH 7.0-7.4; (c) loading the recombinant RSV soluble F protein polypeptide on a resin wherein the resin comprises a ligand bearing substrate comprising Capto Q ImpRes substrate comprising a quaternary amine group, wherein the resin is equilibrated with a buffer comprising 20 mM phosphate, 115 mM sodium chloride, pH 7.0-7.4; (d) eluting the recombinant RSV soluble F protein polypeptide from the resin using a buffer comprising 20 mM sodium phosphate, 225 mM sodium chloride, pH 7.0-7.4.; (e) loading the recombinant RSV soluble F protein on a capto adhere ligand linked substrate comprising N-benzyl-N-methyl ethanolamine in a loading buffer comprising 20 mM sodium phosphate; 225 mM sodium chloride; pH 7.0-7.4; and (f) eluting the recombinant RSV soluble F protein from the substrate utilizing an elution buffer comprising (i) 250 mM sodium citrate; (ii) 300 mM arginine; and (iii) 20 mM sodium phosphate; pH 7.0-7.4.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising the steps of (a) loading the recombinant RSV soluble F protein polypeptide on a Capto DeVirS media comprising dextran sulfate wherein the resin is equilibrated with 40 mM sodium phosphate, pH 7.1-7.5; and (b) eluting the recombinant RSV soluble F protein polypeptide from the media using a buffer comprising 20 mM sodium phosphate, 240 mM sodium chloride, at pH 7.2-7.6; (c) loading the recombinant RSV soluble F protein polypeptide on a resin wherein the resin comprises a ligand bearing substrate comprising Capto Q ImpRes substrate comprising a quaternary amine group, wherein the resin is equilibrated with a buffer comprising 20 mM phosphate, 80 mM sodium chloride, pH 7.3-7.7; (d) eluting the recombinant RSV soluble F protein polypeptide from the resin using a buffer comprising 20 mM sodium phosphate, 180 mM sodium chloride, pH 7.3-7.7; (e) loading the recombinant RSV soluble F protein on a capto adhere ligand linked substrate comprising N-benzyl-N-methyl ethanolamine in a loading buffer comprising 20 mM sodium phosphate; 180 mM sodium chloride; pH 7.3-7.7; and (f) eluting the recombinant RSV soluble F protein from the substrate utilizing an elution buffer comprising (i) 250 mM sodium citrate; (ii) 300 mM arginine; and (iii) 20 mM sodium phosphate; pH 7.2-7.6.

In some embodiments, prior to the step of affinity-like chromatography the recombinant RSV soluble F protein polypeptide is subject to a step of virus inactivation. In some embodiments, after the step of affinity-like chromatography the recombinant RSV soluble F protein polypeptide is subject to a step of virus inactivation. In some embodiments, the step of virus inactivation comprises adding a detergent. In some embodiments, the step of virus inactivation comprises adding polysorbate-80 to the liquid medium comprising the recombinant RSV soluble F protein polypeptide to a concentration of 0.5-2.5 % (e.g., 0.5-1.5%) and incubating said liquid medium for 10-25 hours at a temperature of 15-25 °C. In some embodiments, the step of virus inactivation comprises adding polysorbate-80 to the liquid medium comprising the recombinant RSV soluble F protein polypeptide to a concentration of 0.5-1.5 % and incubating said liquid medium for 10-25 hours at a temperature of 15-25 °C. In some embodiments, the step of virus inactivation comprises adding polysorbate-80 to the liquid medium and adjusting the concentration to 1.0 % and incubating said liquid medium for 15-20 hours at controlled room temperature. In some embodiments, the step of virus inactivation comprises adding polysorbate-80 to the liquid medium and adjusting the concentration to 2.0 % and incubating said liquid medium for 18-24 hours at controlled room temperature.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising the steps of (a) adding polysorbate-80 to the liquid medium comprising the recombinant RSV soluble F protein polypeptide to a concentration of 0.5-1.5 % and incubating said liquid medium for 10-25 hours at a temperature of 15-25 °C; (b) loading the recombinant RSV soluble F protein polypeptide on a media comprising dextran sulfate wherein the resin is equilibrated with 40-60 mM sodium phosphate at pH 6.0-8.0; (c) eluting the recombinant RSV soluble F protein polypeptide from the media using a buffer comprising 10-30 mM sodium phosphate, 200-270 mM sodium chloride, pH 6.0-8.0; (d) loading the recombinant RSV soluble F protein polypeptide on a resin wherein the resin comprises a ligand bearing a quaternary amine group, wherein the resin is equilibrated with a buffer comprising 10-30 mM phosphate and 100-130 mM sodium chloride, pH 6.0-8.0; and (e) eluting the recombinant RSV soluble F protein polypeptide from the resin using a buffer comprising 10-30 mM sodium phosphate, 200-250 mM sodium chloride, pH 6.0-8.0; (f) loading the recombinant RSV soluble F protein on a ligand linked substrate comprising N-benzyl-N-methyl ethanolamine in a loading buffer comprising between 10-30 mM, such as between 15-25 mM, between 16-24 mM, between 17-23 mM, between 18-22 mM, between 19-21 mM sodium phosphate; between 150 - 300 mM, such as between 175 - 275 mM, between 200 - 250 mM, between 210 - 240 mM, between 220 - 230 mM sodium chloride; pH between 6.5 - 7.9, such as between 6.6 - 7.8, between 6.7 - 7.7, between 6.8 - 7.6, between 6.9 - 7.5, or between 7.0 - 7.4; and (g) eluting the recombinant RSV soluble F protein from the substrate utilizing an elution buffer comprising (i) between 150 - 350 mM, such as between 200 - 300 mM, between 225 - 275 mM, between 230 - 270 mM, between 235 -265 mM, between 240 - 260 mM, or between 245 - 255 mM sodium citrate; (ii) between 200 - 400 mM, such as between 250 - 350 mM, between 275 - 325 mM, between 280 - 320 mM, between 285 - 315 mM, between 290 - 310 mM, or between 295 - 305 mM arginine; and (iii) between 10-30 mM, such as between 15-25 mM, between 16-24 mM, between 17-23 mM, between 18-22 mM, between 19-21 mM sodium phosphate; pH between 6.5 - 7.9, such as between 6.6 - 7.8, between 6.7 - 7.7, between 6.8 - 7.6, between 6.9 - 7.5, or between 7.0 - 7.4. In some embodiments, the multimodal liquid chromatography purification is followed by virus filtration. In some embodiments, the virus filtration is followed by a step of ultrafiltration/-diafiltration.

Also disclosed herein are processes for the manufacture of the antigen compositions described elsewhere herein comprising culturing host-cells comprising a vector encoding the recombinant RSV soluble F protein polypeptide to express the recombinant RSV soluble F protein polypeptide and subjecting the expressed recombinant RSV soluble F protein polypeptide to a purification process comprising the steps of (a) loading the recombinant RSV soluble F protein polypeptide on a media comprising dextran sulfate wherein the resin is equilibrated with 30-60 mM sodium phosphate at pH 6.0-8.0; (b) eluting the recombinant RSV soluble F protein polypeptide from the media using a buffer comprising 10-30 mM sodium phosphate, 200-270 mM sodium chloride, pH 6.0-8.0; (c) adding polysorbate-80 to the liquid medium comprising the recombinant RSV soluble F protein polypeptide to a concentration of 0.5-2.5 % and incubating said liquid medium for 10-25 hours at a temperature of 15-25 °C; (d) loading the recombinant RSV soluble F protein polypeptide on a resin wherein the resin comprises a ligand bearing a quaternary amine group, wherein the resin is equilibrated with a buffer comprising 10-30 mM phosphate and 40-120 mM sodium chloride, pH 6.0-8.0; and (e) eluting the recombinant RSV soluble F protein polypeptide from the resin using a buffer comprising 10-30 mM sodium phosphate, 130-230 mM sodium chloride, pH 6.0-8.0; (f) loading the recombinant RSV soluble F protein on a ligand linked substrate comprising N-benzyl-N-methyl ethanolamine in a loading buffer comprising between 10-30 mM, such as between 15-25 mM, between 16-24 mM, between 17-23 mM, between 18-22 mM, between 19-21 mM sodium phosphate; between 100 - 300 mM, such as between 120 - 250 mM, between 140 - 225 mM, between 160 - 200 mM, between 170 - 190 mM sodium chloride sodium chloride; pH between 7.0 - 8.0, such as between 7.1 - 7.9, between 7.2 - 7.8, or between 7.3 - 7.7; and (g) eluting the recombinant RSV soluble F protein from the substrate utilizing an elution buffer comprising (i) between 150 - 350 mM, such as between 200 - 300 mM, between 225 - 275 mM, between 230 - 270 mM, between 235 -265 mM, between 240 - 260 mM, or between 245 - 255 mM sodium citrate; (ii) between 200 - 400 mM, such as between 250 - 350 mM, between 275 - 325 mM, between 280 - 320 mM, between 285 - 315 mM, between 290 - 310 mM, or between 295 - 305 mM arginine; and (iii) between 10-30 mM, such as between 15-25 mM, between 16-24 mM, between 17-23 mM, between 18-22 mM, between 19-21 mM sodium phosphate; pH between 6.9 - 7.9, such as between 7.0 - 7.8, between 7.1 - 7.7, or between 7.2 - 7.6. In some embodiments, the multimodal liquid chromatography purification is followed by virus filtration. In some embodiments, the virus filtration is followed by a step of ultrafiltration/diafiltration.

Also disclosed herein are processes for the manufacture of the antigen compositions described elsewhere herein comprising culturing host-cells comprising a vector encoding the RSV soluble F protein polypeptide to express the recombinant RSV soluble F protein polypeptide and subjecting the expressed recombinant RSV soluble F protein polypeptide to a purification process comprising the steps of (a) adding polysorbate-80 to the liquid medium and adjusting the concentration to 1.0 % and incubating said liquid medium for 15-20 hours at controlled room temperature (b) loading the recombinant RSV soluble F protein polypeptide on a Capto DeVirS media comprising dextran sulfate wherein the resin is equilibrated with 47 mM sodium phosphate, pH 7.0-7.4; (c) eluting the recombinant RSV soluble F protein polypeptide from the media using a buffer comprising 20 mM sodium phosphate, 235 mM sodium chloride, at pH 7.0-7.4 ; (d) loading the recombinant RSV soluble F protein polypeptide on a resin wherein the resin comprises a ligand bearing substrate comprising Capto Q ImpRes substrate comprising a quaternary amine group, wherein the resin is equilibrated with a buffer comprising 20 mM phosphate, 115 mM sodium chloride, pH 7.0-7.4; (e) eluting the recombinant RSV soluble F protein polypeptide from the resin using a buffer comprising 20 mM sodium phosphate, 225 mM sodium chloride, pH 7.0-7.4.; (f) loading the recombinant RSV soluble F protein on a capto adhere ligand linked substrate comprising N-benzyl-N-methyl ethanolamine in a loading buffer comprising 20 mM sodium phosphate; 225 mM sodium chloride; pH 7.0-7.4; and (g) eluting the recombinant RSV soluble F protein from the substrate utilizing an elution buffer comprising (i) 250 mM sodium citrate; (ii) 300 mM arginine; and (iii) 20 mM sodium phosphate; pH 7.0-7.4. In some embodiments, the multimodal liquid chromatography purification using a ligand linked substrate comprising N-benzyl-N-methyl ethanolamine is followed by virus filtration comprising the further steps of pre-filtration with a 0.1 μm membrane and filtration using a 19 nm membrane. In some embodiments, the virus filtration is followed by a step of ultrafiltration/diafiltration utilizing a 50 kDa molecular weight cutoff membrane and a diafiltration buffer comprising 10 mM potassium phosphate, 5% trehalose. In some embodiments, the step of ultrafiltration/diafiltration is followed by a step of adding polysorbate 80 to a concentration of 0.05%. In some embodiments, the step of adding polysorbate 80 is followed by a step of filtration utilizing a filter having a cutoff of about 0.2 μm or smaller.

Also disclosed herein are processes for the manufacture of the antigen compositions described elsewhere herein comprising culturing host-cells comprising a vector encoding the RSV soluble F protein polypeptide to express the recombinant RSV soluble F protein polypeptide and subjecting the expressed recombinant RSV soluble F protein polypeptide to a purification process comprising the steps of (a) loading the recombinant RSV soluble F protein polypeptide on a Capto DeVirS media comprising dextran sulfate wherein the resin is equilibrated with 40 mM sodium phosphate, pH 7.1-7.5; (b) eluting the recombinant RSV soluble F protein polypeptide from the media using a buffer comprising 20 mM sodium phosphate, 240 mM sodium chloride, at pH 7.2-7.6 (c) adding polysorbate-80 to the liquid medium and adjusting the concentration to 2.0 % and incubating said liquid medium for 18-24 hours at controlled room temperature; (d) loading the recombinant RSV soluble F protein polypeptide on a resin wherein the resin comprises a ligand bearing substrate comprising Capto Q ImpRes substrate comprising a quaternary amine group, wherein the resin is equilibrated with a buffer comprising 20 mM phosphate, 80 mM sodium chloride, pH 7.3-7.7; (e) eluting the recombinant RSV soluble F protein polypeptide from the resin using a buffer comprising 20 mM sodium phosphate, 180 mM sodium chloride, pH 7.3-7.7; (f) loading the recombinant RSV soluble F protein on a capto adhere ligand linked substrate comprising N-benzyl-N-methyl ethanolamine in a loading buffer comprising 20 mM sodium phosphate; 180 mM sodium chloride; pH 7.3-7.7; and (g) eluting the recombinant RSV soluble F protein from the substrate utilizing an elution buffer comprising (i) 250 mM sodium citrate; (ii) 300 mM arginine; and (iii) 20 mM sodium phosphate; pH 7.2-7.6.

In some embodiments, the multimodal liquid chromatography purification using a ligand linked substrate comprising N-benzyl-N-methyl ethanolamine is followed by virus filtration comprising the further steps of pre-filtration with a 0.1 μm membrane and filtration using a 19 nm membrane. In some embodiments, the multimodal liquid chromatography purification using a ligand linked substrate comprising N-benzyl-N-methyl ethanolamine is followed by virus filtration comprising the further steps of pre-filtration with a 0.1 μm membrane and filtration using a 20 nm membrane. In some embodiments, the virus filtration is followed by a step of ultrafiltration/diafiltration utilizing a 50 kDa molecular weight cutoff membrane and a diafiltration buffer comprising 10 mM potassium phosphate, 4% trehalose. In some embodiments, the step of ultrafiltration/diafiltration is followed by a step of adding polysorbate 80 to a concentration of 0.05%. In some embodiments, the step of adding polysorbate 80 is followed by a step of filtration utilizing a filter having a cutoff of about 0.2 μm or smaller.

In some embodiments, the processes described above are followed by a step of lyophilizing the recombinant RSV soluble F protein polypeptide. Methods for the lyophilization of recombinant proteins are known, see, e.g., Hsu et al. (1992) "Determining the optimum residual moisture in lyophilized protein pharmaceuticals," Developments in Biological Standardization 74:255-70; Costantine et al. (1998) "Effect of excipients on the stability and structure of lyophilized recombinant human growth hormone," J. Pharm. Sci. 87:1412-1420.

Also disclosed herein is a method of separating the population of trimers described above into a population of trimers wherein the majority of trimers comprise at least one F' protomer and a population of trimers wherein the majority of trimers comprise 3 F protomers, said method comprising a step of Capto MMC ImpRes chromatography. In some embodiments, the step of Capto MMC ImpRes chromatography utilizes NaCl gradient from 0 to 250 mM, 20mM Sodium Phosphate, 1M NaCitrate. In some embodiments, the sodium chloride gradient is carried out over in 20 column volumes.

Also disclosed herein is a method for separating the population of trimers, as described above, into a population of trimers comprising at least one F' protomer and a population of trimers comprising 3 F protomers, said method comprising a step of weak cation exchange liquid chromatography. In some embodiments, the step of weak cation exchange liquid chromatography utilizes a sodium chloride gradient from 0 mM NaCl to 400 mM NaCl to elute the trimer populations. In some embodiments, the sodium chloride gradient is carried out over 25 minutes at a flow rate of 1 mL/min. In some embodiments, the weak cation exchange is carried out using a first mobile phase comprising 100 mM sodium Phosphate monobasic, a second mobile phase comprising 100 mM sodium Phosphate dibasic, a third mobile phase comprising 1 M sodium chloride, and a fourth mobile phase of water. These methods may be suitably carried out on commercially available media and instruments, such as ProPac WCX-10 Column from ThermoFisher (available on the internet at assets.thermofisher.com/TFS-Assets/CMD/manuals/br-21689-propac-vcx-tips-tricks-br21689-en.pdf) using a Waters Acquity UPLC instrument, following the manufacturer's instructions.

IMMUNOGENIC COMPOSITIONS AND METHODS

Also provided are immunogenic compositions comprising an antigen composition of the invention comprising a recombinant RSV soluble F protein polypeptide and a pharmaceutically acceptable carrier or excipient.

Pharmaceutically acceptable carriers and excipients are well known and can be selected by those of skill in the art. For example, the carrier or excipient can favorably include a buffer. Optionally, the carrier or excipient also contains at least one component that stabilizes solubility and/or stability. Examples of solubilizing/stabilizing agents include detergents, for example, laurel sarcosine and/or tween. Alternative solubilizing/stabilizing agents include arginine, and glass forming polyols (such as sucrose, trehalose and the like). Numerous pharmaceutically acceptable carriers and/or pharmaceutically acceptable excipients are known in the art and are described, e.g., in Remington's Pharmaceutical Sciences, by E. W. Martin, Mack Publishing Co., Easton, PA, 5th Edition (975).

Accordingly, suitable excipients and carriers can be selected by those of skill in the art to produce a formulation suitable for delivery to a subject by a selected route of administration. Suitable excipients include, without limitation: glycerol, Polyethylene glycol (PEG), Sorbitol, Trehalose, N-lauroylsarcosine sodium salt, L -proline, Non detergent sulfobetaine, Guanidine hydrochloride, Urea, Trimethylamine oxide, KCl, $Ca2+$, $Mg2+$, $Mn2+$, $Zn2+$ and other divalent cation related salts, Dithiothreitol, Dithioerytrol, and ß-mercaptoethanol. Other excipients can be detergents (including: Tween80, Tween20, Triton X-00, NP-40, Empigen BB, Octylglucoside, Lauroyl maltoside, Zwittergent 3-08, Zwittergent 3-0, Zwittergent 3-2, Zwittergent 3-4, Zwittergent 3-6, CHAPS, Sodium deoxycholate, Sodium dodecyl sulphate, Cetyltrimethylammonium bromide). In some embodiments, the immunogenic composition comprises the recombinant RSV soluble F protein polypeptide, 10 mM potassium phosphate, 5% trehalose, 0.05% polysorbate 80, at pH 7.0. In some embodiments, the immunogenic composition comprises the recombinant RSV soluble F protein polypeptide, 10 mM potassium phosphate, 4% trehalose, 0.05% polysorbate 80, at pH 6.8.

As mentioned elsewhere herein, a step of lyophilization may be applied to the recombinant RSV soluble F protein polypeptide. Thus, in some embodiments, lyophilized immunogenic compositions are provided.

Optionally, the immunogenic compositions also include an adjuvant. In the context of an immunogenic composition suitable for administration to a subject for the purpose of eliciting a protective immune response against RSV, the adjuvant is selected to elicit a Th1 biased immune response.

The adjuvant is typically selected to enhance a Th1 biased immune response in the subject, or population of subjects, to whom the composition is administered. For example, when the immunogenic composition is to be administered to a subject of a particular age group susceptible to (or at increased risk of) RSV infection, the adjuvant is selected to be safe and effective in the subject or population of subjects. Thus, when formulating an immunogenic composition containing a RSV soluble F protein polypeptide composition for administration in an older adult subject (such as a subject at least 50 years of age), the adjuvant is selected to be safe and effective in older adult subjects. Similarly, when the immunogenic composition is intended for administration in neonatal or infant subjects (such as subjects between birth and the age of two years), the adjuvant is selected to be safe and effective in neonates and infants.

Additionally, the adjuvant is typically selected to enhance a Th1 immune response when administered via a route of administration, by which the immunogenic composition is administered. For example, when formulating an immunogenic for nasal administration, proteosome and protollin are favorable Th1-biasing adjuvants. In contrast, when the immunogenic composition is formulated for intramuscular administration, adjuvants including one or more TLR 4 agonists (e.g. 3D-MPL), saponins (e.g., QS21), liposomes, and/or oil and water emulsions are favorably selected. The adjuvant may be formulated separately from the immunogenic composition, though for convenience is typically co-formulated.

When the adjuvant is formulated separately from the immunogenic composition as a liquid and the immunogenic composition is lyophilized, the liquid adjuvant may be utilized to reconstitute the lyophilized immunogenic composition. In some embodiments, methods comprising a step of reconstituting the immunogenic composition comprising a recombinant RSV soluble F protein polypeptide in a liquid adjuvant are provided.

An adjuvant of interest comprises a TLR4 agonist and a saponin in liposomal formulation.

A suitable example of a TLR4 agonist is a lipopolysaccharide, suitably a nontoxic derivative of lipid A, particularly a monophosphoryl lipid A and more particularly 3-de-O-acylated monophosphoryl lipid A (3D-MPL).

3D-MPL is sold under the name 'MPL' by GlaxoSmithKline Biologicals N.A. and is referred throughout the document as 3D-MPL. See, for example, US Patent Nos. 4,436,727; 4,877,611; 4,866,034 and 4,912,094. 3D-MPL can be produced according to the methods described in GB 2 220 211 A. Chemically it is a mixture of 3-deacylated monophosphoryl lipid A with 4, 5 or 6 acylated chains. In the context of the present invention small particle 3D-MPL may be used to prepare the aqueous adjuvant composition. Small particle 3D-MPL has a particle size such that it may be sterile-filtered through a 0.22 um filter. Such preparations are described in WO94/21292.

Other TLR4 agonists which can be used are alkyl glucosaminide phosphates (AGPs) such as those described in WO98/50399 or US patent No. 6,303,347 (processes for preparation of AGPs are also described). Some AGPs are TLR4 agonists, and some are TLR4 antagonists.

Other TLR4 agonists which may be of use in the immunogenic compositions described herein include Glucopyranosyl Lipid Adjuvant (GLA) such as described in WO2008/153541 or WO2009/143457 or the literature articles Coler RN et al. (2011) Development and Characterization of Synthetic Glucopyranosyl Lipid Adjuvant System as a Vaccine Adjuvant. PLoS ONE 6(1): e16333. doi:10.1371/journal.pone.0016333 and Arias MA et al. (2012) Glucopyranosyl Lipid Adjuvant (GLA), a Synthetic TLR4 Agonist, Promotes Potent Systemic and Mucosal Responses to Intranasal Immunization with HIVgp140. PLoS ONE 7(7): e41144. doi:10.1371/journal.pone.0041144. WO2008/153541 or WO2009/143457 are incorporated herein by reference for the purpose of defining TLR4 agonists which may be of use in the present invention.

Typically the TLR4 agonist, such as the lipopolysaccharide and in particular 3D-MPL, is at least 90% pure, such as at least 95% pure, especially at least 98% pure, in particular 99% pure.

A suitable saponin for use in the present invention is Quil A and its derivatives. Quil A is a saponin preparation isolated from the South American tree *Quillaja saponaria* Molina and was first described as having adjuvant activity by Dalsgaard et al. in 1974 ("Saponin adjuvants", Archiv. für die gesamte Virusforschung, Vol. 44, Springer Verlag, Berlin, p243-254). Purified fractions of Quil A have been isolated by HPLC which retain adjuvant activity without the toxicity associated with Quil A (see, for example, EP0362278). Fractions of general interest include QS7, QS17, QS18 and QS-21, for example QS7 and QS-21 (also known as QA7 and QA21). QS-21 is a saponin of particular interest.

Typically the saponin, such as Quil A and in particular QS-21, is at least 90% pure, such as at least 95% pure, especially at least 98% pure, in particular 99% pure.

A beneficial feature is that the saponin is presented in a less reactogenic composition where it is quenched with an exogenous sterol, such as cholesterol. Suitably the ratio of saponin:sterol (e.g. QS21:cholesterol) is from 1:100 to 1:1 w/w, such as from 1:10 to 1:1 w/w, e.g. from 1:5 to 1:1 w/w.

Liposome size may vary from 30 nm to several um depending on the phospholipid composition and the method used for their preparation.

The liposomes may be formed from many different lipids, although desirably contain DOPC, or, consist essentially of DOPC and sterol (with saponin and LTR4 agonist as appropriate).

In the present invention, the liposome size will be in the range of 50 nm to 200 nm, especially 60 nm to 180 nm, such as 70-165 nm. Optimally, the liposomes should be stable and have a diameter of ~100 nm to allow convenient sterilization by filtration.

Structural integrity of the liposomes may be assessed by methods such as dynamic light scattering (DLS) measuring the size (Z-average diameter, Zav) and polydispersity of the liposomes, or, by electron microscopy for analysis of the structure of the liposomes. In one embodiment the average particle size is between 95 and 120 nm, and/or, the polydispersity (Pdl) index is not more than 0.35, in particular not more than 0.3, such as not more than 0.2.

Optionally, the adjuvant can also include mineral salts such as an aluminium or calcium salts, in particular aluminium hydroxide, aluminium phosphate and calcium phosphate. For example, an adjuvant containing 3D-MPL in combination with an aluminium salt (e.g., aluminium hydroxide or "alum") is suitable for formulation in an immunogenic composition for administration to a human subject.

Combinations of different adjuvants, such as those mentioned hereinabove, can also be used in compositions with a RSV soluble F protein polypeptide composition. For example, as already noted, QS21 can be formulated together with 3D-MPL. The ratio of QS21 : 3D-MPL will typically be in the order of 1 : 10 to 10 : 1; such as 1:5 to 5 : 1, and often substantially 1 : 1. Typically, the ratio is in the range of 2.5 : 1 to 1 : 1 3D-MPL: QS21. Another combination adjuvant formulation includes 3D-MPL and an aluminum salt, such as aluminum hydroxide. When formulated in combination, this combination can enhance an antigen-specific Th1 immune response.

In some instances, the adjuvant formulation includes an oil-in-water emulsion, or a mineral salt such as a calcium or aluminum salt, for example calcium phosphate, aluminum phosphate or aluminum hydroxide.

One example of an oil-in-water emulsion comprises a metabolizable oil, such as squalene, a tocol such as a tocopherol, e.g., alpha-tocopherol, and a surfactant, such as polysorbate 80 or Tween 80, in an aqueous carrier. The aqueous carrier can be, for example, phosphate buffered saline. Additionally the oil-in-water emulsion can contain span 85 and/or lecithin and/or tricaprylin.

Where alum is present, e.g., in combination with 3D-MPL, the amount is typically between about 100μg and 1mg, such as from about 100μg, or about 200μg to about 750μg, such as about 500μg per dose.

The pH of a liquid preparation is adjusted in view of the components of the composition and necessary suitability for administration to the subject. Suitably, the pH of a liquid mixture is at least 4, at least 5, at least 5.5, at least 5.8, at least 6. The pH of the liquid mixture may be less than 9, less than 8, less than 7.5 or less than 7. In other embodiments, pH of the liquid mixture is between 4 and 9, between 5 and 8, such as between 5.5 and 8. Consequently, the pH will suitably be between 6-9, such as 6.5-8.5. In a particularly preferred embodiment the pH is between 5.8 and 6.4.

An appropriate buffer may be selected from acetate, citrate, histidine, maleate, phosphate, succinate, tartrate and TRIS. In one embodiment, the buffer is a phosphate buffer such as $Na/Na_2PO_4$, $Na/K_2PO_4$ or $K/K_2PO_4$.

The buffer can be present in the liquid mixture in an amount of at least 6mM, at least 10 mM or at least 40mM. The buffer can be present in the liquid mixture in an amount of less than 100 mM, less than 60 mM or less than 40 mM.

It is well known that for parenteral administration solutions should have a pharmaceutically acceptable osmolality to avoid cell distortion or lysis. A pharmaceutically acceptable osmolality will generally mean that solutions will have an osmolality which is approximately isotonic or mildly hypertonic. Suitably the compositions of the present invention when reconstituted will have an osmolality in the range of 250 to 750 mOsm/kg, for example, the osmolality may be in the range of 250 to 550 mOsm/kg, such as in the range of 280 to 500 mOsm/kg. In a particularly preferred embodiment the osmolality may be in the range of 280 to 310 mOsm/kg.

Osmolality may be measured according to techniques known in the art, such as by the use of a commercially available osmometer, for example the Advanced® Model 2020 available from Advanced Instruments Inc. (USA).

An "isotonicity agent" is a compound that is physiologically tolerated and imparts a suitable tonicity to a formulation to prevent the net flow of water across cell membranes that are in contact with the formulation. In some embodiments, the isotonicity agent used for the composition is a salt (or mixtures of salts), conveniently the salt is sodium chloride, suitably at a concentration of approximately 150 mM. In other embodiments, however, the composition comprises a non-ionic isotonicity agent and the concentration of sodium chloride in the composition is less than 100 mM, such as less than 80 mM, e.g. less than 50 mM, such as less 40 mM, less than 30 mM and especially less than 20 mM. The ionic strength in the composition may be less than 100 mM, such as less than 80 mM, e.g. less than 50 mM, such as less 40 mM or less than 30 mM.

In a particular embodiment, the non-ionic isotonicity agent is a polyol, such as sucrose and/or sorbitol. The concentration of sorbitol may e.g. between about 3% and about 15% (w/v), such as between about 4% and about 10% (w/v). Adjuvants comprising an immunologically active saponin fraction and a TLR4 agonist wherein the isotonicity agent is salt or a polyol have been described in WO2012/080369.

Suitably, a human dose volume of between 0.05 ml and 1 ml, such as between 0.1 and 0.5 ml, in particular a dose volume of about 0.5 ml, or 0.7 ml. The volumes of the compositions used may depend on the delivery route and location, with smaller doses being given by the intradermal route. A unit dose container may contain an overage to allow for proper manipulation of materials during administration of the unit dose.

The saponin, such as QS-21, can be used at amounts between 1 and 100 ug per human dose. QS-21 may be used at a level of about 50 ug. Examples of suitable ranges are 40-60 ug, suitably 45-55 ug or 49-51 ug, such as 50 ug. In a further embodiment, the human dose comprises QS-21 at a level of about 25 ug. Examples of lower ranges include 20-30 ug, suitably 22-28 ug or 24-26 ug, such as 25 ug. Human doses intended for children or infants may be reduced compared to those intended for an adult (e.g. reduction by 50%).

The TLR4 agonist such as a lipopolysaccharide, such as 3D-MPL, can be used at amounts between 1 and 100 ug per human dose. 3D-MPL may be used at a level of about 50 ug. Examples of suitable ranges are 40-60 ug, suitably 45-55 ug or 49-51 ug, such as 50 ug. In a further embodiment, the human dose comprises 3D-MPL at a level of about 25 ug. Examples of lower ranges include 20-30 ug, suitably 22-28 ug or 24-26 ug, such as 25 ug. Human doses intended for children or infants may be reduced compared to those intended for an adult (e.g. reduction by 50%).

When both a TLR4 agonist and a saponin are present in the adjuvant, then the weight ratio of TLR4 agonist to saponin is suitably between 1:5 to 5:1, suitably 1:1. For example, where 3D-MPL is present at an amount of 50 ug or 25 ug, then suitably QS-21 may also be present at an amount of 50 ug or 25 ug per human dose.

The ratio of saponin:DOPC will typically be in the order of 1:50 to 1:10 (w/w), suitably between 1:25 to 1:15 (w/w), and preferably 1:22 to 1:18 (w/w), such as 1:20 (w/w).

An immunogenic composition typically contains an immunoprotective quantity (or a fractional dose thereof) of the antigen and can be prepared by conventional techniques. Preparation of immunogenic compositions, including those for administration to human subjects, is generally described in Pharmaceutical Biotechnology, Vol.61 Vaccine Design-the subunit and adjuvant approach, edited by Powell and Newman, Plenurn Press, 1995. New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

Typically, the amount of protein in each dose of the immunogenic composition is selected as an amount which induces an immunoprotective response without significant, adverse side effects in the typical subject. Immunoprotective in this context does not necessarily mean completely protective against infection; it means protection against symptoms or disease, especially severe disease associated with the virus. The amount of antigen can vary depending upon which specific immunogen is employed. Generally, it is expected that each human dose will comprise 1-1000μg of protein, such as from about 1μg to about 100μg, for example, from about 1μg to about 50μg, such as about 1μg, about 2μg, about 5μg, about 10μg, about 15μg, about 20μg, about 25μg, about 30μg, about 40μg, or about 50μg. The amount utilized in an immunogenic composition is selected based on the subject population, e.g., infant, maternal (mothers who have not yet delivered their child), or older adult. An optimal amount for a particular composition can be ascertained by standard studies involving observation of antibody titres and other responses in subjects. Following an initial vaccination, subjects can receive a boost in about 4 weeks.

It should be noted that regardless of the adjuvant selected, the concentration in the final formulation is calculated to be safe and effective in the target population. For example, immunogenic compositions for eliciting an immune response against RSV in humans are favorably administered to infants (e.g., infants between birth and 1 year, such as between 0 and 6 months, at the age of initial dose) or individuals from a maternal population. Immunogenic compositions for eliciting an immune response against RSV are also favorably administered to older adult humans. It will be appreciated that the choice of adjuvant can be different in these different applications, and the optimal adjuvant and concentration for each situation can be determined empirically by those of skill in the art.

In certain embodiments, the immunogenic compositions are vaccines that reduce or prevent infection with RSV. In some embodiments, the immunogenic compositions are vaccines that reduce or prevent a pathological response following infection with RSV. Optionally, the immunogenic compositions containing a RSV soluble F protein polypeptide are formulated with at least one additional antigen of a pathogenic organism other than RSV. For example, the pathogenic organism can be a pathogen of the respiratory tract (such as a virus or bacterium that causes a respiratory infection). In certain cases, the immunogenic composition contains an antigen derived from a pathogenic virus other than RSV, such as a virus that causes an infection of the respiratory tract, such as influenza or parainfluenza. In other embodiments, the additional antigens are selected to facilitate administration or reduce the number of inoculations required to protect a subject against a plurality of infectious organisms. For example, the antigen can be derived from any one or more of influenza, hepatitis B, diphtheria, tetanus, pertussis, Hemophilus influenza, poliovirus, Streptococcus or Pneumococcus, among others.

Accordingly, the use of a recombinant RSV soluble F protein polypeptide according to the invention in the preparation of a medicament for treating (either therapeutically following or prophylactically prior to) exposure to or infection by RSV is also a feature of this disclosure. Likewise, methods for eliciting an immune response against RSV in a subject are a feature of this disclosure. Such methods include administering an immunologically effective amount of an antigenic composition comprising a RSV soluble F protein polypeptide to a subject, such as a human subject. Commonly, the composition includes an adjuvant that elicits a Th1 biased immune response. The composition is formulated to elicit an immune response specific for RSV without enhancing viral disease following contact with RSV. That is, the composition is formulated to and results in a Th1 biased immune response that reduces or prevents infection with a RSV and/or reduces or prevents a pathological response following infection with a RSV. Although the composition can be administered by a variety of different routes, most commonly, the immunogenic compositions are delivered by an intramuscular or intranasal route of administration.

An immunogenic composition typically contains an immunoprotective quantity (or a fractional dose thereof) of the antigen and can be prepared by conventional techniques. Preparation of immunogenic compositions, including those for administration to human subjects, is generally described in Pharmaceutical Biotechnology, Vol.61 Vaccine Design-the subunit and adjuvant approach, edited by Powell and Newman, Plenurn Press, 1995. New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

Typically, the amount of protein in each dose of the immunogenic composition is selected as an amount which induces an immunoprotective response without significant, adverse side effects in the typical subject. Immunoprotective in this context does not necessarily mean completely protective against infection; it means protection against symptoms or disease, especially severe disease associated with the virus. The amount of antigen can vary depending upon which specific immunogen is employed. Generally, it is expected that each human dose will comprise 1 - 1000$\mu$g of protein, such as from about 1$\mu$g to about 100$\mu$g, for example, from about 1$\mu$g to about 50$\mu$g, such as about 1$\mu$g, about 2$\mu$g, about 5$\mu$g, about 10$\mu$g, about 15$\mu$g, about 20$\mu$g, about 25$\mu$g, about 30$\mu$g, about 40$\mu$g, or about 50$\mu$g. The amount utilized in an immunogenic composition is selected based on the subject population (e.g., infant, maternal, or older adult). An optimal amount for a particular composition can be ascertained by standard studies involving observation of antibody titres and other responses in subjects. Following an initial vaccination, subjects can receive a boost in about 4-12 weeks. For example, when administering an immunogenic composition containing a RSV soluble F protein polypeptide to an infant subject, the initial and subsequent inoculations can be administered to coincide with other vaccines administered during this period.

General

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. The term "plurality" refers to two or more. Additionally, numerical limitations given with respect to concentrations or levels of a substance, such as solution component concentrations or ratios thereof, and reaction conditions such as temperatures, pressures and cycle times are intended to be approximate. The term "about" used herein is intended to mean the amount $\pm$10%. The term "between" with reference to a numerical range is intended to include the endpoints of the ranges unless the range is expressly stated to exclude the endpoints.

The invention will be further described by reference to the following, nonlimiting, figures and examples.

EXAMPLES

Example 1 - Production of recombinant RSV soluble F protein polypeptide in CHO cells

A. Production methodology

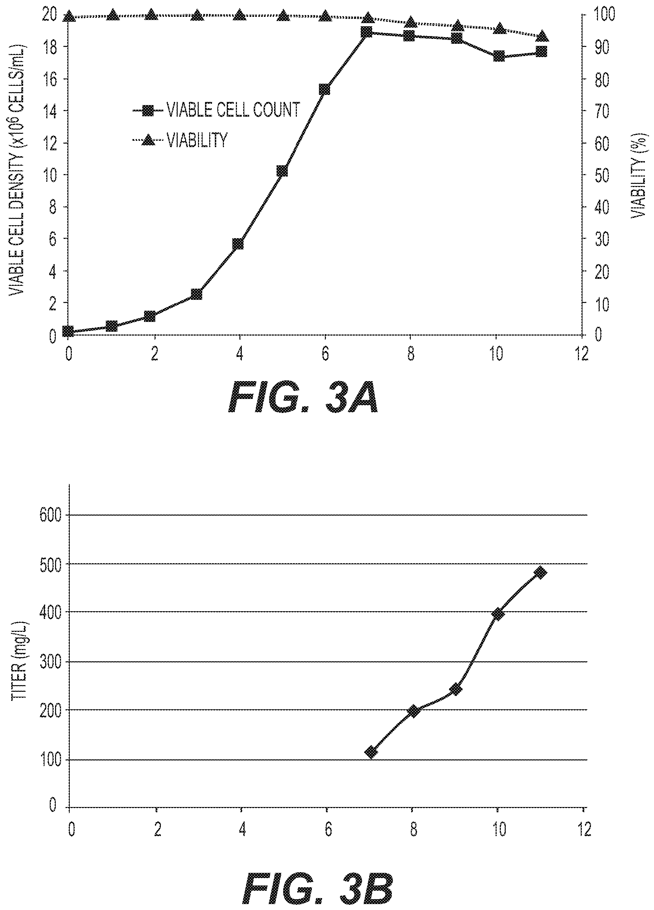

A recombinant polynucleotide sequence encoding the recombinant RSV soluble F protein polypeptide was integrated into the CHO host cells for the antigen production. To produce the recombinant RSV soluble F protein polypeptide, a work cell bank vial is thawed and expanded at the inoculation viable cell density of $0.2 \times 10^6$ cells/mL in three consecutive shake flask steps using cell culture medium in a carbon dioxide incubator, at roughly 37°C, under controlled $CO_2$ conditions for several days, followed by two consecutive seed bioreactor expansions at roughly 37°C, physiological pH, and controlled CO2 conditions for several days. The expanded culture is used to inoculate the production bioreactor at viable cell density at a temperature of roughly 37°C, physiological pH, and controlled $CO_2$ conditions. The bioreactor starts at less than 90% of the working volume (WV) with two different feeds. The first feed is done at 1% of the WV daily when culture reaches about $5 \times 10^6$ cells/mL, the second feed at 0.2% of WV daily two days later. The temperature is lowered when culture density doubles to lower than 35°C, pH is controlled with $CO_2$ and NaOH, dissolved oxygen is controlled with pure oxygen. Culture is harvested when complete. The viable cell density, viability and product accumulation profiles are shown in FIG.3A and FIG.3B.

The culture is harvested through filtration to generate a clarified fermentation broth, involving a primary and secondary filtration connected in parallel. The primary filter is $D_0HC$ with a filtration area of 0.022m$^2$ per 1 liter culture; the secondary filter is $X_0HC$ with a filtration area of 0.011m$^2$ per 1 liter culture. The filtrate is further filtered with 0.2 $\mu$M filter to reduce the bioburden for downstream purification. The harvest yield is about 75%, with 40% total CHO host cell proteins and 4 log of CHO host DNA level reduction.

B. Production methodology supplement

A recombinant polynucleotide sequence encoding the recombinant RSV soluble F protein polypeptide was integrated into the CHO host cells for the antigen production. To produce the recombinant RSV soluble F protein polypeptide, a work cell bank vial is thawed and expanded at the inoculation viable cell density of $0.2 \times 10^6$ cells/mL in three consecutive shake flask steps using cell culture medium in a carbon dioxide incubator, at roughly 37°C, under controlled $CO_2$ conditions for several days, followed by two consecutive seed bioreactor expansions at roughly 37°C, physiological pH, and controlled $CO_2$ conditions for several days. A onetime 5% bioreactor volume addition of the Modified DM133 feed needs to be made 70-74 hours post inoculation for the second seed bioreactor. The expanded culture is used to inoculate the production bioreactor at $1.0 \times 10^6$ cells/mL viable cell density at a temperature of roughly 37°C, physiological pH, and controlled $CO_2$ conditions. The bioreactor starts at less than 90% of the working volume (WV) with a 2.5% (V/V) of amount of Modified DM133 feed added into the bioreactor just prior to inoculation. A 2.5% (V/V) of amount of modified feed is added into the bioreactor just prior to inoculation. The first feed is done at 1.25% of the WV daily starting on day 2, the second feed at 0.5% of WV starts daily two days later. The temperature is lowered when culture density reaches 10-14 $\times 10^6$ cells/mL and is lowered to 33°C, pH is controlled with $CO_2$ and NaOH, dissolved oxygen is controlled with pure oxygen. Culture is harvested when complete. The viable cell density, viability and product accumulation profiles are shown in FIG.23A and FIG.23B.

The culture is harvested through filtration to generate a clarified fermentation broth, involving a primary and secondary filtration connected in parallel. For primary clarification, the culture is pumped through a D0SP filter of filter area of 0.66m$^2$. The partially clarified harvest is then pumped through a X0SP filter, with a filter area of 0.33 m$^2$, connected in parallel. The filtrate is further filtered with 0.2 $\mu$M filter to reduce the bioburden for downstream purification. The harvest yield is about 75%, with 40% total CHO host cell proteins and 4 log of CHO host DNA level reduction.

Example 2 - Purification of recombinant RSV soluble F protein polypeptide from CHO Cells

A. Methods

(1) Manufacturing of the recombinant RSV soluble F protein polypeptide drug substance/bulk intermediate solution is based on one fed batch cell culture production batch.

To recover the recombinant RSV soluble F protein polypeptide the cell culture is harvested by depth filtration. The filtered harvest is subjected to virus inactivation by detergent addition. The virus inactivated intermediate is captured by Capto DeVirS chromatography in bind/elute mode. The Capto DeVirs eluate is then further purified in Capto Q ImPres chromatography in bind/elute mode. The Capto Q ImPres eluate is further polished by Capto Adhere chromatography in bind/elute mode. The Capto Adhere eluate is processed by a virus filtration step. The virus filtrate is then concentrated and buffer exchanged followed by final excipient and concentration adjustment and 0.2 μm filtration. All chromatography gels employed in this purification are manufactured by GE-Healthcare.

The purpose of the virus inactivation step is to eliminate detergent intolerant viruses like enveloped viruses. Polysorbate-80 is added to the harvest material under constant stirring to achieve a target concentration of 1.0 %. Incubation for 15 to 20 hours is performed at controlled room temperature under light protection. Termination of the virus inactivation is carried out through the capture chromatography in process step Capto DeVirs; incubation time ends with the end of load in the Capto DeVirs step.

The first purification step is an affinity-like chromatography using the resin Capto DeVirS in bind/elute mode. The purpose of the Capto DeVirS column is to capture recombinant RSV soluble F protein polypeptide from the virus inactivated intermediate, volume reduction and decrease of general impurities for the further downstream processing. For this step, the virus inactivated pool is diluted with water to achieve a target conductivity of 6 mS/cm. After conductivity adjustment, the material is loaded onto Capto DeVirs which previously had been equilibrated with 47 mM sodium phosphate pH 7.0-7.4. After washing the column with the equilibration buffer, the recombinant RSV soluble F protein polypeptide pool is eluted with 20 mM sodium phosphate, 235 mM sodium chloride pH 7.0-7.4 at 200 cm/h.

The second purification step is an anion exchange chromatography using the resin Capto Q ImPres in bind/elute mode. The purpose of the Capto Q ImPres column is to increase product purity (verified via RP-HPLC) and to reduce HCP and DNA. The Capto Q ImPres chromatography is also defined as a virus removal step. For this step, the conductivity of the Capto DeVirs elution pool is adjusted to 15.0 mS/cm by dilution with water. After conductivity adjustment, the material is loaded onto Capto Q ImPres which previously had been equilibrated with 20 mM sodium phosphate, 115 mM sodium chloride pH 7.0-7.4. After washing the column with the equilibration buffer, the recombinant RSV soluble F protein polypeptide pool is eluted with 20 mM sodium phosphate, 225 mM sodium chloride pH 7.0-7.4 at 200 cm/h.

The third purification step is a mixed mode chromatography using the resin Capto Adhere in bind/elute mode. The purpose of the Capto Adhere column is to reduce high molecular weight species, low molecular weight species, and HCP. The Capto Adhere chromatography is also defined as a virus removal step. For this step, the Capto Q ImPres pool is directly applied onto the Capto Adhere column at 200 cm/h; the column previously had been equilibrated with 20 mM sodium phosphate, 225 mM sodium chloride, pH 7.0-7.4. After washing the column with the equilibration buffer, the recombinant RSV soluble F protein polypeptide pool is eluted with 20 mM sodium phosphate, 250 mM sodium citrate, 300 mM Arginine, pH 7.0-7.4 at 200 cm/h.

Capto Adhere is a multimodal anion exchange resin which has been optimized for aggregate removal, host-cell protein removal and viral clearance. The ligand of the Capto Adhere chromatography resin is N-benzyl-N-methyl ethanolamine; this ligand can interact with proteins via electrostatic interactions, hydrogen bonding, and hydrophobic interactions. For the recombinant RSV soluble F protein polypeptide process, the protein is loaded at 27 mS/cm pH 7.0-7.4; the loading buffer contains approximately 225 mM sodium chloride. The conditions at which recombinant RSV soluble F protein polypeptide binds to Capto Adhere favor the interaction between the hydrophobic components of the sample and the hydrophobic components of the Capto Adhere resin. The elution buffer consists of 20 mM sodium phosphate, 250 mM sodium citrate, 300 mM Arginine, pH 7.0-7.4. Following application of the buffer, the recombinant RSV soluble F protein polypeptide elutes from the Capto Adhere column. The conditions used in this process allowed an MuLV clearance of ≥ 4.28 logs and an MVM clearance of 3.2 logs.

**Table 1.**

| Log reduction Values (LRV) | MuLV #1 | MuLV #2 | MVM #1 | MVM #2 |
|---|---|---|---|---|
| C30 Capto Adhere | ≥ 4.42 ± 0.32 | ≥ 4.28 ± 0.26 | 3.24 ± 0.17 | 4.05 ± 0.31 |

The chromatography steps are followed by a virus filtration step using Planova 20N filters (Asahi Kasei). An Opticap XL5 PVDF filter with a 0.1 μm membrane is used as a pre-filter. The purpose of this process step is to eliminate potential viruses present in the process intermediate pools.

The next purification step is the ultrafiltration/diafiltration step performed with Pellicon 3 PES Biomax (MWCO 50 kDa) manufactured by Millipore. The purpose of this step is to concentrate the product and to exchange the matrix against the diafiltration buffer, consisting of 10 mM potassium phosphate and 5 % trehalose. The diafiltrated retentate is drained from the system before the system rinse is performed. The system rinse is added to the diafiltrated retentate to achieve a concentrating ranging from 1.2 mg/mL to 1.5 mg/mL.

The purpose of the drug substance formulation step is to adjust the final drug substance excipient composition to a final content of 0.05% PS-80 and the concentration to a target of 1 mg/mL. The drug substance is then finally 0.2 $\mu$m filtered, aliquoted and frozen in a freezer system. The drug substance is stored at - 70 °C until further processing.

(2) Manufacturing of the recombinant RSV soluble F protein polypeptide drug substance/bulk intermediate solution based on one fed batch cell culture production batch can also be accomplished as follows.

To recover the recombinant RSV soluble F protein polypeptide the cell culture is harvested by depth filtration. The filtered harvest is captured by Capto DeVirS chromatography in bind/elute mode. The Capto DeVirs eluate is then subjected to virus inactivation by detergent addition. The virus inactivated intermediate is further purified in Capto Q ImpRes chromatography in bind/elute mode. The Capto Q ImpRes eluate is further polished by Capto Adhere chromatography in bind/elute mode. The Capto Adhere eluate is processed by a virus filtration step. The virus filtrate is then concentrated and buffer exchanged followed by final excipient and concentration adjustment and 0.2 $\mu$m filtration. All chromatography gels employed in this purification are manufactured by GE-Healthcare.

The first purification step is an affinity-like chromatography using the resin Capto DeVirS in bind/elute mode. The purpose of the Capto DeVirS column is to capture recombinant RSV soluble F protein polypeptide from the harvest, volume reduction and decrease of general impurities for the further downstream processing. For this step, the harvest is diluted with water to achieve a target conductivity of 5.25 mS/cm. After conductivity adjustment, the material is loaded onto Capto DeVirs which previously had been equilibrated with 40 mM sodium phosphate pH 7.1 - 7.5. After washing the column with the equilibration buffer, the recombinant RSV soluble F protein polypeptide pool is eluted with 20 mM sodium phosphate, 240 mM sodium chloride pH 7.2 - 7.6 at 275 cm/h.

The capture step is followed by the virus inactivation. The purpose of the virus inactivation step is to eliminate detergent intolerant viruses like enveloped viruses. Polysorbate-80 is added to the harvest material under constant stirring to achieve a target concentration of 2.0 %. Incubation for 18 to 24 hours is performed at controlled room temperature (22-24C) under light protection. Termination of the virus inactivation is carried out through dilution of the virus inactivated pool prior to the next chromatography step.

The virus inactivation is followed by anion exchange chromatography using the resin Capto Q ImpRes in bind/elute mode. The purpose of the Capto Q ImpRes column is to increase product purity (verified via RP-HPLC) and to reduce HCP and DNA. The Capto Q ImpRes chromatography is also defined as a virus removal step. For this step, the conductivity of the Capto DeVirs elution pool is adjusted to 10.9 mS/cm by dilution with water. After conductivity adjustment, the material is loaded onto Capto Q ImpRes which previously had been equilibrated with 20 mM sodium phosphate, 80 mM sodium chloride pH 7.3-7.7. After washing the column with the equilibration buffer, the recombinant RSV soluble F protein polypeptide pool is eluted with 20 mM sodium phosphate, 180 mM sodium chloride pH 7.3-7.7 at 200 cm/h.

The third purification step is a mixed mode chromatography using the resin Capto Adhere in bind/elute mode. The purpose of the Capto Adhere column is to reduce high molecular weight species, low molecular weight species, and HCP. The Capto Adhere chromatography is also defined as a virus removal step. For this step, the Capto Q ImpRes pool is directly applied onto the Capto Adhere column at 200 cm/h; the column previously had been equilibrated with 20 mM sodium phosphate, 180 mM sodium chloride, pH 7.3-7.7. After washing the column with the equilibration buffer, the recombinant RSV soluble F protein polypeptide pool is eluted with 20 mM sodium phosphate, 250 mM sodium citrate, 300 mM Arginine, pH 7.2-7.6 at 150 cm/h.

Capto Adhere is a multimodal anion exchange resin which has been optimized for aggregate removal, host-cell protein removal and viral clearance. The ligand of the Capto Adhere chromatography resin is N-benzyl-N-methyl ethanolamine; this ligand can interact with proteins via electrostatic interactions, hydrogen bonding, and hydrophobic interactions. For the recombinant RSV soluble F protein polypeptide process, the protein is loaded at 21 mS/cm pH 7.3-7.7; the loading buffer contains approximately 180 mM sodium chloride. The conditions at which recombinant RSV soluble F protein polypeptide binds to Capto Adhere favor the interaction between the hydrophobic components of the sample and the hydrophobic components of the Capto Adhere resin. The elution buffer consists of 20 mM sodium phosphate, 250 mM sodium citrate, 300 mM Arginine, pH 7.2-7.6. Following application of the buffer, the recombinant RSV soluble F protein polypeptide elutes from the Capto Adhere column. The conditions used in this process allowed an MuLV clearance of > 4.81logs and an MVM clearance of >3.15 logs.

The chromatography steps are followed by a virus filtration step using Virosart HF (Sartorius Stedim). A Virosart Max filter with a 0.1 $\mu$m membrane is used as a pre-filter. The purpose of this process step is to eliminate potential viruses present in the process intermediate pools.

The next purification step is the ultrafiltration/diafiltration step performed with Pellicon 3 PES Biomax (MWCO 50 kDa) manufactured by Millipore. The purpose of this step is to concentrate the product and to exchange the matrix against the diafiltration buffer, consisting of 10 mM potassium phosphate and 4 % trehalose. The diafiltrated retentate is drained from the system before the system rinse is performed. The system rinse is added to the diafiltrated retentate and formulation buffer is added to achieve a concentrating ranging from 0.9 mg/mL to 1.3 mg/mL.

The purpose of the drug substance formulation step is to adjust the final drug substance excipient composition to a final content of 0.05% PS-80. The drug substance is then finally 0.2 $\mu$m filtered, aliquoted and frozen in a freezer system. The drug substance is stored at - 70 °C until further processing.

**Table 1A.** The process described above consistently produces product having the purity (% recombinant RSV soluble F protein polypeptide determined by SE-HPLC for selected lots.

| Assay | Process Run | | | | | | | | |
|---|---|---|---|---|---|---|---|---|---|
| | CR 1 | CR 2 | CR 3 | CR 4 | SUIT29 | SUIT30 | SUIT32 | SUIT34 | SUIT36 |
| Production Scale | 12 L | 12 L | 12 L | 12 L | 200 L | 200 L | 200 L | 200L | 200L |
| % Purity | 99 | 98.7 | 98.6 | 98.9 | 98 | 97.6 | 97.9 | 97.8 | 98.3 |

B. Content of p27 in drug substance generated

Applicant observed that in a certain proportion of the recombinant RSV soluble F protein polypeptide protomers produced during expression, the sequence of the p27 peptide was detected; this was termed " F' " to distinguish it from the proportion of recombinant RSV soluble F protein polypeptide protomers in which the sequence of the p27 peptide was not detected (" F "). Whilst not wishing to be bound by theory, it is thought that during expression of the recombinant RSV soluble F protein polypeptide from host cells, a proportion of furin cleavage sites remain uncleaved.

Following the procedure described above has produced drug substance with the following proportions of F (also referred to as preF peak) and F' (also referred to as preF' or p27) determined by RP-HPLC as shown in Table 2.

**Table 2.** The process described above consistently produces product having the purity (% recombinant RSV soluble F protein polypeptide determined by RP-HPLC) as set forth in the table.

| Assay | Process Run | | | | | | | | |
|---|---|---|---|---|---|---|---|---|---|
| | CR 1 | CR 2 | CR 3 | CR 4 | TT01 | CR01 | TX01 | MSR01 | GMP 1 |
| Production Scale | 10 L | 10 L | 10 L | 10 L | 10 L | 10 L | 30 L | 30 L | 225 L |
| % Purity (F + F' peak) | 96.7 | 99.1 | 99.5 | 98.9 | 100 | 100 | 99.9 | 99 | 99.8 |

C. Separation of Trimers Enriched in F protomers from trimers containing F' protomers

Recombinant RSV soluble F protein polypeptide purified using the process described previously was further purified using Capto MMC ImpRes chromatography (GE Healthcare). The load material (pH 7.1, conductivity 1.5 mS/cm) was applied onto a Capto MMC ImpRes column (9.4 mL HiScreen). The column previously had been equilibrated with 20 mM sodium phosphate, pH 7.2. After washing the column with the equilibration buffer, elution was performed with a NaCl gradient from 0 to 250 mM, 20mM Sodium Phosphate, 1M NaCitrate in 20 column volumes (fraction size 3 mL). Fractions were analyzed on SDS PAGE gel by Coomassie staining and RP-HPLC. Fractions were pooled in two pools based on their RP-HPLC purity and p27 peptide content. Pool 1 consisted of fractions with ~100% Main peak by RP-UPLC. Pool 2 consisted of fractions with ~ 33% p27 (F' protomer) and 67% Main Peak. Therefore, two populations were identified and isolated: a trimer containing ~100 % Main Peak (F protomer) and another trimer with ~33% p27 peptide (F' protomer) and 67% Main Peak (F protomer).

Example 3

A. Host cell protein profiling and GRP78 quantitation

Recombinant RSV soluble F protein polypeptide as produced by the process above was reduced by DTT based on labelled concentrations, alkylated by isoacetamide, and digested by trypsin overnight at 37 °C. Then the digest was acidified and analyzed by LC-MS/MS using a Thermo Fusion Orbitrap operated under data-dependent acquisition mode. Peak lists from the raw data were searched against a protein database containing recombinant RSV soluble F protein polypeptide sequence and sequences of all known and predicted CHO proteins using the Mascot search engine. Only one host cell protein (HCP) was detected, the GRP78 (78 kDa glucose-regulated protein), which is a known HCP.

A targeted quantitation of GRP78 was also performed by an isotope dilution mass spectrometry method. Host-cell protein content of the preparation was also determined by ELISA, such as Cygnus CHO hcp ELISA. The results showed that the GRP78 content in the materials produced according to the process herein was 0.013%.

B. Aggregation

An in-house developed Size-Exclusion Chromatography (SEC) assay was used to evaluate the aggregation levels of recombinant RSV soluble F protein polypeptide produced by the process described above. SEC resolves proteins by their sizes. The % distribution of low, main, and high molecular weight species is shown in Table 3.

**Table 3.** Percentage distribution of high molecular weight, main peak, and low molecular weight species in recombinant RSV soluble F protein polypeptide materials.

| Sample | % HMW | % Main Peak | % LMW |
|---|---|---|---|
| recombinant RSV soluble F protein polypeptide as produced by the process (above) | 1.5 | 96.9 | 1.6 |

C. N500 site occupancy

Recombinant RSV soluble F protein polypeptide is modified by N-glycans on 3 Asn residues (N27, N70, and N500. Amino acid numberings are based on full length RSV F precursor). The N500 site may be non-glycosylated in a fraction of a population of recombinant RSV soluble F protein polypeptides. To determine the levels of non-glycosylated N500, tryptic peptides of recombinant RSV soluble F protein polypeptide as produced by the process described above was analyzed by LC-MS/MS before and after PNGase F treatment, from which the intensities of K.I500NQSLAFIR.K (SEQ ID NO:4) (representing non-glycosylated N500) and K.I500DQSLAFIR.K (SEQ ID NO:5) (representing N500 with glycan removed by PNGase F) were compared. The result shows it contains ~7.2% incomplete glycosylation.

D.

*1. Primary sequence integrity and post translational modification*

Intact molecular weights of the recombinant RSV soluble F protein polypeptide produced by the process described above were analyzed by LC-MS on a Waters Xevo G2-S QTOF mass spectrometer. After spectra deconvolution, the molecular weight of the main species was determined to be 61,226 Da. The same material was de-N-glycosylated by PNGase F and then similarly analyzed. The molecular weight was determined to be 54,162 Da, which was in excellent agreement with theoretical mass calculated from recombinant RSV soluble F protein polypeptide primary sequence. These results support a conclusion that 1) the primary sequences is as expected; and 2) there is no unexpected post translational modification (other than glycosylation).

*2. FFF' trimer and F' protomer content*

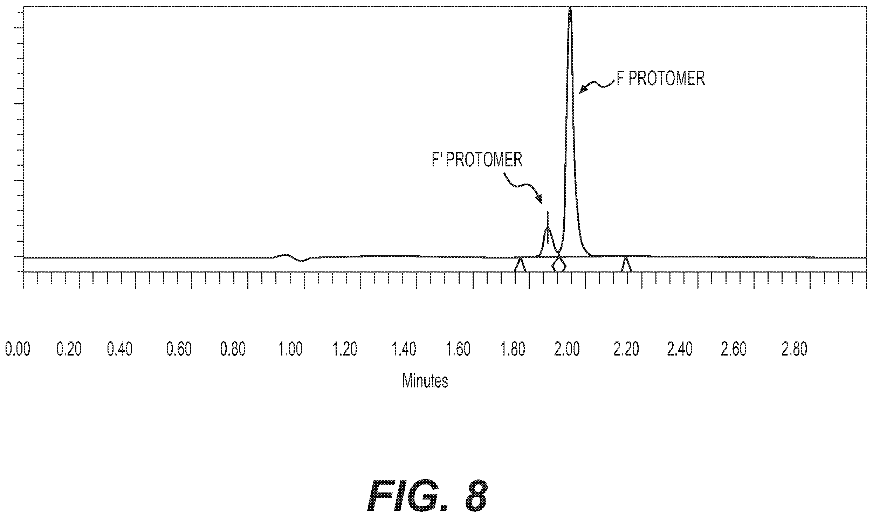

RP-HPLC analysis was then performed. As shown in FIG.8, F' was easily detectable in the recombinant RSV soluble F protein polypeptide material. WCX-LC, which resolves FFF trimer and F'-containing trimers, was also used. Recombinant RSV soluble F protein polypeptide was baseline resolved into two peaks (trimers comprising only F protomers and trimers comprising at least one F' protomer).

E. Glycan profile

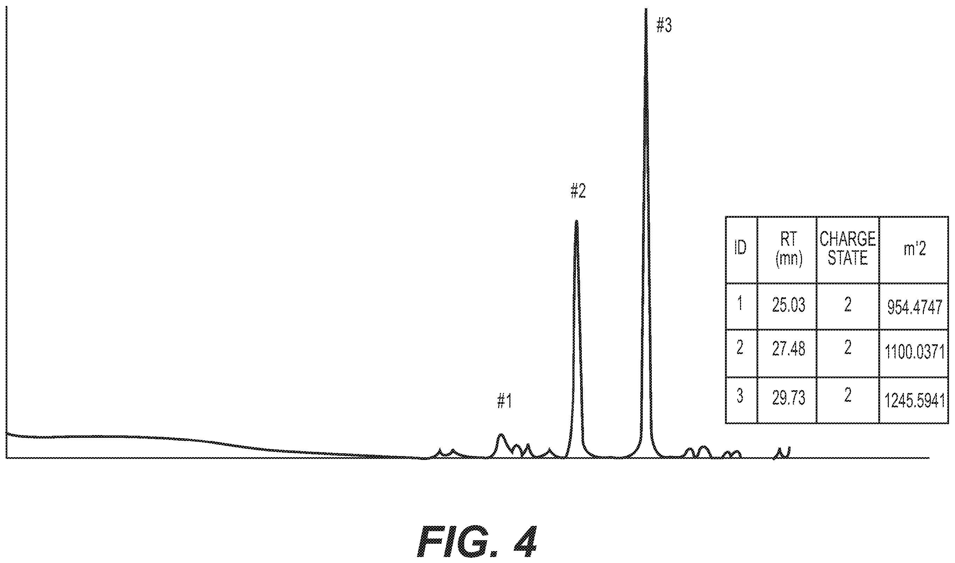

Glycan profiling was performed. N-glycans were first cleaved from recombinant RSV soluble F protein polypeptide produced by the process described above, then labelled with 2-AB through reductive amination, resolved by hydrophilic interaction liquid chromatography (HILIC) and detected/quantified by FLR and MS detectors. The glycan structures (in schematic form) and relative abundance of glycan species are shown in FIG.4. As expected, over 75% of the glycan molecules were represented by 3 main glycan species observed in recombinant RSV soluble F protein polypeptide produced by the process described above, being di-sialylated/fucosylated biantennary, mono-sialylated/fucosylated biantennary, and non-sialylated/fucosylated biantennary glycans. The majority of the glycans in this material are sialylated glycans.

## F. Site Ø and Site V epitope integrity

It was observed that the recombinant RSV soluble F protein polypeptide is unstable under certain storage conditions and undergoes a spontaneous conformational change leading to a measurable decrease (by Fab antibody shift assay) in antibody binding at Site Ø and Site V, which conformational change affects an increasing proportion of RSVPreF3 {originally identified using the term "DS-Cav1", herein re-termed RSVPreF3 to distinguish it from the material produced as disclosed in McLellan et. al. Science (2013) vol. 342 p. 592, WO2014160463, and ClinicalTrials.gov Identifier: NCT03049488}, over time during storage in liquid buffer.

A Fab-Shift assay was developed to evaluate the integrity of Site Ø and Site V. The assay uses Size Exclusion Chromatography - Liquid Chromatography (SEC-LC) to resolve recombinant RSV soluble F protein polypeptide that is previously incubated with Fabs which specifically bind to Site Ø and Site V, respectively. Fab-bound recombinant RSV soluble F protein polypeptide will have larger molecular size and thus elute earlier from SEC column, hence be resolved from unbound recombinant RSV soluble F protein polypeptide. The results are shown in Table 4 (percentage unshifted in assay).

**Table 4.** Fab-Shift SEC chromatograms of recombinant RSV soluble F protein polypeptide.

|  | % Unshifted against | |
| --- | --- | --- |
| **Material** | **Site V Ab** | **Site Ø Ab** |
| recombinant RSV soluble F protein polypeptide | 11 | 15 |

## G. Isoelectric point (pI)

Recombinant RSV soluble F protein polypeptide produced according to the process described above contains sialylated N-glycans. Isoelectric focusing gel electrophoresis was used to resolve the different charge species in recombinant RSV soluble F protein polypeptide. The pI of recombinant RSV soluble F protein polypeptide was between ~5.3-6.0.

## H. Sedimentation velocity

Sedimentation velocity analytical ultracentrifugation (SV-AUC) experiments were performed with an Optima XL-A (Beckman Coulter, Fullerton, CA) analytical ultracentrifuge equipped with a scanning UV-Visible optical system. All experiments were conducted at 10 °C after 1h of equilibration after the AUC reached temperature, at a rotor speed of 30,000 RPM with detection at 280 nm. Samples and reference were loaded into Beckman charcoal-epon two sector cells with a 12 mm centerpiece and quartz windows. The data were analyzed using Sedfit (courtesy of Peter Schuck, NIH). A partial specific volume of 0.74 mL/g was calculated for recombinant RSV soluble F protein polypeptide produced using the process described above using Sednterp based on amino acid sequence, and used in the analysis. The buffer density and viscosity used in the analysis were also calculated using Sednterp software based on buffer composition. A continuous c(s) distribution was used with 300 scans. A range of 0 to 15 Svedbergs was used, after verifying that there was no signal sedimenting outside of this range, with a resolution of 300 points per distribution and a confidence level of 0.95. Baseline, radial independent noise, and time independent noise were fit, while the meniscus and bottom positions were set manually. The best fit friction ratio was used in the analysis.

The main peak is consistent with a moderately to highly elongated species with a molecular weight of ~178 kDa. Of note is the high degree of asymmetry observed. In the crystal structure, recombinant RSV soluble F protein polypeptide appears to be roughly a prolate ellipsoid with only a slight difference between the major and minor axes. A species with friction ratio of ~2 can be modelled as either a prolate ellipsoid with major and minor axes of 69.1 and 2.5 nm or an oblate ellipsoid with major and minor axes of 26.6 nm and 0.6 nm.

## I. Summary

Physicochemical studies of recombinant RSV soluble F protein polypeptide produced according to the process described above have been observed to produce at least 99.9% pure recombinant RSV soluble F protein polypeptide (w/w). In addition, glycosylation profiles of recombinant RSV soluble F protein polypeptide produced according to the process described above display over 75% of sialylated glycans represented by three major glycan peaks compared to seven or more in recombinant RSV soluble F protein polypeptide produced according to standard methods. And the pI of recombinant RSV soluble F protein polypeptide produced according to the process described above has pI of less than 7.8, indicating optimum CHO cell conditions (the pI difference is likely a reflection of glycosylation profile). Aggregation level produced according to the process described above is lower than recombinant RSV soluble F protein polypeptide prepared according to standard methods. Site Ø and Site V binding as measured by Fab shift assay both decrease over time during storage in liquid. In addition, the above referenced process results in HCP content lower than that for published recombinant RSV soluble F protein polypeptide compositions.

Example 4 - Site Ø and Site V binding studies

Recombinant RSV soluble F protein polypeptide harbors a metastable antigenic site Ø that is targeted by a neutralizing antibody. A gradual loss of Site Ø antibody binding was observed when recombinant RSV soluble F protein polypeptide was stored in a liquid formulation at 5°C. McLellan et al. (2013) "Structure-Based Design of a Fusion Glycoprotein for Respiratory Syncytial Virus," Science, 342: 592-598. The authors hypothesized from the superimposed crystal structures that significant movement and disorder in the Heptad Repeat A (HRA) region of recombinant RSV soluble F protein polypeptide may result in a second prefusion conformation with a compromised site Ø and it was proposed that mutations to stabilize the loops connecting the secondary structure parts of the HRA region could maintain site Ø during long term storage at 5°C. N67I and S215P modifications of recombinant RSV soluble F protein polypeptide showed no loss of Site Ø binding antibody-binding over a 60 day period stored at 5°C. Krarup et al (2015), "A highly stable prefusion RSVF vaccine derived from structural analysis of the fusion mechanism," Nat Commun, 6:8143. Another study observed the same phenomena in recombinant RSV soluble F protein polypeptide stability materials stored at 4°C and in addition, showed that the compromise of site Ø was paralleled by a better exposed site I epitope. Flynn (2016) "Stability Characterization of a Vaccine Antigen Based on the Respiratory Syncytial Virus Fusion Protein," Plos One, 10:1-18. The present applicants have observed that the domain implicated in loss of Site Ø binding overlapped with site V and subsequently also affected Site V antibody binding.

A combination of analytical and biophysical techniques was used to further probe the cause of Site Ø antibody-binding loss. Protein primary sequence modifications and aggregation were not observed to contribute to Site Ø antibody-binding loss. Recombinant RSV soluble F protein polypeptide appeared to exist in two distinct conformations after extended storage in liquid solution. A localized conformational change at a domain near site Ø rather than the site Ø epitope itself was considered likely in view of the data. Although the data showed protein stabilizing reagents did not protect recombinant RSV soluble F protein polypeptide from losing site Ø antibody-binding efficiency in the liquid formulation, the lyophilized formulation was protected.

A. Materials and methods

Tagless recombinant RSV soluble F protein polypeptide and postfusion RSV-F materials used in this study were expressed in CHO cells, purified from cell culture supernatant by extensive column fractionation, and formulated at ~ 1 mg/mL in phosphate buffer with low levels of surfactants and disaccharides at neutral pH. All other reagents were purchased at Fisher Scientific unless otherwise noted.

1. Size Exclusion Chromatography and Fabshift assay

Size exclusion chromatography Liquid Chromatography (SEC-LC) was performed using an Acquity BEH SEC column (4.6 mm X 150 mm, 1.7 $\mu$m) on a Waters Acquity UPLC system run in isocratic mode with a 100 mM sodium phosphate, 50 mM sodium chloride buffer at pH 7. The flow rate was 0.2 ml/min and detection was by UV at 215 nm and 280 nm. For Fabshift assay, recombinant RSV soluble F protein polypeptide was first mixed and incubated with Site Ø binding antibody or Site V binding antibody Fab at 37 °C for approximately one hour. recombinant RSV soluble F protein polypeptide , the respective Fab, and the reaction mixture were then each run by SEC-LC to establish the retention time of free recombinant RSV soluble F protein polypeptide (unshiftable fraction), free Fab and recombinant RSV soluble F protein polypeptide /Fab complex (shifted fraction), respectively. The chromatogram was integrated using Empower 3 software and peak areas were used for relative quantitation.

2. SDS-PAGE

Gel electrophoresis was performed using a 4-12% Bis-Tris precast polyacrylamide gel. Approximately 10ug of the sample mixed with SDS-PAGE sample buffer containing DTT was first heated at 70 °C, loaded to each well, and then subjected to 200 V for 45 min. After running, the gels were stained by Coomasie Blue, destained with a methanol/water/-acetic acid solution and then visualized on BioRad ChemiDoc Imaging System.

*3. Peptide Mapping*

Recombinant RSV soluble F protein polypeptide produced per the process disclosed above denatured with guanidinium chloride was buffer exchanged into 50 mM ammonium bicarbonate, reduced by DTT at 56 °C and alkylated by iodoacetamide at room temperature in the dark. Trypsin was added at a 10:1 enzyme-to-substrate ratio and incubated overnight at 37 °C. The digest was acidified by formic acid, injected on an Acquity BEH C18 column (2.1 mm X 150 mm, 1.7 μm), resolved by an increasing gradient of acetonitrile in 0.1% formic acid and electrosprayed into a Waters Xevo G2-S QTOF mass spectrometer for detection.

*4. Differential Scanning Fluorimetry (DSF)*

DSF was performed using a Prometheus nano-DSF instrument (NanoTemper Technologies, Inc. Munich, Germany). 10 μL of each test sample at 1 mg/mL (~5.5 μM) was added to thin capillaries and loaded into the instrument. A temperature ramp was run from 20°C to 110°C at 1°C per minute. The nano-DSF instrument utilizes an intrinsic fluorescence technique to monitor the changes of tyrosine and tryptophan fluorescence at 330nm and 350nm wavelengths as a function of temperature. A first derivative trace is also derived from the raw data to more accurately determine the onset of protein unfolding and the overall melting temperature.

*5. Circular Dichroism (CD)*

CD experiments were performed on a ChiraScan Q100 spectrometer (Applied Photophysics, Inc. Beverly, MA) at room temperature. Near-UV CD (250-340nm) data were collected on a 1mg/mL sample at 60 nm/min with a bandwidth of 1 nm in a quartz cuvette (1cm path length). Collected spectra were baseline corrected with the buffer and normalized using the absolute area under the curve. All data were collected in triplicate and the average of each storage time point was compared for similarity using a statistical analysis (data not shown).

*6. Hydrogen-Deuterium Exchange Mass Spectrometry (HDX-MS)*

A HDX-MS analysis was performed on a Waters HDX manager with a LEAP system coupled to a Xevo G2-S QTOF mass spectrometer. All test materials were analyzed in triplicate. Each test material was diluted 20 fold by 10 mM potassium phosphate in $H_2O$ or $D_2O$ and incubated at a time course of 0, 0.5, 5, 10, 30, and 240 minutes. Exchange was quenched at 0°C with equal volume addition of 1 M Guanidine hydrochloride, 0.25 M Tris (2-carboxyethyl) phosphine hydrochloride (TCEP) at pH 2.5. Samples were immediately digested on a pepsin column (Waters) at 20 °C. Peptides were concentrated and buffer exchanged using Waters Vanguard pre-column (2.1x50 mm), separated at 0 °C on an Acquity BEH C18 column (1.7 μm, 1.0 X 100 mm, Waters) and then analyzed in the MS. Data analysis was performed using the ProteinLynx Global Server (PLGS) and the DynamX software (version 3.0.0)

*7. Homology model*

The homology model of Site Ø binding antibody bound to recombinant RSV soluble F protein polypeptide was generated by using two different crystal structure templates (pdbs: 4mmv and 4jhw) with the Molecular Operating Environment modeling program. The recombinant RSV soluble F protein polypeptide sequence was used as the template sequence. Two crystal structures were implemented because the foldon region in the 4mmu region is disordered and the Site Ø binding antibody Fabs are not present in any of the recombinant RSV soluble F protein polypeptide structures. As for Site V binding antibody bound / epitope site II binding antibody bound recombinant RSV soluble F protein polypeptide (the site II antibody binds prefusion and postfusion RSV-F), the homology models were also generated by MOE with the 4mmv and 4zyk crystal structures. For each of the two final homology models, ten individual models were created and lowest energy model was chosen as the final representation of homology model for each individual system. The final homology model was minimized with the AMBER10:EHT Forcefield with tethered constraints.

B. <u>Results</u>

*1. Recombinant RSV soluble F protein polypeptide loses Site Ø binding antibody-binding in liquid but not lyophilized formulation*

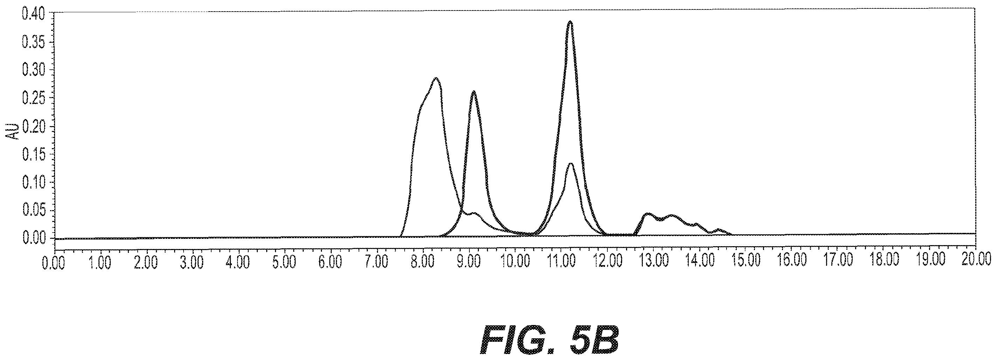

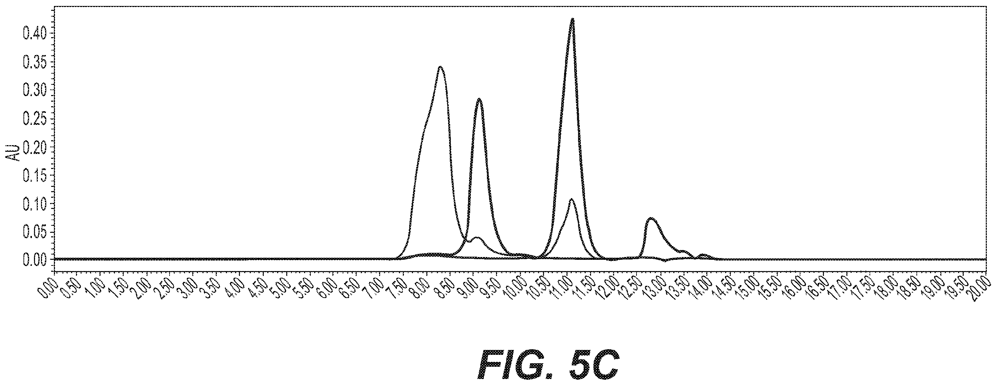

Complementary to immunoassay experiments such as ELISA and SPR, a Fabshift assay was implemented as an orthogonal method to evaluate the binding between recombinant RSV soluble F protein polypeptide and its corresponding antibodies. After incubation of the Fab and recombinant RSV soluble F protein polypeptide together at 37°C for one hour, the mixed sample was then resolved by size exclusion chromatography run under non-denaturing conditions. Fab-bound recombinant RSV soluble F protein polypeptide trimer elutes earlier, thus its retention time shifts to the left on the chromatogram because of the increased molecular weight and hydrodynamic size (~330 kDa, if 3 Fabs are bound, vs. ~183 kDa for unbound recombinant RSV soluble F protein polypeptide trimer). By comparing the peak areas of the shifted and unshifted species, the percentage of recombinant RSV soluble F protein polypeptide losing Site Ø binding antibody-binding can be estimated. A recombinant RSV soluble F protein polypeptide + Site Ø binding antibody Fabshift chromatogram of the 3-month stability sample is shown in Fig. 5. Free recombinant RSV soluble F protein polypeptide trimer eluted around 9.2 min while the Site Ø binding antibody-bound recombinant RSV soluble F protein polypeptide shifted to a shorter retention time of 8.2 min. The asymmetry of this shifted peak was presumably due to the heterogeneity of the Fab, whose elution peak at 11.2 min also showed asymmetry. As shown in the table insert in FIG.5A, this particular recombinant RSV soluble F protein polypeptide material had 86% shiftable fraction and the percentages gradually decreased to 76%, 69%, and 58% in 1-month, 3-month, and 7-month stability materials, which were all stored in liquid formulation at 5 °C. This observation was in agreement with a number of previously reported results. Against previous suggestion to use protein-stabilizing excipients, the liquid formulation involved in the current study contained levels of disaccharide and nonionic surfactant but failed to prevent loss of Site Ø binding antibody-binding. In contrast to the liquid formulation, no Site Ø binding antibody-binding loss was observed for samples held at 40 °C for 2 weeks (FIG.5B) and up to 6 months at 5 °C (FIG.5C for lyophilized recombinant RSV soluble F protein polypeptide with the same formulation excipients. This confirms that the recombinant RSV soluble F protein polypeptide is amenable to lyophilisation and suggested lyophilized formulation may be preferred in order to extend the shelf life of recombinant RSV soluble F protein polypeptide produced according to the process disclosed and other recombinant RSV soluble F protein polypeptide RSV vaccines.

*2. Primary sequence integrity and aggregation are not causes of Site Ø binding antibody-binding loss*

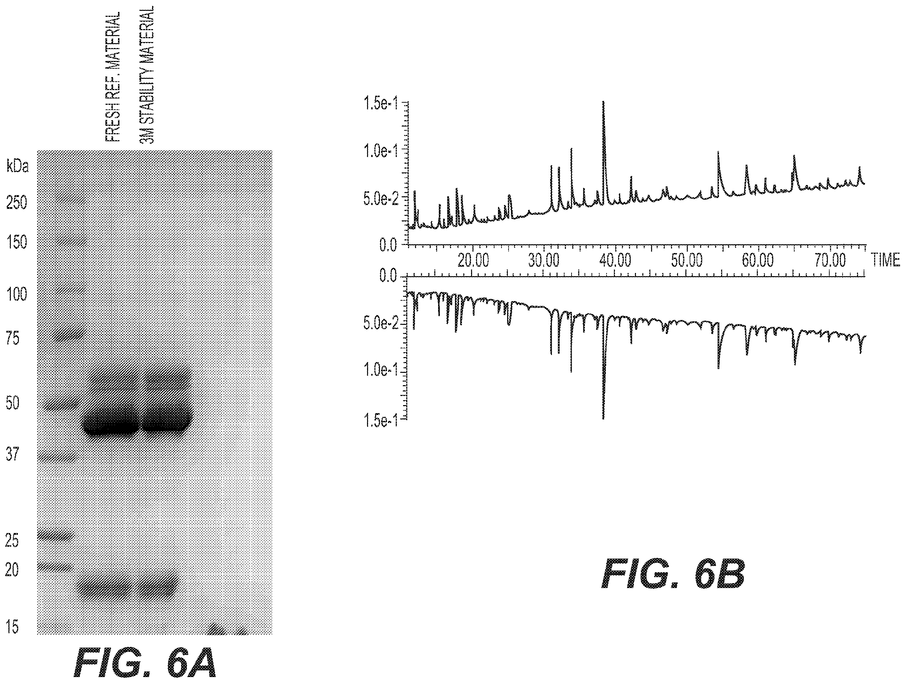

To determine if protein clipping occurred, a reduced SDS-PAGE gel was run to compare the fresh reference and 3-month 5°C stability materials. Mature recombinant RSV soluble F protein polypeptide is a two-chain molecule comprising F1 and F2 polypeptides linked together by intramolecular disulfide bonds. As shown in FIG.6A, reduced recombinant RSV soluble F protein polypeptide migrated on the gel as two main bands. The higher molecular weight band of ~ 45 kDa was the F1 polypeptide; the lower molecular weight band of ~ 10 kDa was the F2 polypeptide; the minor bands above F1 were different F1 glycoforms. Overall, the gel band patterns were very similar between the fresh reference and 3-month stability materials, suggesting that the Site Ø binding antibody-binding loss in the 3-month material (FIG.5A) was not due to a protein clipping event.

To investigate if protein aggregation was factor, the fresh, 1-month, 3-month, and 7-month materials were compared by SEC-LC and the results showed very similarly low levels of high molecular weight species among all materials (data not shown), indicating that protein aggregation was not a factor in loss of Site Ø binding antibody-binding (inset panel, FIG.5A).

In addition, peptide mapping by LC-MS/MS was also performed with over 96% sequence coverage. As shown in FIG.6B, the fresh reference and 3-month materials had exactly the same peptide fingerprint, which suggested that chemical modifications, such as oxidation, deamination, and disulfide bond scrambling, were also not cause of the loss of Site Ø binding antibody-binding.

*3. Higher order structure changes are linked to loss* of *Site Ø binding antibody-binding*

*3. 1. Circular Dichroism*

Tertiary structures were investigated by near-UV CD with wavelengths of 250-340 nm. Postfusion RSV-F as a positive control, showed a drastically different spectrum than prefusion recombinant RSV soluble F protein polypeptide (not shown). Fresh reference and 1, 3, 7-month stability materials showed visually similar spectra and demonstrated differences after analysis using a more sophisticated statistical approach. The current trend in higher-order structure comparison methods is with the use of the weighted spectral difference (WSD) analysis. According to a Tier 2 statistical analysis, if all of the WSD values from different spectra fall beyond the $-2\sigma/+2\sigma$ of the mean of base comparison sample, then the two spectra are considered to be statistically different. The mean WSD and $-2\sigma/+2\sigma$ values of fresh reference recombinant RSV soluble F protein polypeptide material were 0.000222 and 0.000148/0.000297, respectively. All WSD measurements for 1, 3, and 7-month stability materials all fell outside of the $-2\sigma/+2\sigma$ range of the fresh reference recombinant RSV soluble F protein polypeptide (not shown). In addition, the differences in WSD values increased over time, suggesting that rather than a random shift, the shift in higher order structure was in one direction towards the postfusion conformation (not shown).

*3.2. Differential Scanning Fluorimetry*

The detected differences in higher order structure between fresh reference material and stability recombinant RSV soluble F protein polypeptide material appeared visually small, which might be attributed to the insensitivity of near-UV CD towards localized small conformational changes. To further characterize and confirm the conformational changes, analysis and comparison of the materials was carried out using a nano-Differential Scanning Fluorimetry (DSF) instrument, which measures the thermal stability of a protein by determining its onset melting temperature ($T_{onset}$) and the melting temperature ($T_m$). $T_{onset}$ is the temperature at which protein starts to unfold and $T_m$ is the point at which the protein is 50% unfolded. Higher structural stability correlates with increased $T_m$. The nano-DSF detects and records the changes in the intrinsic tyrosine and tryptophan fluorescence that occur as protein unfolds with increasing temperature. Recombinant RSV soluble F protein polypeptide contains 4 tryptophans and 20 tyrosines, which are well spread out in different regions on the primary sequence and distinct domains on the 3-dimentional structure. As protein unfolds, the buried tryptophan and tyrosine residues gain more exposure to the aqueous environment. Correspondingly, the tyrosine and tryptophan intrinsic fluorescence decreases and its emission peak shifts from 330 nm (when in a buried environment) to 350 nm (when in aqueous environment). Thus, ratio fluorescence signals recorded at 350nm and 330nm will start to increase at the onset of the protein unfolding event. Since the melting temperature is the midpoint of the 350/330nm curve, the first derivative of the recorded data was calculated to determine onset of the unfolding event and the melting temperature.

Fresh reference, 1-month, 3-month, 7-month recombinant RSV soluble F protein polypeptide and postfusion RSV-F at ~ 5.5 $\mu$M concentration were analyzed by nano-DSF. Fresh reference recombinant RSV soluble F protein polypeptide demonstrated a broad melting curve with low $T_{onsel}$ and multiple peaks, suggesting that recombinant RSV soluble F protein polypeptide had a 'flexible' structure and that multiple unfolding events were involved. The $T_m$, recorded as the apex of the most intense transition, was 72.2 °C. In contrast, postfusion RSV-F demonstrated a high $T_{onset}$ and a distinct single-event unfolding curve with $T_m$ at 92.5°C, which suggested that postfusion RSV-F had a 'rigid' and more stable structure. Interestingly, for the stability materials, although the $T_{onset}$ remained largely unchanged, the melting curves shifted to higher temperature with time. A new transition with higher $T_m$ at ~79.1°C started to appear. The magnitude of this transition intensified with longer time at 5 °C. Concurrently, magnitude of the main recombinant RSV soluble F protein polypeptide transition at 72.2 °C decreased. Although the melting curves of recombinant RSV soluble F protein polypeptide stability materials shifted toward postfusion, no transition higher than 79.1 °C was ever obtained. The characteristic sharp transition of postfusion RSV-F at 92.5 °C remained absent in recombinant RSV soluble F protein polypeptide stability samples. (Data not shown.) Together, these data suggested that while in liquid formulation at 5 °C, recombinant RSV soluble F protein polypeptide adopted a new conformation that was relatively more stable than the prefusion conformation but different and less stable than the postfusion conformation. The new conformation could not further transit into the postfusion state.

An investigation was carried out of whether gain of the new conformation was the cause of Site Ø binding antibody-binding loss and the increase of unshiftable fraction in the Fabshift assay. The Site Ø binding antibody Fab unshiftable fraction peak on the SEC-LC was collected, buffer changed into the same formulation as fresh reference recombinant RSV soluble F protein polypeptide, diluted to the same concentration, and analyzed similarly by nano-DSF. The unshiftable fraction demonstrated the same $T_{onset}$ and $T_m$ as the new conformation gained by the recombinant RSV soluble F protein polypeptide stability materials and was completely missing the 72.2 °C transition observed in the fresh reference recombinant RSV soluble F protein polypeptide. In addition, the Site Ø binding antibody shifted fraction was also collected and analyzed, which showed a complete lack of the 79.1 °C transition (data not shown). Together, the DSF data suggested that the new conformation gained by recombinant RSV soluble F protein polypeptide in liquid conformation over time, which is distinctly different from both pre- and postfusion conformation, was the root cause of Site Ø binding antibody-binding loss, and that there were two populations of recombinant RSV soluble F protein polypeptide in the liquid recombinant RSV soluble F protein polypeptide stability materials, one adopting the new conformation and the other retaining the initial prefusion conformation.

*3.3. HDX-MS*

Near-UV CD and nano-DSF techniques were only able to detect overall conformational changes. To further pinpoint the exact location(s) of the conformational change in the 3D-structure, a comparison was carried out of the fresh reference and 1-month, 7-month stability materials by HDX-MS. HDX-MS probes for solvent accessibility and/or hydrogen binding of certain domains on a protein structure by measuring the exchange rates of hydrogens of the amide backbone with deuterium in solution. In this study, a total of 249 pepsin-digested peptides from each sample representing a sequence coverage of 96.9%. No significant back exchange was observed. The significance threshold was set at 0.2 Da at each time point. Thus, only $\Delta$mass greater than 1.2 Da per peptide was considered as true difference. Most peptides had none or less than significant levels of difference in deuteration uptake among the three materials tested. The only detected difference resided in amino acid residues 147-163 and the magnitude of such difference between fresh reference and stability materials increased with longer time in liquid at 5 °C. Amino acid residues 147-163 reside within the $\beta_3$-$\beta_4$ hairpin β loop motif that is in immediate proximity to the site Ø epitope. More interestingly, the five pepsin-digested peptides that constituted the majority of the site Ø epitope itself did not show any difference in deuterium uptake. Together, the HDX-MS results suggested the conformational change behind the loss of Site Ø binding antibody-binding did not impact the solvent accessibility or hydrogen bonding patterns at site Ø itself; instead stearic hindrance might have been created and interfere with the docking of Site Ø binding antibody Fab to site Ø when the amino acid residues 142-163 leading to site Ø become more solvent exposed or lose more hydrogen binding potential. This result hinted that introduction of mutation(s) within this short sequence to stabilize this hairpin domain might help maintain the potency of recombinant RSV soluble F protein polypeptide as a vaccine antigen.

*3.4. The conformational change is localized*

The HDX-MS experiment, in which only a small stretch of peptide was found to show deuterium uptake difference, further suggested that the conformational change behind Site Ø binding antibody-binding loss was a local rather than global structural change. An investigation was carried out into whether long term storage in liquid formulation at 5°C would impact binding between recombinant RSV soluble F protein polypeptide and two other RSV-F antibodies, Site V binding antibody and Site II binding antibody. Site V binding antibody is a potent neutralizing antibody that recognizes a quaternary epitope site V present only on trimeric form of prefusion RSV-F. Site II binding antibody recognizes the epitope site II that is present in both prefusion and postfusion RSV-F. Site V and site II are similarly spaced from site Ø epitope, but site V epitope significantly overlaps with the $\beta_3$-$\beta_4$ motif (not shown), while the site II epitope does not (not shown). Fabshift assays using Site V binding antibody and Site II binding antibody Fabs clearly showed long term storage in liquid at 5 °C led to similar rate of Site V binding antibody-binding loss as in the case of Site Ø binding antibody, but Site II binding antibody binding remained unaffected (not shown). These data provided additional evidence that the conformational change occurred around the $\beta_3$-$\beta_4$ regions. Furthermore, these data supported that the new conformation adopted by recombinant RSV soluble F protein polypeptide through storage in liquid formulation was result of a subtle localized rather than a global structural change.

Example 6 - Analytical Separation of preF' and preF by Liquid Chromatography

There are two approaches of analytical separation of F' and F protomers. The first is by reversed-phase liquid chromatography (RP-HPLC) under denaturing condition, during which trimers are denatured into F and F' protomers and the protomers are then resolved based on their differences in hydrophobicity; the second is by weak cation exchange liquid chromatography (WCX-LC) under native condition, during which the trimer conformation (FFF or FFF') is maintained and then resolved based on their intrinsic charge difference.

Analytical separation F' and F protomers by RP-HPLC

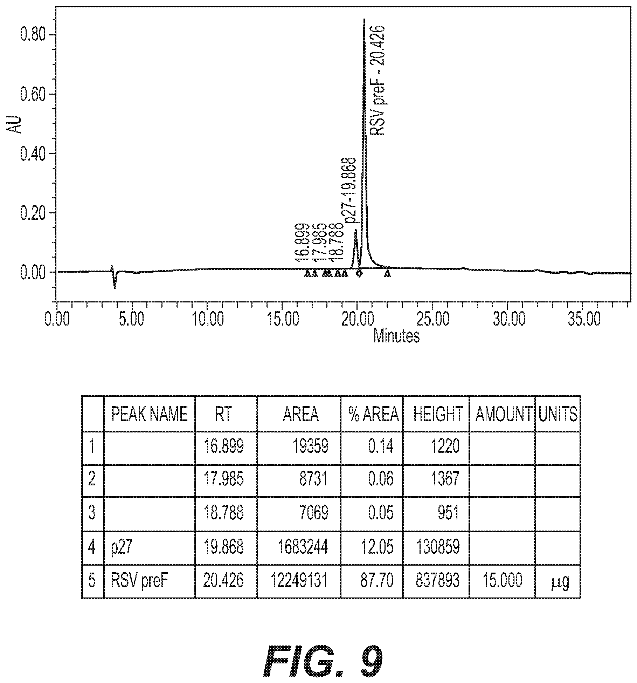

The partially purified and purified drug substance and lyophilized drug product (trimers of recombinant RSV soluble F protein polypeptides) have been analyzed by using reverse phase (RP) high performance liquid chromatography (HPLC) method, and further optimized into ultra-high performance liquid chromatography (UPLC) method. The purpose of the RP HPLC and RP UPLC is to quantitatively determine the concentration and purity of the drug substance. Two peaks of F' and F were separated based on difference of their hydrophobicity on the RP columns. The p27 peptides are extensively glycosylated by hydrophilic N-linked glycans, which renders F' less hydrophobicity. Thus, F' may elute earlier on the C18-based reversed-phase column. The two peaks (designated Peak #4 and 5) as shown in FIG.9 were collected, digested by trypsin, and analyzed by LC-MS/MS, which showed the presence of tryptic peptides originating from p27 peptide sequence in Peak #4 but not in Peak# 5. The results unambiguously confirmed Peak #4 as F' and Peak #5 as F. The RP-HPLC and RP-UPLC methods used for quantitation of these peaks are summarized in Table 5.

| Method | RP HPLC | RP UPLC |
|---|---|---|
| Instrument | Waters Acquity UPLC, Agilent 1100, Agilent 1260 | Waters Acquity UPLC |
| Mobile phase A | 0.1% Trifluoroacetic Acid (TFA) in water | 0.1% Trifluoroacetic Acid (TFA) in water |
| Mobile phase B | 0.1% Trifluoroacetic Acid (TFA) in acetonitrile | 0.1% Trifluoroacetic Acid (TFA) in acetonitrile |

| Method | RP HPLC | | | RP UPLC | | |
|---|---|---|---|---|---|---|
| Column | XBridge C4 column, 300Å, 4.6 x 150 mm, 3.5µm, Waters, Part # 186004504 | | | BEH, C4 column, 300Å, 2.1 x 150 mm, 1.7µm, Waters, Part # 186004497 | | |
| Gradient | Time (min) | % Mobile Phase A | % Mobile Phase B | Time (min) | % Mobile Phase A | % Mobile Phase B |
| | 0.0 | 98.0 | 2.0 | 0.00 | 59.0 | 41.0 |
| | 0.1 | 98.0 | 2.0 | 0.55 | 41.0 | 59.0 |
| | 25.0 | 30.0 | 70.0 | 0.56 | 1.0 | 99.0 |
| | 30.0 | 10.0 | 90.0 | 0.75 | 1.0 | 99.0 |
| | 35.0 | 10.0 | 90.0 | 0.76 | 59.0 | 41.0 |
| | 37.0 | 98.0 | 2.0 | 3.00 | 59.0 | 41.0 |
| | 45.0 | 98.0 | 2.0 | | | |
| Flow Rate | 0.5mL/min | | | 0.4mL/min | | |
| Absorbance | UV 215nm | | | UV 280nm | | |
| Column Temperature | 60°C ± 1°C | | | 60°C ± 1°C | | |
| Sample Temperature | 5°C ± 3°C | | | 5°C ± 3°C | | |
| Run time | 45min | | | 3min | | |

**Table 5.** Summary of RP HPLC and RP UPLC Methods.

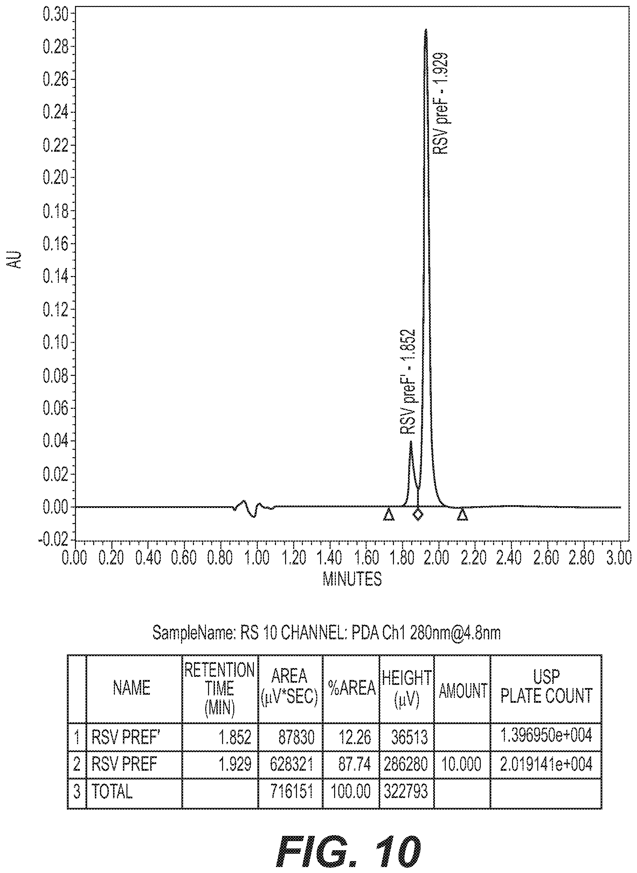

Both methods were thoroughly evaluated for specificity, linearity, repeatability, accuracy, limit of quantitation, and limit of detection. Multiple lots of drug substance were analyzed and showed the range of 12% to 16% of F'. Representative chromatograms of drug substance from HPLC and UPLC methods are shown in FIG.9 and FIG.10.

Analytical separation FFF and FFF' trimers by WCX-LC

Existing analytical methods, such as reversed phase liquid chromatography (RP-HPLC) and SDS-PAGE, are capable of quantifying amount of F' and F but not the population of trimer that contained F' as these methods are performed in denaturing condition and trimers are not covalently linked. A gap was identified and need for new analytical method was desired to understand the molecule in native condition and accurately quantify presence of F' in trimers while maintaining trimeric form.

A new analytical method, Weak Cation Exchange liquid chromatography (WCX-LC), has been developed to characterize the trimers. This method is capable of separating different forms of trimers (FFF or FFF'). The p27 peptide is a 27-amino acid residue peptide on N-terminus of the F1 protomer of a F' protomer, which is not cleaved by furin during cell culture process. The WCX-LC method is capable of baseline separating a FFF trimer from a trimer comprising at least one F', i.e., FFF'.

Ion exchange chromatography separation is based on overall surface charge of the analyte and in a non-denaturing condition. Weak cation exchange method is feasible to separate trimers based on presence or absence of p27 peptide as this length of peptide has 9 positively charged amino acids and 4 sialic acids on the two glycosylation sites resulting in the presence of p27 peptide adding a net five positive charges compared to a protomer lacking p27 peptide.

**Table 6 (cont.).** Summary of WCX-LC method for RSVPreF3 {originally identified using the term "DS-Cav1", herein re-termed RSVPreF3 to distinguish it from the material produced as disclosed in McLellan et. al. Science (2013) vol. 342 p. 592, WO2014160463, and ClinicalTrials.gov Identifier: NCT03049488},.

| Method | WCX-LC |
|---|---|
| Instrument | Waters Acquity UPLC |
| Mobile phase A | 100 mM sodium Phosphate monobasic |
| Mobile phase B | 100 mM sodium Phosphate dibasic |
| Mobile phase C | 1 M sodium chloride |
| Mobile phase D | Water |
| Column | ProPac 10 WCX Column, 4 mmX150 mm, 10 $\mu$m |
| Gradient | Sodium chloride concentration is increased from 0 mM to 400 mM in 25 minutes to elute the protein of interest |
| Flow Rate | 1 mL/min |
| Absorbance | UV 215nm |
| Run time | 30 min |

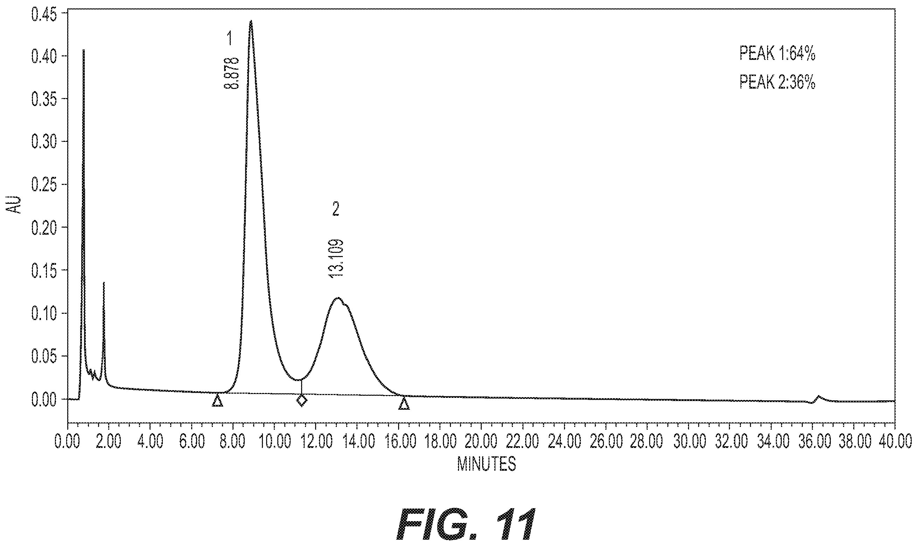

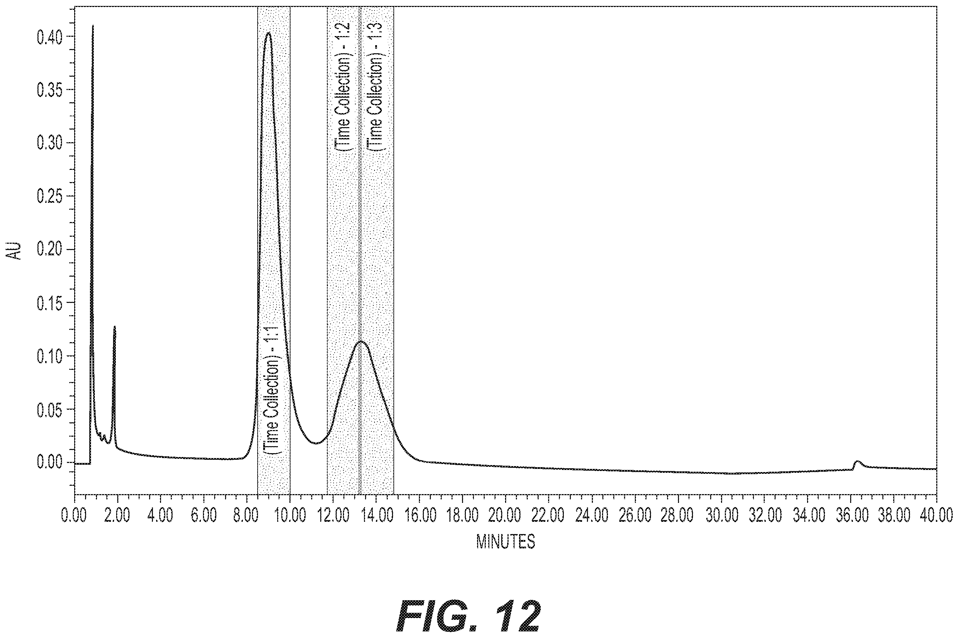

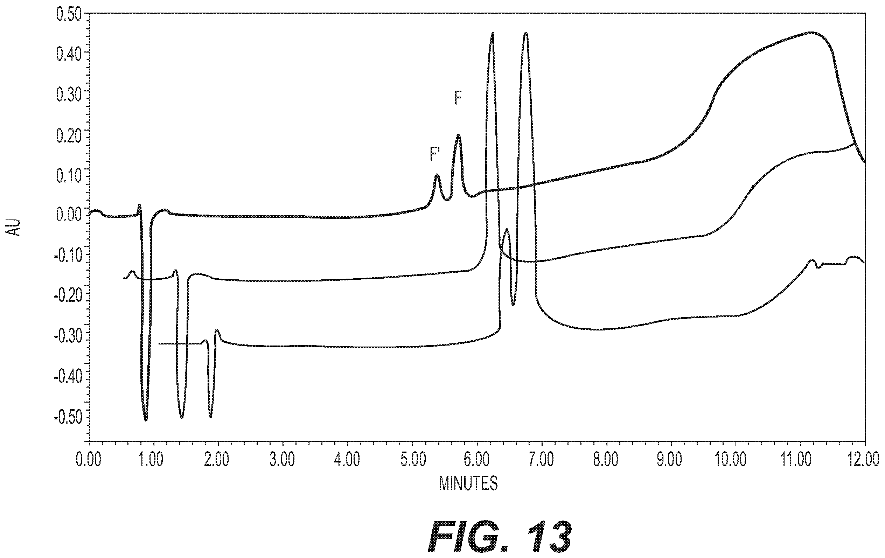

The WCX-LC is performed using mild non-denaturing condition, under which trimers retain trimeric form together by the C-terminal foldon domain. Trimers of recombinant RSV soluble F protein polypeptide, when subjected to weak cation exchange chromatography according to above mentioned experimental condition, separated into two distinct baseline separated peaks denoted as Peak 1 and Peak 2 (FIG.11). Area under Peak 1 is approximately two thirds of total peak area and that of Peak 2 is approximately one third. To characterize the composition of two peaks, timed fractions were collected using automated fraction collector as shown in FIG.12. These fractions were concentrated and subjected to further separation by RP-HPLC as described in previous sections. The results from RP-HPLC demonstrated that Peak 1 from weak cation exchange is pure FFF trimer, whereas Peak 2 is F'-containing trimers (FIG.13). Composition of F' on Peak 2 is approximately one-third and F is two thirds, suggesting that the trimer in Peak 2 consists of one F' and two F protomers. Further evidence is discussed in later section.

Homology modeling of trimers of recombinant RSV soluble F protein polypeptide suggests that unmodified p27 peptide just fits inside the cavity of the trimer and modelling suggests it would not fit inside if it has glycans attached on glycosylation motifs (not shown). Previous glycosylation profiling experiments indicate that both glycosylation sites of the p27 peptide are fully decorated with sialylated bi-antennary glycans. In addition, *in vitro* treatment with furin cleaves off the p27 peptide (data not shown). Also, as previously described, p27 peptide has 5 positive charges on its side chains. Homology modelling indicates that same charge repelling makes the preferred location to be on the surface. Further, F'-containing trimers are separated from trimers without F' in WCX-LC, a chromatography method which separates molecules by surface charges. Whilst not wishing to be bound by theory, these indicia support a conclusion that the p27 peptide protrudes out on the surface of a trimer of recombinant RSV soluble F protein polypeptides.

Besides the observation that the WCX-LC Peak 2 contains F and F' protomers with the ratio of ~2, the following observations support a conclusion that the trimers tested herein have one F' protomer. First, the predicted pI of FFF, FFF', and FF'F' from 3D structural modeling are 8.46, 8.66, and 8.84, respectively. The WCX-LC uses a linear salt gradient for elution. Thus the differences in retention time between FFF/FFF' and FFF'/FF'F' should be roughly the same. At the predicted retention time of FF'F', there is no detectable peak eluting from WCX column See FIG.14, depicting the putative retention time for a FF'F' peak (dashed curved line within the dashed circle).

To further investigate, a mutated form of RSVPreF3 {originally identified using the term "DS-Cav1", herein re-termed RSVPreF3 to distinguish it from the material produced as disclosed in McLellan et. al. Science (2013) vol. 342 p. 592, WO2014160463, and ClinicalTrials.gov Identifier: NCT03049488}, with the p27 peptide stably attached to F1 polypeptide, (referred to herein as "R295"), was investigated by weak cation exchange chromatography. In R295, the furin cleavage site at the N-terminus of the F1 chain is mutated such that three positively charged amino acids are replaced by uncharged amino acids, specifically Lys->Ser at the second amino acid, Arg->Gly at the third amino acid, and Arg->Ser at the fourth amino acid. These mutations prevent furin cleavage and R295 exists in the form of F'F'F' trimer. One F' in R295 has two net positive charges (5-3=2) in the p27 peptide. Thus, the charge difference between regular FFF and R295 F'F'F' is six positive charges (2 X 3=6). In WCX-LC, R295 F'F'F' elutes ~1/2 minute later than the predicted FFF' trimers described above. See FIG.14. Since the charge difference between R295 F'F'F' and regular FFF' is only one (6-5=1), this demonstrates that the WCX-LC method is capable of distinguishing a charge difference of only 1. In addition, via chemical crosslinking of the protomers comprising trimers was utilized to stabilize the trimer conformation by covalent bonds. LC-MS analysis of the cross-linked trimer did not detect a mass corresponding to that of FF'F' nor F'F'F' (data not shown). In conclusion, by testing the population of trimers herein only FFF and FFF' were observed, in one instance with the FFF' trimers representing ~36% of the total.

Example 7 - RSVPreF3 compared to DS-Cav1

Exemplary recombinant RSV soluble F protein polypeptide (hereinafter "RSVPreF3") was compared to the DS-Cav1 composition described in McLellan et. al. Science (2013) vol. 342 p. 592, WO2014160463, and ClinicalTrials.gov Identifier: NCT03049488 using techniques including those described in Examples 1-4. The following summary provides an overview of RSVPreF3 and DS-Cav1 utilized in the studies.

    1. Formulations:

        a. RSVPreF3

            i. 10mM potassium phosphate, 5% trehalose, 0.05% polysorbate 80, at pH 7.0
            ii. Presentation - lyophilized

        b. DS-Cav1

            i. 20mM Histidine, 100mM potassium chloride, 100 mM arginine, 2.5% sucrose at pH6.5
            ii. Presentation is frozen liquid

    2. Production:

        a. RSVPreF3 is produced using a CHO cell line different from the CHO cell line used to produce DS-Cav1
        b. RSVPreF3 is produced as described elsewhere herein, whereas DS-Cav1 is produced using a different method.

Physicochemical testing

SE-HPLC. High molecular weight species (HMWS) were investigated using SE-HPLC to study trimer content for the two compositions with the following results:

| SAMPLE | % HMW | % Main Peak | % Low Molecular Weight (LMW) |
|---|---|---|---|
| RSVPreF3* | 1.5 | 96.9 | 1.6 |

(continued)

| SAMPLE | % HMW | % Main Peak | % Low Molecular Weight (LMW) |
|---|---|---|---|
| DS-Cav1** | 21.0 | 74.5 | 4.5 |

*1.0 mg/mL
**0.5 mg/mL

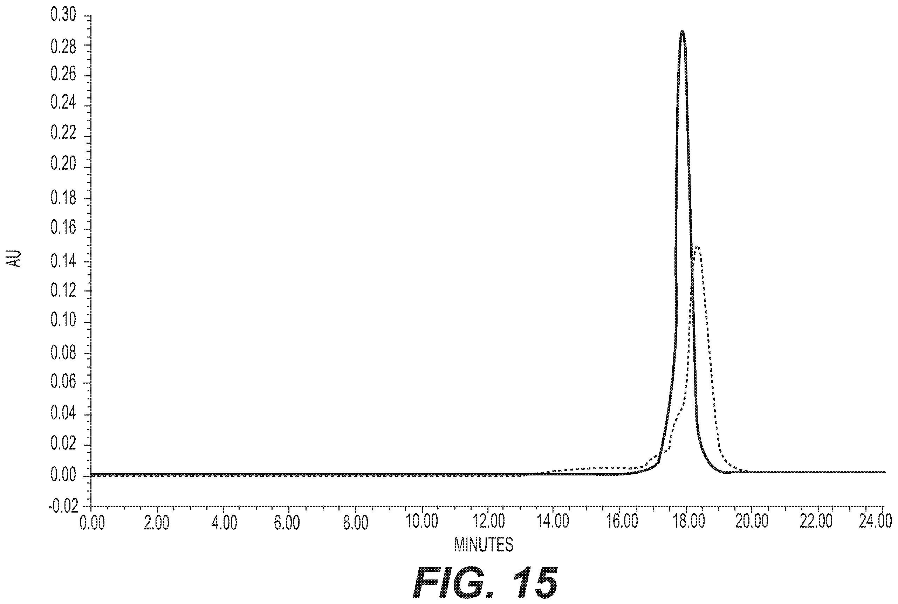

The chromatogram is shown in FIG.15. DS-Cav1 appears more heterogeneous, has a relatively high level of aggregates, and appears smaller in size than RSVPreF3.

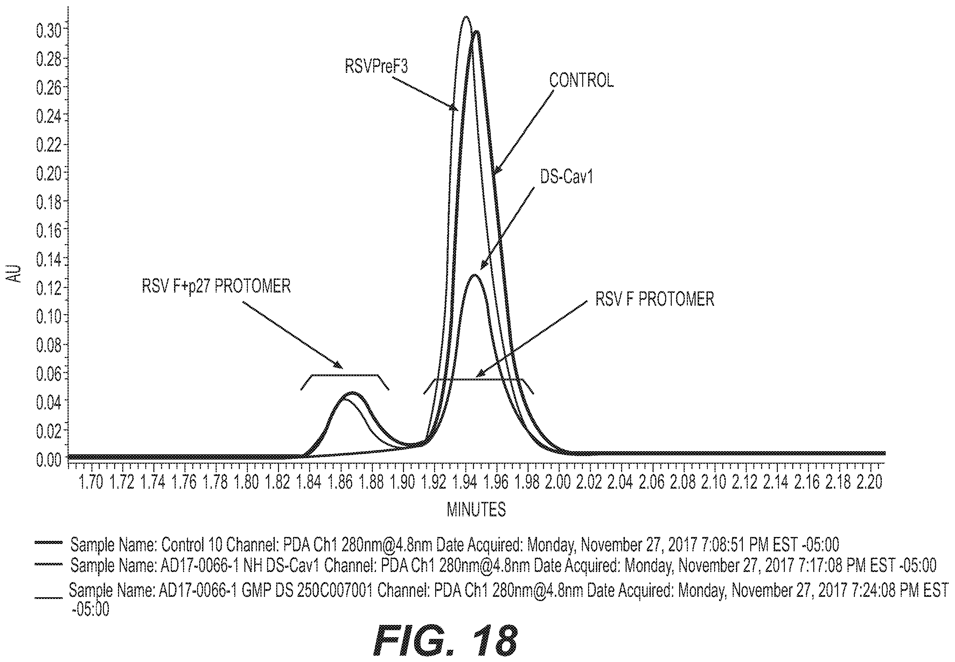

Content and purity. RP-HPLC was used to investigate content and purity of RSVPreF3 and DS-Cav1 compositions. See FIG.18. The results are summarized in the following tables.

| Attribute or Method | RSVPreF3 Ref Std | DS-Cav1 |
|---|---|---|
| RP-HPLC content & purity | 12.9% p27 | No p27 |
| P27 Western blot | P27 detected | P27 not detected |

| Item description | Concentration ($\mu$g/mL) | RSV PreF' % Area | RSV PreF % Area | Other % Area |
|---|---|---|---|---|
| RSVPreF3 1 | 940 | 12.9 | 87.1 | NA |
| RSVPreF3 2 | 987 | 14.8 | 85.2 | NA |
| DS-Cav1 | 428 | 0.0 | 99.5 | 0.5 |

Trimer profile. Weak Cation Exchange (WCX) was used to investigate the FFF and FFF' populations of RSVPreF3 and DS-Cav1. The results are summarized in the following table.

| Attribute or Method | RSVPreF3 Ref Std | DS-Cav1 |
|---|---|---|
| FFF & FFF' Populations (Weak Cation Exchange) | 2 distinct, symmetrical, well resolved peaks (FFF and FFF') | DS-Cav1 is a single relatively broad peak with retention time between FFF and FFF' |

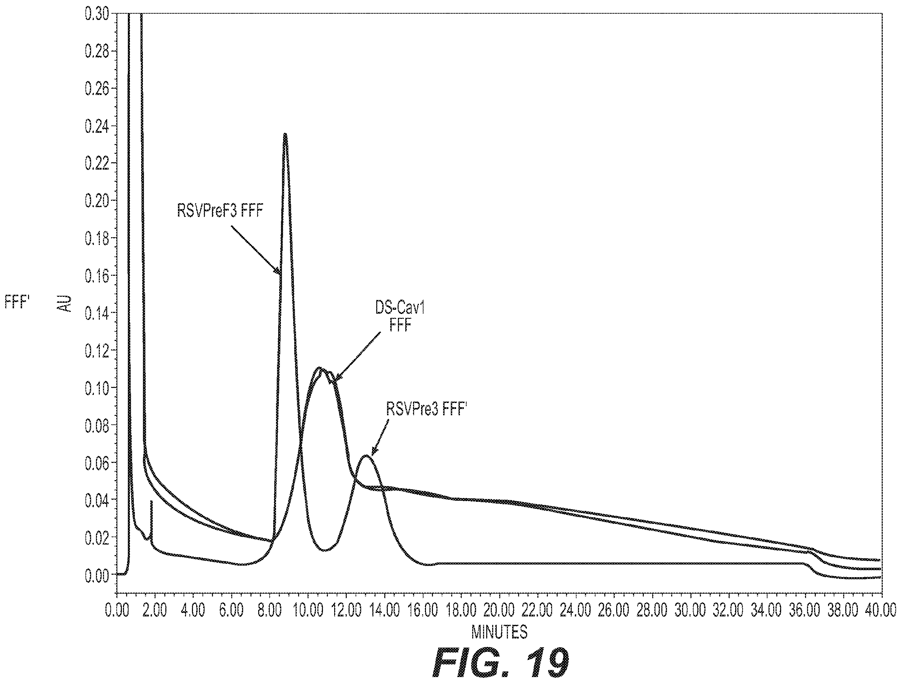

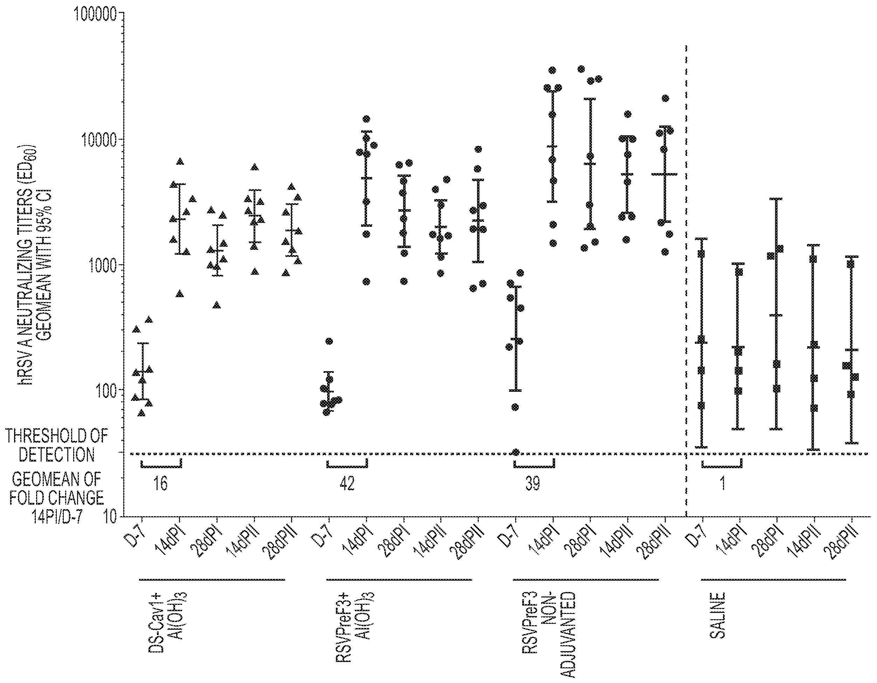

Notably, DS-Cav1 shows as relatively broad peak with retention time (RT) between FFF and FFF'. Shift in RT suggests DS-Cav1 is more basic. No FFF' present in DS-Cav1 material. The tails of DS-Cav1 is presumably due to HCP impurities and the heterogeneous nature of the material. See FIG.19.

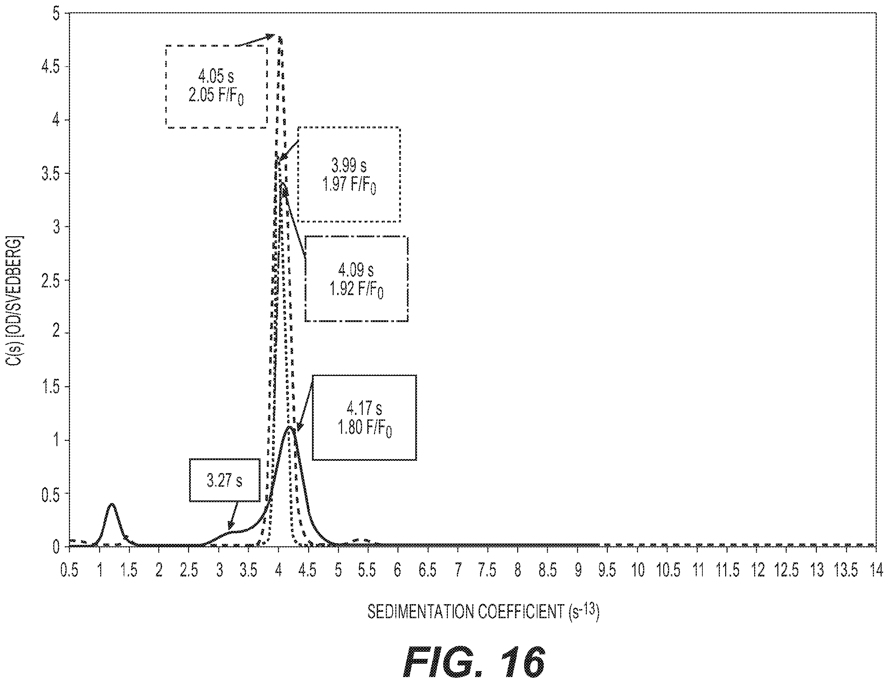

AUC. Sedimentation velocity was utilized to investigate molecular weight of the RSVPreF3 and DS-Cav1 compositions. See FIG.16. Two RSVPreF3 lyophilization samples were run; one RSVPreF3 liquid sample was run; and one DS-Cav1 liquid sample was run. RSVPreF3 ran relatively consistently run-to-run, with a single species observed with no shoulder on the chromatogram peaks. DS-Cav1 main peak fits to lower molecular weight than RSVPreF3, with an additional peak at ~3 s.

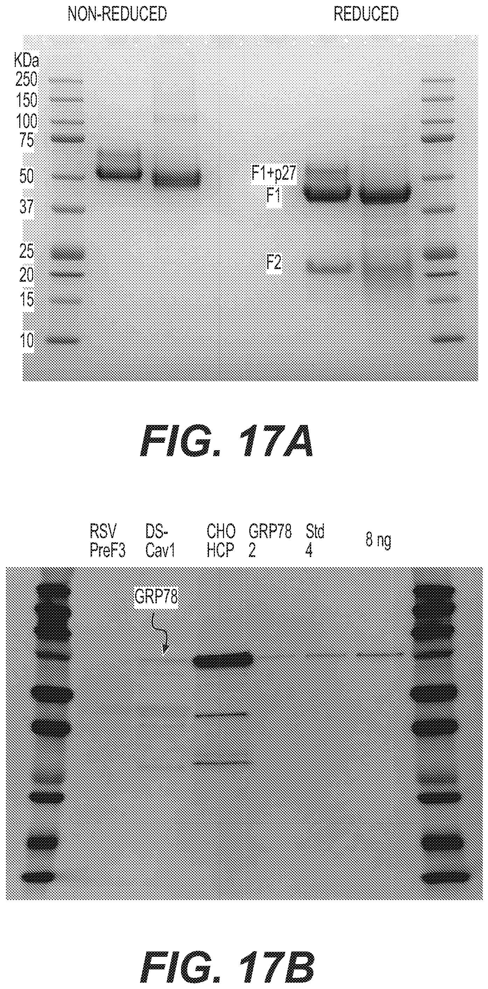

HCP. HCP-content was investigated by non-reduced and reduced SDS-PAGE, HCP kit (Cygnus F550 kit, available from Cygnus Technologies, 4332 Southport Supply Rd., SE Southport, NC 28461), and using mass-spectometry. The SDS-PAGE findings are summarized in the following table and FIG.17A.

| Attribute or Method | RSVPreF3 | DS-Cav1 |
|---|---|---|
| Non-reduced SDS-PAGE - (4 $\mu$g load) | Shows expected bands known to be glycoforms of F and F+p27. No HMW bands | Primary species is a single band smaller than RSVPreF3 F-protomer. Several HMW bands could be aggregates or HCPs |
| Reduced SDS-PAGE - (8 $\mu$g load) | Shows expected bands known to be glycoforms of F1, F1+p27, F2. One faint HMW band | Primary species is a single band same size as RSVPreF3 F1. Several HMW bands (could be aggregates or HCPs) and numerous LMW bands (could be clipped forms or HCPs). |

The HCP kit and MS findings are summarized in the table below.

| Attribute | RSVPreF3 | DS-Cav1 |
|---|---|---|
| ng HCP/ 120 ug sample (Cygnus F550 kit) | 108 ng/120 $\mu$g RSVPreF3 | 1800 ng / 120 $\mu$g DS-Cav1 |
| HCP Profiles in MS | DS-Cav1 has many more HCPs detected. The most abundant HCP is GRP78 | |

DS-Cav1 contains additional identified HCPs, including (decreasing abundance):

- GRP78
- Glyceraldehyde-3-phosphate dehydrogenase
- Follistatin-related protein 1
- Phosphoglycerate kinase
- Elongation factor 2
- Fructose-bisphosphate aldolase A
- Pyruvate kinase PKM
- Peroxiredoxin-1
- Alcohol dehydrogenase
- Peptidyl-prolyl cis-trans isomerase A
- Collagen alpha-1
- *Legumain*
- *Lysosomal protective protein*
- Clusterin
- Prostaglandin reductase

Two proteases (italics) are detected. Proteases have been known to lead to clipping in recombinant compositions.

Impurities. The most prevalent HCP is GRP78. GRP78 was measured by the targeted isotope dilution mass spectrometry method (IDMS) method. IDMS works by adding various known amounts of stable-isotope labelled standard peptides into testing samples. A standard curve was constructed by plotting peak area ratios versus the concentration of internal standards (3 peptides from GRP78). The unknown GRP78 concentration can be calculated in an interpolative manner by comparing the relative signal intensities of the analyte with the internal standards:

DS-Cav1 material has at least 10-fold higher GRP78 content in comparison to RSVPreF3 (see table, below and FIG.17B).

| Materials | GRP78 content | |
| --- | --- | --- |
| | % (w/w) | ng/120ug |
| DS-Cav1 | 0.19% | 445ng/120ug |
| RSVPreF3 GMP Lot | 0.013% | 15ng/120ug |
| RSVPreF3 Ref Std | 0.047% | 62ng/120ug |

Glycoprofile and Intact Molecular Weight (MW). Intact MW was determined by LC-MS and spectrum deconvolution, revealing RSVPreF3 to have an intact MW of 61,226 Da (F, not F') and DS-Cav1 to have an intact MW of 59,461 Da. After de-glycosylation, both molecules have the same MW of 54,162.3 Da (the calculated theoretical MW is 54,163 Da). This supports a conclusion that the molecules' primary sequence integrity and identity. The smaller MW in the DS-Cav1 composition is believed to originate from glycan differences from RSVPreF3.

| Attribute | RSVPreF3 Ref Std | DS-Cav1 |
| --- | --- | --- |
| Glycoprofile | Overall profile of DS-Cav1 is similar to RSVPreF3 Ref Std; fully glycosylated on N27 and N70; partially glycosylated on N500. Abundance of N500 non-glycosylated is slightly higher in DS-Cav1 (12.3% DS-Cav1 vs 7.2% RSVPreF3). | |
| Intact MW | DS-Cav1 MW is slightly lower than RSVPreF3 (59461 Da DS-Cav1 vs 61226 Da RSVPreF3). Appears to originate from glycosylation differences (not sequence differences). These data are consistent with AUC and SE-HPLC data | |

Immunological testing.

In Vitro Relative Potency (IVRP) was investigated using assays comprised of site-specific antibodies and polyclonal sera, with the following results:

| Attribute | RSVPreF3 Ref Std | DS-Cav1 |
| --- | --- | --- |
| IVRP Site 0 (site 0 + anti-DS-Cav1 polyclonal serum) | DS-Cav1 slightly lower binding than RSVPreF3 Ref Std | |
| IVRP Site V (site V + anti-DS-Cav1 polyclonal serum) | DS-Cav1 significantly lower binding than RSVPreF3 Ref Std. | |
| IVRP Site II (site II + anti-DS-Cav1 polyclonal serum) | DS-Cav1 similar binding as RSVPreF3 Ref Std | |
| IVRP Site III (site III + anti-DS-Cav1 polyclonal serum) | DS-Cav1 slightly lower binding than RSVPreF3 Ref Std | |
| IVRP Site I (site I + anti-DS-Cav1 polyclonal serum) | DS-Cav1 significantly higher binding than RSVPreF3 Ref Std. | |
| Surface plasmon resonance (Biacore) | No significant differences in binding | |

Loss of Site 0 or Site V binding was investigated using Fab shift (assay described elsewhere herein), with the following results:

| Attribute | RSVPreF3 Ref Std | DS-Cav1 |
| --- | --- | --- |
| % Product Binding to Site 0 mAb | 11% - 17% unshifted | 17% |
| % Product Binding to Site V mAb | 9% unshifted | 16% |

Summary of the Analytical Comparability Study of RSVPreF3 and DS-Cav1

Comparable Attributes:

- Primary sequence integrity (intact and peptide maps)
- Absence of unexpected post-translational modifications
- Higher order structure (thermostability) as measured by DSF
- Secondary structure as measured by far-UV CD
- Incomplete glycan occupancy at N500
- Comparable binding (FabShift)

Different Attributes:

- DS-CAV1 material has lower overall purity level (aggregation, HCP) and higher GRP78 content
- DS-Cav1 has lower p27 content
- DS-Cav1 has significantly lower p27 content
- DS-CAV1 material has higher aggregation level
- DS-CAV1 contains very different glycan profile, predominately neutral and smaller glycans
- Because of the difference in glycan profile, DS-CAV1 material is smaller in MW and more basic in pI
- DS-CAV1 material has a pI of ~8 while the RSVPreF3 material has a pI ~5-6

Preclinical Cow Study

The immunogenicity of the RSVPreF3 composition and the DS-Cav1 composition was compared in terms of RSV A NAb (FIG.20) according to the following immunisation and sample collection schedule:

D0      D28

D-7    14PI    28PI    14PII    28PII

The RSVPreF3 lot formulated with either Al(OH)$_3$, or non-adjuvanted elicit higher RSV A NAb titers compared to the saline group (p values <0.0001 at 14dPI). Thus, the immunogenicity of RSVPreF3 lot in primed bovines was demonstrated. A statistical difference was observed between RSVPreF3-Al(OH)$_3$ and DS-Cav1-Al(OH)$_3$ based on RSVA Nab titers (GMR=2.7 ,95% CI: 1.24-5.82) and a difference was also observed on their respective fold increase with a higher boosting of RSVA NAb titers (Geomean ratio 14PI / Day -7) observed for RSVPreF3-Al(OH)$_3$ (2,7-Fold higher with 95% CI : 1.03-7.12).

p27 Immunogenicity Impact in Naïve CB6F1 Mice

FFF' trimer was compared to FFF trimer by RSVPreF3-induced RSV A neutralizing antibody responses. Non-inferiority was demonstrated for FFF' trimers compared to FFF trimers for RSVA neutralizing antibody responses (14dpIII) (GMR = 0.80, 90%CI [0.48,1.32]).

SEQUENCE LISTINGS

SEQ ID NO:1 (precursor recombinant RSV soluble F protein polypeptide comprising S155C, S290C, S190F, and V207L substitutions)

MELLILKANAITTILTAVTFCFASGQNITEEFYQSTCSAVSKGYLSALRTGWYTSVITIELS
NIKENKCNGTDAKVKLIKQELDKYKNAVTELQLLMQSTPATNNRARRELPRFMNYTLNNAKK
TNVTLSKKRKRRFLGFLLGVGSAIASGVAVCKVLHLEGEVNKIKSALLSTNKAVVSLSNGVS
VLTFKVLDLKNYIDKQLLPILNKQSCSISNIETVIEFQQKNNRLLEITREFSVNAGVTTPVS
TYMLTNSELLSLINDMPITNDQKKLMSNNVQIVRQQSYSIMCIIKEEVLAYVVQLPLYGVID
TPCWKLHTSPLCTTNTKEGSNICLTRTDRGWYCDNAGSVSFFPQAETCKVQSNRVFCDTMNS
LTLPSEVNLCNVDIFNPKYDCKIMTSKTDVSSSVITSLGAIVSCYGKTKCTASNKNRGIIKT
FSNGCDYVSNKGVDTVSVGNTLYYVNKQEGKSLYVKGEPIINFYDPLVFPSDEFDASISQVN
EKINQSLAFIRKSDELLSAIGGYIPEAPRDGQAYVRKDGEWVLLSTFL

SEQ ID NO:2 (F1 chain of mature recombinant RSV soluble F protein polypeptide comprising S155C, S290C, S190F, and V207L substitutions)

FLGFLLGVGSAIASGVAVCKVLHLEGEVNKIKSALLSTNKAVVSLSNGVSVLTFKVLDLKNY
IDKQLLPILNKQSCSISNIETVIEFQQKNNRLLEITREFSVNAGVTTPVSTYMLTNSELLSL
INDMPITNDQKKLMSNNVQIVRQQSYSIMCIIKEEVLAYVVQLPLYGVIDTPCWKLHTSPLC
TTNTKEGSNICLTRTDRGWYCDNAGSVSFFPQAETCKVQSNRVFCDTMNSLTLPSEVNLCNV
DIFNPKYDCKIMTSKTDVSSSVITSLGAIVSCYGKTKCTASNKNRGIIKTFSNGCDYVSNKG
VDTVSVGNTLYYVNKQEGKSLYVKGEPIINFYDPLVFPSDEFDASISQVNEKINQSLAFIRK
SDELLSAIGGYIPEAPRDGQAYVRKDGEWVLLSTFL

SEQ ID NO:3 (F2 chain of mature recombinant RSV soluble F protein polypeptide comprising S155C, S290C, S190F, and V207L substitutions)

QNITEEFYQSTCSAVSKGYLSALRTGWYTSVITIELSNIKENKCNGTDAKVKLIKQELDKYK
NAVTELQLLMQSTPATNNRARR

SEQ ID NO:4 (representing non-glycosylated N500)
KINQSLAFIRK

SEQ ID NO:5(representing N500 with glycan removed by PNGase F) KIDQSLAFIRK

SEQ ID NO:6(fragment of p27 peptide detectable by mass spec) FMNYTLNNAK

SEQ ID NO:7(p27)
ELPRFMNYTLNNAKKTNVTLSKKRKRR

SEQ ID NO:8 (F1 chain of F' protomer-containing p27)

```
ELPRFMNYTLNNAKKTNVTLSKKRKRRFLGFLLGVGSAIASGVAVCKVLHLEGEVNKIKSAL
LSTNKAVVSLSNGVSVLTFKVLDLKNYIDKQLLPILNKQSCSISNIETVIEFQQKNNRLLEI
TREFSVNAGVTTPVSTYMLTNSELLSLINDMPITNDQKKLMSNNVQIVRQQSYSIMCIIKEE
VLAYVVQLPLYGVIDTPCWKLHTSPLCTTNTKEGSNICLTRTDRGWYCDNAGSVSFFPQAET
CKVQSNRVFCDTMNSLTLPSEVNLCNVDIFNPKYDCKIMTSKTDVSSSVITSLGAIVSCYGK
TKCTASNKNRGIIKTFSNGCDYVSNKGVDTVSVGNTLYYVNKQEGKSLYVKGEPIINFYDPL
VFPSDEFDASISQVNEKINQSLAFIRKSDELLSAIGGYIPEAPRDGQAYVRKDGEWVLLSTF
L
```

SEQ ID NO:9 (uncleaved furin cleavage site, X and $X_1$ are independently any amino acid)
$RXRRX_1$

**Claims**

1. An antigen composition comprising a recombinant respiratory syncytial virus (RSV) soluble F protein polypeptide comprising a F1 chain comprising an amino acid sequence of SEQ ID NO:2 and a F2 chain comprising an amino acid sequence of SEQ ID NO:3, wherein said composition comprises greater than 96%, 97%, 98%, 99%, 99.1%, 99.2%, 99.3%, 99.4%, 99.5%, 99.6%, 99.7%, 99.8%, or 99.9% recombinant RSV soluble F protein polypeptide w/w versus host-cell protein.

2. The antigen composition of claim 1, wherein said composition comprises less than 0.19%, 0.18%, 0.17%, 0.16%, 0.15%, 0.14%, 0.13%, 0.12%, 0.11%, 0.10%, 0.09%, 0.08%, 0.07%, 0.06%, 0.05%, 0.04%, 0.03%, 0.02%, 0.019%, 0.018%, 0.017%, 0.016%, 0.015%, 0.014%, or 0.013% GRP78 host-cell protein w/w versus recombinant RSV soluble F protein polypeptide.

3. An antigen composition of any preceding claim, wherein said antigen composition is lyophilized.

4. An immunogenic composition comprising an antigen composition of any preceding claim and a pharmaceutically acceptable carrier or excipient.

5. The immunogenic composition of claim 4, further comprising an adjuvant.

6. The antigen composition of any one of claims 1-3 or the immunogenic composition of any one of claims 4-5 for use in medicine.

7. The antigen composition of any one of claims 1-3 or the immunogenic composition of any one of claims 4-5 for use in the prophylaxis or treatment of RSV-infection.

**Patentansprüche**

1. Antigenzusammensetzung, umfassend ein rekombinantes, respiratorisches Synzytial-Virus (RSV)lösliches F-Protein-Polypeptid, umfassend eine F1-Kette, umfassend eine Aminosäuresequenz von SEQ ID NO: 2, und eine F2-Kette, umfassend eine Aminosäuresequenz von SEQ ID NO: 3, wobei die Zusammensetzung mehr als 96 %, 97 %, 98 %, 99 %, 99,1 %, 99,2 %, 99,3 %, 99,4 %, 99,5 %, 99,6 %, 99,7 %, 99,8 % oder 99,9 % rekombinantes RSV lösliches F-Protein-Polypeptid, w/w des Wirtszellproteins umfasst.

2. Antigenzusammensetzung gemäß Anspruch 1, wobei die Zusammensetzung weniger als 0,19 %, 0,18 %, 0,17 %, 0,16 %, 0,15 %, 0,14 %, 0,13 %, 0,12 %, 0,11 %, 0,10 %, 0,09 %, 0,08 %, 0,07 %, 0,06 %, 0,05 %, 0,04 %, 0,03 %, 0,02 %, 0,019 %, 0,018 %, 0,017 %, 0,016 %, 0,015 %, 0,014 % oder 0,013 % GRP78-Wirtszellprotein w/w des rekombinantem RSV löslichem F-Protein-Polypeptid umfasst.

3. Antigenzusammensetzung gemäß irgendeinem der vorhergehenden Ansprüche, wobei die Antigenzusammensetzung lyophilisiert ist.

4. Immunogene Zusammensetzung, umfassend eine Antigenzusammensetzung gemäß irgendeinem der vorhergehenden Ansprüche und einen pharmazeutisch akzeptablen Träger oder Hilfsstoff.

5. Immunogene Zusammensetzung gemäß Anspruch 4, ferner umfassend ein Adjuvans.

6. Antigenzusammensetzung gemäß irgendeinem der Ansprüche 1 bis 3 oder immunogene Zusammensetzung gemäß irgendeinem der Ansprüche 4 bis 5 zur Verwendung in der Medizin.

7. Antigenzusammensetzung gemäß irgendeinem der Ansprüche 1 bis 3 oder immunogene Zusammensetzung gemäß irgendeinem der Ansprüche 4 bis 5 zur Verwendung bei der Prophylaxe oder Behandlung einer RSV-Infektion.

**Revendications**

1. Composition antigénique comprenant un polypeptide de protéine F soluble de virus respiratoire syncytial (VRS) recombinant comprenant une chaîne F1 comprenant une séquence d'acides aminés de SEQ ID No : 2 et une chaîne F2 comprenant une séquence d'acides aminés de SEQ ID No : 3, dans laquelle ladite composition comprend plus de 96 %, 97 %, 98 %, 99 %, 99,1 %, 99,2 %, 99,3 %, 99,4 %, 99,5 %, 99,6 %, 99,7 %, 99,8 % ou 99,9 % p/p de polypeptide de protéine F soluble du VRS recombinant par rapport à la protéine de la cellule hôte.

2. Composition antigénique selon la revendication 1, dans laquelle ladite composition comprend moins de 0,19 %, 0,18 %, 0,17 %, 0,16 %, 0,15 %, 0,14 %, 0,13 %, 0,12 %, 0,11 %, 0,10 %, 0,09 %, 0,08 %, 0,07 %, 0,06 %, 0,05 %, 0,04 %, 0,03 %, 0,02 %, 0,019 %, 0,018 %, 0,017 %, 0,016 %, 0,015 %, 0,014 % ou 0,013 % p/p de protéine de cellule hôte GRP78 par rapport au polypeptide de protéine F soluble du VRS recombinant.

3. Composition antigénique selon l'une quelconque des revendications précédentes, dans laquelle ladite composition antigénique est lyophilisée.

4. Composition immunogène comprenant une composition antigénique selon l'une quelconque des revendications précédentes et un support ou un excipient pharmaceutiquement acceptable.

5. Composition immunogène selon la revendication 4, comprenant en outre un adjuvant.

6. Composition antigénique selon l'une quelconque des revendications 1 à 3 ou composition immunogène selon l'une quelconque des revendications 4 à 5, pour une utilisation en médecine.

7. Composition antigénique selon l'une quelconque des revendications 1 à 3 ou composition immunogène selon l'une quelconque des revendications 4 à 5, pour une utilisation dans la prophylaxie ou le traitement de l'infection par le VRS.

*FIG. 1A*

*FIG. 1B*

FURIN CLEAVAGE SITES

FIG. 2

**FIG. 3A**

**FIG. 3B**

| ID | RT (mn) | CHARGE STATE | m'2 |
|----|---------|--------------|-----------|
| 1 | 25.03 | 2 | 954.4747 |
| 2 | 27.48 | 2 | 1100.0371 |
| 3 | 29.73 | 2 | 1245.5941 |

**FIG. 4**

**FIG. 5A**

EP 3 829 618 B1

**FIG. 5B**

EP 3 829 618 B1

**FIG. 5C**

FIG. 6A

FIG. 6B

**FIG. 7**

*FIG. 8*

| | PEAK NAME | RT | AREA | % AREA | HEIGHT | AMOUNT | UNITS |
|---|---|---|---|---|---|---|---|
| 1 | | 16.899 | 19359 | 0.14 | 1220 | | |
| 2 | | 17.985 | 8731 | 0.06 | 1367 | | |
| 3 | | 18.788 | 7069 | 0.05 | 951 | | |
| 4 | p27 | 19.868 | 1683244 | 12.05 | 130859 | | |
| 5 | RSV preF | 20.426 | 12249131 | 87.70 | 837893 | 15.000 | µg |

## FIG. 9

SampleName: RS 10 CHANNEL: PDA Ch1 280nm@4.8nm

| | NAME | RETENTION TIME (MIN) | AREA (µV*SEC) | %AREA | HEIGHT (µV) | AMOUNT | USP PLATE COUNT |
|---|---|---|---|---|---|---|---|
| 1 | RSV PREF' | 1.852 | 87830 | 12.26 | 36513 | | 1.396950e+004 |
| 2 | RSV PREF | 1.929 | 628321 | 87.74 | 286280 | 10.000 | 2.019141e+004 |
| 3 | TOTAL | | 716151 | 100.00 | 322793 | | |

*FIG. 10*

FIG. 11

## FIG. 12

EP 3 829 618 B1

**FIG. 13**

FIG. 14

**FIG. 15**

*FIG. 16*

## FIG. 17A

## FIG. 17B

FIG. 18

*FIG. 19*

*FIG. 21*

**FIG. 22A**

TITER (mg/L)

**FIG. 22B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014160463 A
- US 4436727 A
- US 4877611 A
- US 4866034 A
- US 4912094 A
- GB 2220211 A
- WO 9421292 A
- WO 9850399 A
- US 6303347 B
- WO 2008153541 A
- WO 2009143457 A
- EP 0362278 A
- WO 2012080369 A
- US 4235877 A, Fullerton
- US 4372945 A
- US 4474757 A, Armor

**Non-patent literature cited in the description**

- **MCLELLAN et al.** Characterization of a Prefusion-Specific Antibody That Recognizes a Quaternary, Cleavage-Dependent Epitope on the RSV Fusion Glycoprotein. *PLOS Pathog*, 2015, vol. 11, e1005035
- **MCLELLAN**. *Science*, 2013, vol. 342, 592
- **MOEHRING et al.** Strains of CHO-K1 cells resistant to Pseudomonas exotoxin A and cross-resistant to diphtheria toxin and viruses. *Infect. Immun.*, 1993, vol. 41, 998-1009
- **E. W. MARTIN**. Remington's Pharmaceutical Sciences. Mack Publishing Co., 1975
- **SMITH** ; **WATERMAN**. *Adv. Appl. Math.*, 1981, vol. 2, 482
- **NEEDLEMAN** ; **WUNSCH**. *J. Mol. Biol.*, 1970, vol. 48, 443
- **HIGGINS** ; **SHARP**. *Gene*, 1988, vol. 73, 237
- **HIGGIN** ; **SHARP**. *CABIOS*, 1989, vol. 5, 151
- **CORPET et al.** *Nucleic Acids Research*, 1988, vol. 16, 10881
- **PEARSON** ; **LIPMAN**. *Proc. Natl. Acad. Sci. USA*, 1988, vol. 85, 2444
- **ALTSCHUL et al.** *Nature Genet.*, 1994, vol. 6, 119
- **ALTSCHUL et al.** *J. Mol. Biol.*, 1990, vol. 215, 403
- **SAMBROOK et al.** Molecular Cloning: A Laboratory Manual. Cold Spring Harbor Laboratory Press, 1989
- **SAMBROOK et al.** Molecular Cloning: A Laboratory Manual. Cold Spring Harbor Press, 2001
- **AUSUBEL et al.** Current Protocols in Molecular Biology. Greene Publishing Associates, 1992
- **AUSUBEL et al.** Short Protocols in Molecular Biology: A Compendium of Methods from Current Protocols in Molecular Biology. Wiley & Sons, 1999
- **SAMBROOK et al.** Molecular Cloning: A Laboratory Manual. Cold Spring Harbor Laboratory Press
- **AUSUBEL et al.** Short Protocols in Molecular Biology. John Wiley & Sons
- **FRESHNEY**. Culture of Animal Cells, a Manual of Basic Technique. Wiley- Liss, 1994
- **PAYNE et al.** Plant Cell and Tissue Culture in Liquid Systems. John Wiley & Sons, 1992
- Plant Cell, Tissue and Organ Culture. Fundamental Methods Springer Lab Manual. Springer-Verlag, 1995
- The Handbook of Microbiological Media. CRC Press, 1993
- **VAN HEEKE** ; **SCHUSTER**. *J Biol Chem*, 1989, vol. 264, 5503-5509
- **GRANT et al.** *Methods in Enzymology*, 1987, vol. 153, 516-544
- **KGWU et al.** The Effect of Buffers on Protein Conformational Stability. *Pharmaceutical Technology*, 2004, vol. 28, 86-108
- **HSU et al.** Determining the optimum residual moisture in lyophilized protein pharmaceuticals. *Developments in Biological Standardization*, 1992, vol. 74, 255-70
- **COSTANTINE et al.** Effect of excipients on the stability and structure of lyophilized recombinant human growth hormone. *J. Pharm. Sci.*, 1998, vol. 87, 1412-1420
- **E. W. MARTIN**. Remington's Pharmaceutical Sciences. Mack Publishing Co.

- **COLER RN et al.** Development and Characterization of Synthetic Glucopyranosyl Lipid Adjuvant System as a Vaccine Adjuvant. *PLoS ONE*, 2011, vol. 6 (1), e16333
- **ARIAS MA et al.** Glucopyranosyl Lipid Adjuvant (GLA), a Synthetic TLR4 Agonist, Promotes Potent Systemic and Mucosal Responses to Intranasal Immunization with HIVgp140. *PLoS ONE*, 2012, vol. 7 (7), e41144
- Saponin adjuvants. **DALSGAARD et al.** Archiv. für die gesamte Virusforschung. Springer Verlag, 1974, vol. 44, 243-254
- Vaccine Design-the subunit and adjuvant approach. Pharmaceutical Biotechnology. Plenurn Press, 1995, vol. 61
- New Trends and Developments in Vaccines. University Park Press, 1978
- **MCLELLAN et al.** Structure-Based Design of a Fusion Glycoprotein for Respiratory Syncytial Virus. *Science*, 2013, vol. 342, 592-598
- **KRARUP et al.** A highly stable prefusion RSVF vaccine derived from structural analysis of the fusion mechanism. *Nat Commun*, 2015, vol. 6, 8143
- **FLYNN**. Stability Characterization of a Vaccine Antigen Based on the Respiratory Syncytial Virus Fusion Protein. *Plos One*, 2016, vol. 10, 1-18